(19) Europäisches Patentamt / European Patent Office / Office européen des brevets

(11) **EP 4 140 544 A1**

(12) **EUROPEAN PATENT APPLICATION**
published in accordance with Art. 153(4) EPC

(43) Date of publication:
**01.03.2023 Bulletin 2023/09**

(21) Application number: **21792866.2**

(22) Date of filing: **19.04.2021**

(51) International Patent Classification (IPC):
***A61Q 17/04*** *(2006.01)* ***A61Q 19/00*** *(2006.01)*
***A61Q 19/08*** *(2006.01)* ***A61H 23/02*** *(2006.01)*
***A61K 8/9761*** *(2017.01)*

(52) Cooperative Patent Classification (CPC):
**A61H 23/02; A61K 8/9761; A61Q 17/04; A61Q 19/00; A61Q 19/08**

(86) International application number:
**PCT/JP2021/015919**

(87) International publication number:
**WO 2021/215409 (28.10.2021 Gazette 2021/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.04.2020 JP 2020075019**

(71) Applicant: **Shiseido Company, Ltd.**
**Chuo-ku,**
**Tokyo 104-0061 (JP)**

(72) Inventors:
- **HORIBA, Satoshi**
  **Tokyo 104-0061 (JP)**
- **HOSOI, Junichi**
  **Tokyo 104-0061 (JP)**
- **INOUE, Daigo**
  **Tokyo 104-0061 (JP)**
- **KAJIYA, Kentaro**
  **Tokyo 104-0061 (JP)**
- **TAKAGI, Masaya**
  **Tokyo 104-0061 (JP)**
- **MATSUURA, Yuko**
  **Tokyo 104-0061 (JP)**
- **IINO, Masato**
  **Tokyo 104-0061 (JP)**

(74) Representative: **Cabinet Beau de Loménie**
**158, rue de l'Université**
**75340 Paris Cedex 07 (FR)**

(54) **AGENT FOR PREVENTING AND/OR IMPROVING PHOTOAGING AND/OR DERMAL PIGMENTATION, COSMETIC METHOD USING SAME, AND COSMETIC DEVICE TO BE APPLIED IN SAID METHOD**

(57) The present invention provides an agent for preventing and/or improving photoaging and/or dermal pigmentation comprising Thujae Semen extract as an active ingredient. The present invention also provides a cosmetic method which includes a step for applying the agent for preventing and/or improving photoaging and/or dermal pigmentation to skin to be treated, and a cosmetic device to be applied in said method.

FIG. 18

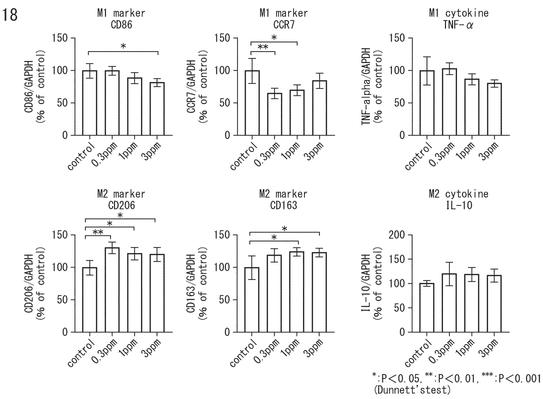


EP 4 140 544 A1

**Description**

FIELD

The present invention relates to an agent for preventing and/or improving photoaging and/or dermal pigmentation, to a cosmetic method using it, and to a cosmetic device to be applied in the method.

BACKGROUND

Aging of human skin is generally classified into the categories of "natural aging" and "photoaging". Photoaging is skin-specific, occurring in a site-specific manner as a result of exposure to light. In photoaging, it is believed that UV rays elicit production of active oxygen and DNA damage in cells, leading to damage to the fibrous tissue of skin and the resulting in appearance of wrinkles and sagging. Some of the phenomena associated with photoaging of skin are reduction in collagen fibers (composed of collagen) and degeneration of elastic fibers (composed of elastin). Melanocytes also suffer damage, resulting in overproduction of melanin pigment which can lead to skin spots.

Pigmentation, including skin spots and loss of skin clarity, occurs due to accumulation of melanin produced by melanocytes in the basal lamina of the epidermis. Melanin is normally present in the epidermis and basal lamina, but the melanin tends to be discharged due to the relatively high turnover of the epidermis. Melanin is often also present in the dermis layer as it accrues in the dermis through gaps in the basal membrane. Because the turnover cycle for dermal cells is much slower than the epidermis, the melanin usually accumulates without being discharged. Consequently it is extremely difficult to ameliorate dermal pigmentation.

As one cosmetic method for anti-photoaging, PTL 1 discloses an agent for preventing or inhibiting skin photoaging by preventing inhibition of leukocyte elastase. PTL 2 discloses a photoaging inhibitor composition comprising a plant extract of Amaranthaceae *Pfaffia*, which has a collagen synthesis-promoting effect, and an animal-derived collagen peptide.

NPL 13 proposes a cosmetic method for improving pigmentation of the dermis, wherein the basal membrane is reinforced, to prevent sinking of melanin into the dermis.

Inflammation has also been suggested as one factor in the photoaging phenomenon of skin, and numerous anti-inflammatory agents have been developed. PTL 3 discloses a cosmetic composition for anti-aging that includes a compound that elicits increased expression of adiponectin and activation of autophagy. It has been suggested to utilize the phagocytotic effect of macrophages to improve dermal pigmentation, and PTL 8 discloses an agent for preventing or ameliorating dermal blemishes by attracting macrophages to fibroblasts that have taken up melanin and fallen into the dermis.

[CITATION LIST]

[PATENT LITERATURE]

[PTL 1] Japanese Patent Publication No. 5657723
[PTL 2] Japanese Unexamined Patent Publication No. 2017-203004
[PTL 3] Japanese Unexamined Patent Publication No. 2018-177805
[PTL 4] Japanese Patent Public Inspection No. 2014-504629
[PTL 5] Japanese Unexamined Patent Publication No. 2015-140334
[PTL 6] Japanese Patent Publication No. 6178088
[PTL 7] Japanese Patent Publication No. 6273304
[PTL 8] Japanese Unexamined Patent Publication No. 2018-072098
[PTL 9] Japanese Patent Publication No. 4781842
[PTL 10] International Patent Publication No. WO2012/057123


[NON PATENT LITERATURE]

[NPL 1] Journal of the American College of Cardiology, Vol. 62, No. 20, 2013, November 12, 2013:1890-901
[NPL 2] Experimental & Molecular Medicine (2014) 46, e70; doi:10.1038/emm.2013.135
[NPL 3] Nature, Vol. 495, 28 March 2013, pp524-530, doi:10.1038/nature11930

[NPL 4] Journal of Investigative Dermatology, 2009 April; 129(4):1016-25. Epub 2008 Oct 9.
[NPL 5] Stem Cell Research & Therapy (2018) 9:88, https://doi.org/10.1186/s13287-018-0821-5
[NPL 6] Journal of the European Academy of Dermatology and Venereology 2011 European Academy of Dermatology and Venereology, 2012, 26, 1577-1580
[NPL 7] British Journal of Dermatology, 2005, 153, pp733-739
[NPL 8] Pigment Cell Melanoma Research, Vol. 27, Issue 3, pp502-504
[NPL 9] Ann Dermatol Vol. 28, No. 3, pp279-289, 2016
[NPL 10] Endocrinology, 2011. 152(10): pp3779-90
[NPL 11] British Journal of Dermatology, 2005. 153 Suppl 2: pp37-46
[NPL 12] Experimental Dermatology, 2011. 20(11): pp953-5
[NPL 13] Fujifilm Research & Development (No. 55-2010):pp33-37
[NPL 14] Nat Immunol, 2014. 15(9): pp846-855
[NPL 15] Cell Metab. 2017 Feb 7; 25(2):412-427
[NPL 16] Theranostics. 2018 Sep 9; 8(17):4620-4632

## SUMMARY

### [TECHNICAL PROBLEM]

It is an object of the present invention to provide an agent for preventing and/or improving photoaging and/or dermal pigmentation, to a cosmetic method using it, and to a cosmetic device to be applied in the method.

### [SOLUTION TO PROBLEM]

As a result of avidly searching for an agent for preventing and/or ameliorating photoaging and/or dermal pigmentation, the present inventors have found that hakushinin (e.g. *Thuja orientalis* L.) extract regulates the M1/M2 balance of macrophages and thereby exhibits an effect of preventing and/or ameliorating photoaging and/or dermal pigmentation. The present invention has been developed on the basis of this finding. Specifically, the invention encompasses the following.

[1] An agent for preventing and/or improving photoaging and/or dermal pigmentation, which contains hakushinin (e.g. *Thuja orientalis* L.) extract as an active ingredient.
[2] The agent according to [1] above, which prevents and/or ameliorates photoaging and/or dermal pigmentation by regulation of M1/M2 balance.
[3] The agent according to [2] above, wherein the regulation of M1/M2 balance is increase in the ratio of M2 with respect to M1.
[4] A cosmetic method for preventing and/or ameliorating photoaging and/or dermal pigmentation of a subject, wherein the method includes:

(a) a process in which an agent according to any one of [1] to [3] is applied to the skin of a subject.

[5] The cosmetic method according to [4] above, which further includes a process of applying weak physical stimulation to the skin, the process of applying physical stimulation comprising application of physical stimulation to the skin of the subject in which a cycle that includes, for example:

(b-1) stretching the skin of the subject to a stretching rate of 0.1% to 50.0%, where the stretching rate is calculated by:
[Mathematical Formula 1]

$$\text{Stretching rate (\%)} = \frac{\text{Distance from fixed point A to fixed point B after application of physical stimulation} - \text{distance from fixed point A to fixed point B before application of physical stimulation}}{\text{Distance from fixed point A to fixed point B before application of physical stimulation}} \times 100 \quad \text{(Formula 1)}$$

(where fixed points A and B are any arbitrary locations on the epidermis or a matrix bonded to the epidermis, a straight line passing through fixed points A and B being parallel to the stretching direction); and
(c-1) restoring the skin of the subject from the stretched state; and/or

(b-2) compressing the skin of the subject by 1 μm to 1000 μm; and
(c-2) restoring the skin of the subject from the compressed state;

wherein the cycle of (b-1) and (c-1), and/or (b-2) and (c-2), is carried out at a vibrational frequency of 60 Hz or lower.
[6] A cosmetic device for application in the cosmetic method according to [5] above, wherein the device comprises:

a stimulation generator that produces physical stimulation, and
a stimulation applicator that applies the physical stimulation generated by the stimulation generator to skin,
the device serving to carry out a process in which weak physical stimulation is applied to skin, and
the process carries out a cycle that includes, for example:

(i-1) stretching the skin to a stretching rate of 0.1% to 50.0%; and
(ii-1) restoring the skin of the subject from the stretched state; and/or
(i-2) compressing the skin of the subject by 1 μm to 1000 μm; and
(ii-2) restoring the skin of the subject from the compressed state;

at a vibrational frequency of 60 Hz or lower,
where the stretching rate is calculated by Formula 1 above.

[ADVANTAGEOUS EFFECTS OF INVENTION]

Application of the present invention can regulate the M1/M2 balance of macrophages, thereby preventing and/or ameliorating photoaging and/or dermal pigmentation.

BRIEF DESCRIPTION OF DRAWINGS

Fig. 1a shows the types of macrophage markers used in Experiment 1, and the ages, skin types and genders of subjects.
Fig. 1b is a photomicrograph showing macrophages (CD11b: red) and M1 macrophages (CD86: green) in skin tissue of young and elderly persons.
Fig. 1c is a photomicrograph showing macrophages (CD11b: red) and M2 macrophages (CD206: green) in skin tissue of young and elderly persons.
Fig. 1d is a graph showing the number of M1 and M2 macrophages and the total number of all macrophages (cells/$mm^2$), in skin tissue of young and elderly persons.
Fig. 1e (left) is a set of photomicrographs showing co-staining of M1 macrophages (CD86: red) or M2 macrophages (CD206: red) and procollagen (green), in skin tissue of young and elderly persons. The rectangular sections in each of the 4 panels at left are magnified portions of particular sections. Fig. 1e (right) is a pair of diagrams schematically illustrating the relationship between M1, M2, fibroblasts and collagen production/breakdown.
Fig. 2a shows in overview the method used in Experiment 2.
Fig. 2b is a photomicrograph of M0, M1 and M2 macrophages derived in Experiment 2.
Fig. 2c is a set of graphs showing gene expression levels of cytokines (IL-1 beta, TNF-alpha and IL-10) produced by M0, M1 and M2 macrophages derived in Experiment 2. The lower part of Fig. 2c shows graphs for expression levels of mRNA of cell surface markers on M1 and M2 macrophages (M1: CD86, M2: CD206). Shown are relative values (%) calibrated against GAPDH mRNA expression level, with respect to M0 as 100%.
Fig. 3a shows in overview the method used in Experiment 3.
Fig. 3b shows procollagen amounts (μg/well) in fibroblasts to which M1 or M2 macrophage supernatant was added in Experiment 3.
Fig. 3c is a set of photomicrographs showing collagen (red) and hyaluronic acid (green) in fibroblasts to which M1 or M2 macrophage supernatant was added in Experiment 3.
Fig. 3d is a set of photomicrographs showing β-galactosidase (β-Gal) in fibroblasts to which M1 or M2 macrophage supernatant was added in Experiment 3.
Fig. 3e is a pair of graphs showing the results of age-related β-galactosidase (SA β-Gal) assay (left: percentage (%) of SA β-gal-positive cells with respect to total number of DAPI-positive cells) in fibroblasts to which M1 or M2 macrophage supernatant was added in Experiment 3, and the total number of DAPI-positive cells per well (right).
Fig. 3f is a set of graphs showing mRNA expression levels of different melanogenesis-promoting factors (HGF, ET1, bFGF, IL-1 alpha, SCF) and melanogenesis-inhibiting factors (clusterin, DKK1), in fibroblasts to which M1 or M2 macrophage supernatant was added in Experiment 3.

Fig. 4a is a set of photomicrographs showing β-Gal in young and elderly fibroblasts to which M1 or M2 macrophage supernatant was added in Experiment 4.

Fig. 4b is a set of graphs showing the results of SA β-Gal assay (percentage (%) of SA β-gal-positive cells with respect to total number of DAPI-positive cells) and the total number of DAPI-positive cells per well, for young (top) and elderly (bottom) fibroblasts to which M1 or M2 macrophage supernatant was added in Experiment 4.

Fig. 4c shows procollagen amounts (μg/well) in young and elderly fibroblasts to which M1 or M2 macrophage supernatant was added in Experiment 4.

Fig. 4d is a set of photomicrographs showing collagen (red) and DAPI-stained nuclei (blue) in young and elderly fibroblasts to which M1 or M2 macrophage supernatant was added in Experiment 3.

Fig. 5 is a set of photomicrographs showing type I collagen (red) and macrophages (CD68: green) in neonatal prepuce-derived fibroblasts to which M1 or M2 macrophage supernatant was added in Experiment 5. The photograph at top left shows fibroblasts cultured without addition of macrophage supernatant.

Fig. 6 is a set of photomicrographs showing macrophages (CD68: red) and M1 macrophages (CD86: green) or M2 macrophages (CD206: green) in a 3D model prepared according to Experiment 6. The triangles indicate portions double stained with CD68 and CD86 or with CD68 and CD206.

Fig. 7 is a set of photomicrographs showing p21 (red) and DAPI-stained nuclei (blue) in a 3D model prepared according to Experiment 6.

Fig. 8 is a pair of graphs showing percentage (%) of number of p21-positive cells with respect to total number of cells in each layer of a 3D model prepared according to Experiment 6 (left: upper layer = epidermal cell layer, right: lower layer = fibroblast layer).

Fig. 9 is a graph showing the number of all macrophages and the numbers of M1 and M2 macrophages in an *ex vivo* skin model irradiated with a solar simulator in Experiment 7. Shown are bars for each number of macrophages without irradiation as 100% (left bar: irradiation (-)), and bars at right as the relative values (%) for the number of macrophages with irradiation (irradiation (+)).

Fig. 10 shows setting of stretching stimulation for Experiment 8.

Fig. 11 shows the device and skin sample used in Experiment 8.

Fig. 12 shows a comparison of M1 and M2 macrophage numbers and total macrophage numbers, for Experiment 8 with stretching stimulation (stretch) applied to an *ex vivo* skin model and without (control). At left is a graph showing the number of macrophages per 1 $mm^2$ of skin sample (cells/1 $mm^2$). At right is a graph showing the percentage (%) of each macrophage type (M1, M2) with respect to the total number of macrophages.

Fig. 13 shows an example of a device of the invention.

Fig. 14a is a set of graphs showing mRNA expression levels of collagen catabolic enzymes (MMP-1, MMP-2) and an inflammatory cytokine (IL-1β) in fibroblasts to which M1 or M2 macrophage supernatant was added in Experiment 9, as relative values (%) with respect to 100% as the results with M1.

Fig. 14b is a set of graphs showing mRNA expression levels of collagen production and maturation factors (COL1A1, COL1A2, HSP47 and ADAMTS-2) in fibroblasts to which M1 or M2 macrophage supernatant was added in Experiment 9, as relative values (%) with respect to 100% as the results with M1.

Fig. 15a shows ages of subjects in a young group and an elderly group from which samples were taken for Experiment 10.

Fig. 15b is a pair of graphs showing the number of M1 and M2 macrophages (cells/$mm^2$) in skin tissue of young and elderly persons (50 μm directly under the basal membrane) for Experiment 10.

Fig. 15c is a pair of graphs showing the number of M1 and M2 macrophages (cells/$mm^2$) in skin tissue of young and elderly persons (200 μm directly under the basal membrane) for Experiment 10.

Fig. 15d is a set of graphs showing the number of CD68-negative cells and M1 and M2 macrophages exhibiting 3/4 collagen positivity (cells/$mm^2$) in skin tissue of young and elderly persons for Experiment 10.

Fig. 16a shows the state of fibroblasts, M1 macrophages and M2 macrophages 24 hours after melanin addition in Experiment 11. The melanin-phagocytic cells had circular forms, as shown at right.

Fig. 16b shows the melanin amounts (melanin/almar blue) per cell, taken up by fibroblasts, M1 macrophages and M2 macrophages 24 hours after melanin addition in Experiment 11.

Fig. 16c shows the state of M1 macrophages and M2 macrophages 5 days after melanin addition in Experiment 11.

Fig. 17 (top) is a graph showing numbers of melanin-phagocytic M1 macrophages and M2 macrophages in the dermis layer, for different age groups, as counted in Experiment 12. Fig. 17 (bottom) shows the average numbers of melanin-phagocytic M1 macrophages and M2 macrophages for all of the subjects.

Fig. 18 shows the results of screening in Experiment 13. The bars in the graphs indicate expression levels of M1 and M2 markers (marker/GAPDH) with hakushinin extract added at different concentrations (0.3 ppm, 1.0 ppm and 3.0 ppm), represented as relative values (%) with respect to 100% for the control (no hakushinin hydrolysis).

Fig. 19a shows the state of immature (M0) THP-1 cells with addition of 3 ppm of non-hydrolyzed hakushinin (control) or 1.0 ppm or 3.0 ppm of hakushinin hydrolysate, 30 minutes after melanin addition, in Experiment 14.

Fig. 19b shows magnified views of Fig. 19a. The black arrows indicate macrophages that have taken up melanin.

Fig. 20 shows the results for (A) oxygen consumption rate (OCR), (B) extracellular acidification rate (ECAR) and (C) OCR/ECAR, based on analysis of intracellular activity of M0 macrophages, M1 macrophages and M2 macrophages using a flux analyzer, in Experiment 15.

Fig. 21 shows the results of using a flux analyzer to analyze the effects on intracellular activity of M0 macrophages 48 hours after addition of hakushinin extract, in Experiment 16. (A) normalized OCR, (B) Basal OCR (OCR value at 3rd A measurement).

Fig. 22 shows the results of using a flux analyzer to analyze the effects on intracellular activity of M0 macrophages 48 hours after addition of hakushinin extract, in Experiment 16. (A) normalized OCR, (B) mitochondrial respiration OCR (difference between 3rd and 12th A measurement).

Fig. 23 shows the results of using a flux analyzer to analyze the effects on intracellular activity of M0 macrophages 48 hours after addition of only unsaturated fatty acids typically present in hakushinin extract, in Experiment 17. (A) Basal OCR, (B) mitochondrial respiration OCR.

Fig. 24 shows the results of using a flux analyzer to analyze the effects on intracellular activity of M0 macrophages 24 hours after addition of different components present in hakushinin extract, in Experiment 18. (A) Basal OCR, (B) mitochondrial respiration OCR.

Fig. 25 shows in overview the method used in Experiment 19.

Fig. 26 shows the results of using a flux analyzer to analyze the effect of hakushinin extract or different components present in hakushinin extract on intracellular activity of M1 macrophages during differentiation, in Experiment 19. (A) Basal OCR, (B) mitochondrial respiration OCR, (C) OCR/ECAR.

Fig. 27 shows expression levels of SDF-1α in M1 and M2 macrophage supernatants. (A) Overview of Experiment 20, (B) amount of SDF-1α in M1 macrophage or M2 macrophage culture supernatant.

Fig. 28 shows effects on SDF-1α expression by skin fibroblasts cultured with addition of M1 or M2 macrophage supernatant, in Experiment 21. (A) Overview of Experiment 21, (B) amount of SDF-1α in cultured fibroblasts 3 days after addition of M1 macrophage or M2 macrophage culture supernatant.

DESCRIPTION OF EMBODIMENTS

Macrophages are cells that are localized in various tissues of the body and elicit an immune response against foreign matter and pathogens, and they are known to be associated with inflammation. Macrophages differentiate from undifferentiated M0 macrophages (M0) into M1 and M2 macrophages. M1 macrophages (M1) are the inflammatory type, while M2 macrophages (M2) are the repair (anti-inflammatory) form. Imbalance between M1 macrophages and M2 macrophages has been reported to be associated with conditions such as obesity, type II diabetes and arteriosclerosis (PTLs 4 to 6, NPLs 1 to 5). However, the relationship between M1/M2 balance and skin photoaging or pigmentation is still unclear.

The present inventors have found that the balance between M1 macrophages and M2 macrophages (M1/M2 balance) is specifically disturbed at light-exposed skin or sites where pigmentation has occurred, and that regulating the M1/M2 balance is extremely important for preventing and ameliorating photoaging or pigmentation of the dermis. The present inventors therefore searched for substances that can prevent or ameliorate photoaging and/or dermal pigmentation, using M1/M2 balance as the index, and having found that hakushinin regulates M1/M2 balance, have developed the present invention which prevents and ameliorates photoaging and/or pigmentation in the skin, and particularly the dermis. It has been reported that hakushinin acts on fibroblasts causing proliferation of fibroblasts, and has effects of promoting collagen and hyaluronic acid production, and of inhibiting melanin production (PTLs 9 and 10). However, the present inventors are the first to have found that hakushinin has an effect of regulating and improving M1/M2 balance.

The present inventors also found that M1/M2 balance can be regulated by applying specific physical stimulation to the skin of a subject. More specifically, it has been found that even in photoaged skin or skin with pigmentation, M1/M2 balance is regulated and improved by applying weak physical stimulation with a specific stretching rate and a vibrational frequency of 60 Hz or lower, such as 1 Hz or lower or 10 Hz or lower, for example.

The invention therefore provides an agent that prevents and/or improves photoaging and/or dermal pigmentation by adjusting M1/M2 balance, as well as a cosmetic method using the same and a cosmetic device to be applied in the method. The method of the invention will often be for the purpose of beautifying, instead of treatment by a doctor or health care professional.

Throughout the present specification, the term "pigmentation" refers to deposition of pigments in the dermis and epidermis, and it includes not only pigmentation by melanin as a result of photoaging, but also artificially injected pigments (such as tattoos). The present invention is effective for both the dermis and the epidermis, but it is particularly promising as a countermeasure against dermal pigmentation in light of limitations of improvement methods other than phagocytosis by melanophages. Application of the present invention improves skin spots, loss of skin clarity and eye circles associated with pigmentation. It is also effective for removing tattoos that are difficult to remove after pigments have been injected into the dermis layer.

M1 macrophages can be measured by markers such as CD86, CD80 and iNOS. M2 macrophages can be measured by markers such as CD206, CD163 and Agr1. CD11b and CD68 are general markers for macrophages including M1 and M2. Measurement may also be by quantifying M1-specific cytokines such as IL-1 beta and TNF-alpha, or M2-specific cytokines such as IL-10, either in addition to or together with the above. However, the markers used are not limited to these so long as they allow measurement of M1/M2 balance.

Throughout the present specification, "M1/M2 balance" may refer to the proportion of the number of M1 macrophages and the number of M2 macrophages, or it may refer to the ratio of the mRNA level of M1 macrophage markers (such as CD86, CD80 or iNOS) to the mRNA level of M2 macrophage markers (such as CD206, CD163 or Agr1). Since the ratio of M1 is high and the proportion of M2 is low in photoaged skin, and M2 macrophages have high melanin phagocytosis activity, regulation and improvement in M1/M2 balance may be achieved by an increase in the proportion of M2 with respect to M1 (number of M2/number of M1, and/or mRNA level of M2 markers/mRNA level of M1 markers). Such an increase may be, for example, an increase in the statistically significant difference (in a Student's t test, for example) at a significance level of 5%, and/or, an increase of 1% or greater, 5% or greater, 10% or greater, 20% or greater, 30% or greater, 40% or greater, 50% or greater, 60% or greater, 70% or greater, 80% or greater, 90% or greater or 100%, for example. Alternatively, regulation or improvement of M1/M2 balance may be bringing the ratio of M2 to M1 (number of M2/number of M1, and/or mRNA level of M2 markers/mRNA level of M1 markers) to within a specified range of, e.g., from about 4/6 to about 9/1, about 5/5 to about 8/2 or about 5/5 to about 7/3, or approaching such a range, or maintaining such a range, or supporting homeostasis centered around such a range.

Throughout the present specification, "M1/M2 balance" may also refer to the balance between intracellular activity of M1 macrophages and intracellular activity of M2 macrophages, and may be represented using, for example, an index of intracellular activity with the most common state of use of M1 macrophages (in the glycolysis state, for example), and/or intracellular activity with the most common state of use of M2 macrophages (in a state of aerobic respiration (mitochondrial respiration), for example). Intracellular activity may be evaluated, for example, by measuring the oxygen consumption rate (OCR value), extracellular acidification rate (ECAR value) or OCR/ECAR value of cells. As used herein, an "increase in the ratio of M2 to M1" may be an increase in the intracellular mitochondria activity for the most common use of M2 macrophages, and for example, it may be an increase in aerobic respiration (mitochondrial respiration) in a group including M1 macrophages and M2 macrophages (for example, aerobic respiration (mitochondrial respiration) being greater in a group including M1 macrophages and M2 macrophages), and/or it may be a decrease in glycolytic activity as anaerobic respiration in a group including M1 macrophages and M2 macrophages, such as an increase in the OCR value in a group including M1 macrophages and M2 macrophages, and/or a decrease in the ECAR value in a group including M1 macrophages and M2 macrophages, and/or an increase in the OCR/ECAR value in a group including M1 macrophages and M2 macrophages. Intracellular activity may be measured by non-invasive and highly sensitive periodic measurement of cells to determine their state of glycolysis and mitochondrial aerobic respiration, as the main energy metabolism pathway of the cells, using an XFe24 extracellular flux analyzer (24-well) by Agilent Technologies (previously Seahorse Bioscience) as a non-limitative example, with the understanding that any device and method may be used for measurement and evaluation of the intracellular activity.

Photoaged skin has a high proportion of M1 and a low proportion of M2, and since M2 is associated with higher phagocytosis of melanin, regulation or improvement of M1/M2 balance may be achieved by increasing the proportion of M2 intracellular activity with respect to M1, such as increasing the OCR value in a group including M1 macrophages and M2 macrophages, and/or reducing the ECAR value in a group including M1 macrophages and M2 macrophages. As used herein, "increase or decrease" may be increase or decrease in the statistically significant difference (in a Student's t test, for example) at a significance level of 5%, and/or increase or decrease of 1% or greater, 5% or greater, 10% or greater, 20% or greater, 30% or greater, 40% or greater, 50% or greater, 60% or greater, 70% or greater, 80% or greater, 90% or greater or 100%, for example.

Hakushinin (or hakushijin) is a galenical obtained by drying seeds of *Platycladus orientalis* Franco, *Thuja orientalis* L. or *Biota orientalis* Endl. of the family Cupressaceae. The hakushinin to be used for the invention is preferably from the seeds (endosperm part), but the whole seeds may also be used since the active ingredient is also included in the whole seeds. The hakushinin may be produced by a common method, or a commercial product may be used.

The method for preparing the hakushinin extract to be used for the invention may be produced with reference to publicly known methods (such as PTLs 9 and 10), although this is not limitative. For example, the method for preparing hakushinin extract to be used for the invention may be a method of alkali treatment of a plant extract obtained by extraction from the plant seeds (hereunder referred to as "first method"), and a method of pulverizing the plant seeds and extraction after maturation for 1 week or longer at a temperature of 10 to 35°C and a relative humidity of 20 to 90% (hereunder referred to as "second method").

The extraction solvent used in the first method and second method may be any volatile solvent commonly used for extraction, and particularly polar or non-polar organic solvents including alcohols such as methanol and ethanol, water-containing alcohols, acetone, ethyl acetate ester, hexane and ethers may be used, either alone or in combinations, but acetone, ethyl acetate and hexane are especially preferred because of they are inexpensive and are easily concentrated under reduced pressure. Extraction may also be accomplished using supercritical carbon dioxide gas. Extraction solvents containing large amounts of water, on the other hand, are not preferred because they inhibit extraction of active ingredients.

Extraction is carried out for a fixed period with a solvent at a mass of about 1 to 100 times and preferably 1 to 30 times the mass of the seeds. The seeds used may be pulverized as necessary and appropriate, but they may also be used without pulverizing when clogging can be problematic during filtration. Repeated extraction with small amounts of solvent is also possible, and a Soxhlet recycler may be used. When extraction is with supercritical carbon dioxide gas, the extraction may be carried out under generally employed conditions of near 40°C and 20 to 40 MPa.

In the first method, the extraction solvent is removed from the extract prior to alkali treatment for hydrolysis of the extract. The alkali treatment is addition of a concentrated aqueous alkali solution in a 0.2- to 2-fold volume to the solvent-removed extract, followed by thorough stirring and mixing, and ageing for about 30 minutes to several days. The temperature during mixing is preferably in the range of room temperature to 70°C, and more preferably in the range of 30 to 60°C. Appropriate stirring is preferably carried out during the ageing to avoid separation of the hydrophilic components and lipophilic components. The concentrated aqueous alkali solution may be a 1 to 10 N aqueous solution of NaOH or KOH.

If the hydrolyzed extract obtained by the alkali treatment is further neutralized with an acid to near neutral, the oily substances will separate out. Oily substances are highly safe for skin while also exhibiting a very high effect against dermal pigmentation. Alcohol, acetone or the like may also be added as appropriate after the alkali treatment in order to promote recovery of the oily substances, and procedures such as decoloration, deodorization, desalting and distillation may also be carried out afterwards as necessary.

In the second method for preparing the hakushinin extract, the seeds are appropriately pulverized or pressed at an early stage of extraction, and allowed to age for a fixed period at near room temperature. The ageing period is not particularly specified so long as it is a range allowing the desired effect to be obtained, but if the ageing period is as short as less than a week it will be difficult to obtain an adequate whitening effect, and conversely if it is as long as several years, problems such as decay and oxidative odor production will occur, and therefore neither case is desirable. If necessary, antioxidant addition or nitrogen exchange may be carried out during the ageing to inhibit production of odors.

Extraction from the aged seeds is carried out using the aforementioned extraction solvent and extraction method. The extraction may also be followed by solvent removal, decoloration, deodorization and distillation, as necessary. The extract obtained by pulverizing of specific seeds, aging and extraction in this manner is highly safe for skin while also exhibiting an effect against dermal pigmentation, similar to the extract obtained by the aforementioned alkali treatment.

According to the invention it is possible to provide an agent for preventing and/or improving photoaging and/or dermal pigmentation comprising an extract prepared from plant seeds by the first method or second method, which is plant-based, safe and has an excellent dermal pigmentation effect, as described above, as well as an external preparation for skin comprising the agent.

The agent of the invention may also be used in combination with any publicly known substances that are known to regulate or improve M1/M2 balance, such as the substances described in PTLs 4 to 7, for example. The route of administration may be selected from among transdermal administration, oral administration, subcutaneous administration, transmucosal administration and intramuscular administration, with transdermal administration being preferred since it allows administration to a specific site of skin for preventing and/or improvement of dermal pigmentation. For pigmentation occurring in the epidermis or dermis, transdermal administration is also sometimes preferred to allow penetration from the skin to the epidermis or dermis. Regulation or improvement of M1/M2 balance may be inducing differentiation to M2 macrophages, or it may be any other method of regulating and improving M1/M2 balance.

For example, an agent or composition used for the invention may regulate or improve M1/M2 balance, resulting in inhibition of photoaging and/or dermal pigmentation. The regulation or improvement in M1/M2 balance, the anti-photoaging agent, the pigmentation inhibiting agent and the agent against dermal pigmentation according to the invention (hereunder these may also be referred to collectively as "agent of the invention") may comprise any one of the aforementioned active ingredients alone, or it may comprise any combination of two or more of them in any proportion.

The agent of the invention may be prepared as a composition comprising the aforementioned active ingredient in combination with one or more other components, such as an excipient, carrier and/or diluent. The constitution and form of the composition may be as desired, and it may be appropriately selected depending on conditions including the active ingredient and the purpose of use. The composition may be prepared by a common method as a formulation in an appropriate combination with an excipient, carrier and/or diluent or other components, depending on the dosage form.

The agent of the invention may also be combined in a cosmetic or the like for humans or animals, or administered as a medical preparation to humans or animals. Different types of foods and beverages or feeds may also be added for ingestion by humans and animals.

When an agent of the invention is to be added to an external preparation for skin such as a cosmetic, drug or quasi drug, the content (dry weight) of the plant body or its extract may be appropriately determined according to the type of plant, the purpose, the form and the method of use. For example, hakushinin may be added at 0.00001% to 50% (in terms of dry mass for an extract or galenical) of the total cosmetic.

In addition to the aforementioned components, there may also be appropriately added, as necessary, components that are commonly used in external preparations for skin such as cosmetics, drugs or quasi drugs, including antioxidants, oils, ultraviolet protecting agents, surfactants, thickeners, alcohols, powder constituents, coloring materials, aqueous components, water and skin nutrient preparations, in ranges that do not interfere with the effect of the invention.

An external preparation for skin according to the invention is not particularly restricted so long as can be used as a cosmetic to be applied to the outer skin, or a quasi drug, and most preferably as a cosmetic, and the dosage form is also not restricted if it can be applied to skin and may be a dosage form such as a solvent-based, solubilized, emulsified or powder-dispersed form, or a water-oil two-layer system, water-oil-powder three-layer system, ointment, cosmetic water, gel or aerosol.

When an agent of the invention is to be used as a cosmetic, it may be used in the form of a cosmetic water, latex, foundation, lipstick, lip cream, cleansing cream, massage cream, pack, hand cream, hand powder, body, shampoo, body, lotion, body, cream or bath cosmetic.

The form of the agent and composition of the invention are not limited to the dosage forms mentioned above, however. The agent or composition of the invention may also be used in conjunction with the device or method of the invention, or with another device or method.

The method, device, agent and composition of the invention may be used for a subject with objective or subjective skin photoaging and/or pigmentation (such as dermal pigmentation), or for a subject desiring to prevent pigmentation. For example, it may be used for a subject judged to have disrupted M1/M2 balance. According to one embodiment, the subject may be a subject judged to have a high degree of pigmentation (such as high dermal pigmentation) using M1/M2 balance in skin as the index. Alternatively, the subject may be a subject concerned with a phenotype specific to photoaging of skin, such as skin spots, wrinkles or sagging, or a subject concerned with epidermis or pigmentation, such as skin spots, loss of skin clarity, bruises or tattoo remnants. Skin spots, wrinkles, sagging, loss of skin clarity, bruises and tattoo remnants can be identified using visual assessment or a publicly known index.

One embodiment of the invention provides a cosmetic method for preventing and/or ameliorating photoaging and/or dermal pigmentation in a subject, wherein the method includes:

(a) a process in which an agent for preventing and/or improving photoaging and/or dermal pigmentation that contains hakushinin extract as an active ingredient is applied to the skin of a subject.

In addition to process (a) above, the cosmetic method according to one embodiment of the invention may also be carried out by applying to the skin a process in which weak physical stimulation is applied to skin, such as weak physical stimulation such as stretching stimulation, pressing stimulation or massaging. For example, the process in which weak physical stimulation is applied to skin may be carrying out a cycle that includes (b-1) stretching skin to a stretching rate of 0.1% to 50.0%; and (c-1) restoring it from the stretched state; at a vibrational frequency of 60 Hz or lower.

The stretching rate is calculated by Formula 1 above. The physical stimulation may be carried out to a stretching rate of 0.001% to 80.0%, 0.01% to 60.0% or 0.1% to 50.0%, and preferably 0.1% to 50.0%. A stretching rate in any arbitrary range may be employed, such as 0.1% to 1.0%, 0.1% to 5.0%, 0.1% to 10.0%, 0.1% to 20.0%, 0.1% to 30.0%, 1.0% to 5.0%, 1.0% to 10.0%, 1.0% to 20.0%, 1.0% to 30.0%, 1.0% to 50.0%, 10.0% to 20.0% or 10.0% to 30.0%, for example.

The stretching speed is the speed (%/s) at which the maximum stretching rate (%) is reached during one cycle. The recovery speed is the speed (%/s) at which it returns to a non-stretched state from the maximum stretching rate. The stretching speed and recovery speed may be any arbitrary speed such as 0.010%/s to 40%/s, 0.05%/s to 30%/s, 0.10%/s to 20%/s, 0.2%/s to 15%/s or 0.3%/s to 10%/s. The stretching speed and recovery speed may be the same or different.

The process in which weak physical stimulation is applied to skin may be carrying out a cycle that includes, for example, (b-2) compressing the skin of a subject by 1 μm to 1000 μm; and (c-2) restoring the skin of the subject from the compressed state; at a vibrational frequency of 60 Hz or lower.

The phrase "compressing the skin by 1 μm to 1000 μm" means that the skin is compressed to a depth of 1 μm to 1000 μm from the outer surface of the skin. The depth of compression may be set as desired, such as 1 μm to 1000 μm, 10 μm to 1000 μm, 10 μm to 300 μm or 10 μm to 100 μm from the outer surface of the skin.

The vibrational frequency is the number of cycles per second, where one cycle is a cycle from the start of stretching or compression to recovery to a non-stretched or non-compressed state. One cycle may also include maintaining a stretched or compressed state for a fixed time period, and/or pausing in a non-stretched or non-compressed state. For example, one cycle may also include: maintaining a stretched or compressed state for 0 seconds to 30 minutes, 1 second to 20 minutes, 5 seconds to 10 minutes or 10 seconds to 5 minutes, either after (b-1) and before (c-1), or after (b-2) and before (c-2); and/or: pausing at the non-stretched or non-compressed state for 0 seconds to 30 seconds, 0 seconds to 20 seconds, 0 seconds to 10 seconds, 1 second to 10 seconds, 1 second to 20 seconds or 1 second to 10 seconds, either after (c-1) and before (b-1) in the next cycle, or after (b-2) and before (c-2). The vibrational frequency may be 0.0000001 Hz to 10 kHz, 0.000001 Hz to 1 kHz or 0.00001 Hz to 100 Hz, and is preferably 0.0001 Hz to 60 Hz or 0.0001 Hz to 10 Hz. For example, a vibrational frequency in any arbitrary range such as 0.001 Hz to 60 Hz, 0.01 Hz to 60 Hz, 0.001 Hz to 10 Hz, 0.01 Hz to 10 Hz, 0.1 Hz to 60 Hz, 0.1 Hz to 10 Hz, 0.5 Hz to 60 Hz, 0.5 Hz to 50 Hz, 0.5 Hz to 10 Hz, 0.5 Hz to 5 Hz, 0.5 Hz to 1 Hz, 0.001 Hz to 0.01 Hz, 0.001 Hz to 0.1 Hz, 0.001 Hz to 1 Hz, 0.01 Hz to 1 Hz, 0.1 Hz to 1 Hz, 1 Hz to 60 Hz, 1 Hz to 10 Hz or 1 Hz to 5 Hz may be employed.

Commercially available facial massagers and other massage equipment use electromagnetic waves with a frequency of about 0.3 to 300 MHz (RF waves), or ultrasonic waves with a frequency of about 1 MHz to 7 MHz. The vibrational frequency used by the method/device of the invention is extremely low compared to these frequency/vibrational frequency ranges. When a strong vibrational frequency is applied to skin as with a conventional facial massager, it can potentially produce adverse effects on skin such as redness, pressure marks, scratches, pain or inflammation, but application in the vibrational frequency range of the invention reduces this risk and allows noninvasive physical stimulation. The present inventors have found that an excessively high stretching rate or vibrational frequency causes overly strong stimulation, and that it is therefore preferred to adjust the values to appropriate ranges to stimulate skin in a more gentle manner.

It has been technical knowledge to those skilled in the art that with the types of cosmetic devices conventionally used in the field, which employ common motor mechanisms, it is only possible to select vibrational frequencies above 60 Hz due to the mechanical mechanisms of the motors. Construction of special machinery has been required to employ vibrational frequencies in the range of the present invention, such as 60 Hz or lower, 10 Hz or lower or 1 Hz or lower, which are vibrational frequencies of "below 60 Hz", considered to be the lower limit for cosmetic equipment in the prior art. In addition, it has been firmly accepted that such low vibrational frequencies are "too low" to exhibit the effect of the invention, for which reason almost no research has been done in this regard. The present inventors nevertheless attempted to actually subject skin to physical stimulation using vibrational frequencies that are extremely low in terms of the common technical knowledge of the prior art and found, surprisingly, that a satisfactory effect was exhibited even with gentle stimulation at such low vibrational frequencies.

While low-to-medium frequency devices are commercially available, such as EMS devices, they are specially designed to act in the deep layers such as muscle or subcutaneous fat, and it is unclear what effects they have on the skin surface layer, as according to the present invention. Such devices often produce prickly stimulation even when a low-frequency current flows, and therefore differ from the present invention which applies more gentle stimulation to skin. The present invention can provide a simple cosmetic method of applying stretching stimulation directly to the skin, without application of energy such as ultrasonic waves, electrical current or a magnetic field. In addition, while stretching stimulation at such a vibrational frequency is gentle, it produces an effect of regulation or improvement of M1/M2 balance, as illustrated in the Examples. Using the method/device of the invention is therefore expected to provide an effect of preventing and/or ameliorating photoaging and/or dermal pigmentation without adverse effects on the skin.

Physical stimulation may be massaging using an instrument such as a facial massager, or an experimental device, or a human hand or instrument, or facial exercising, and it may be either with or without contact. According to one aspect, mechanically generated physical stimulation can be applied to skin using a device that comprises a stimulation generator that produces physical stimulation, and a stimulation applicator that applies the physical stimulation either with or without contact. The physical stimulation may be carried out by contact, such as tension, compression, tapping, pinching or suction of the skin, and/or by non-contact such as causing displacement by application of shock waves to the skin using ultrasonic waves, air pressure or water pressure, for example. Exercising of the face may be inflation of the cheeks or wide opening of the eyes. Massaging may be performed using the hand of the subject undergoing the operation or of an operator such as a cosmetologist, or using an instrument such as a roller. This is not limitative, however, so long as it is within the scope of physical stimulation according to the invention.

For example, the device to be used for the invention may be a cosmetic device comprising a skin-contacting part that contacts with the skin of the user to apply weak physical stimulation according to the invention. It may also comprise a gripping part and a skin stretching part or skin compressing part. For example, the device shown at left in Fig. 13 is designed so that the skin-contacting part contacts with skin, thus stretching the skin with a specific vibrational frequency and stretching rate.

Alternatively, for example, the device of the invention may comprise a power source, a stimulation generator and a skin stimulating part, the power source generating an electrical signal, the stimulation generator converting the electrical signal from the power source to physical stimulation and providing the physical stimulation, and the skin stimulating part receiving the physical stimulation generated by the stimulation generator and applying the physical stimulation to the skin of the user.

The device shown at left in Fig. 13, for example, comprises a gripping part, a power source, a controller that controls the physical stimulation, a stimulation generator, and a skin-contacting part that includes a skin stimulating part and a skin fixing part. The design is such that the user holds the gripping part with the skin-contacting part against the skin and fixes the skin with the skin fixing part, operating the controller to convert electrical signals from the power source to physical stimulation by the stimulation generator, with the physical stimulation being transmitted to the skin stimulating part and causing the skin to be stretched at the specified vibrational frequency and stretching rate by the skin stimulating part while it is fixed by the skin fixing part. For example, the stimulation generator may be driven by a motor to convert electrical signals to physical stimulation. The skin stimulating part shown at left in Fig. 13 applies stretching stimulation to skin, but it may also apply compression stimulation to skin.

Alternatively, the device of the invention may be a cosmetic device comprising a power source, a controller that controls physical stimulation, a stimulation generator, and a skin-contacting part that includes a skin contact surface made of a sheet-like material. An example is the skin-contacting part of the cosmetic device shown at right in Fig. 13. The sheet-like material may be one that allows flow of current to convert electrical signals from a power source into physical stimulation. Such sheet-like materials include Dielectric Elastomer Actuators (DEA), conductive polymers, IPMC, PVC gels and McKinnen-type materials.

The power source of the device of the invention may be an internal power source or external power source, and may also be rechargeable. The device of the invention may use data stored in a cellular phone or cloud, for example, and may be remotely operated in a wireless manner.

The physical stimulation may be such as to cause stretching of the skin by application of the physical stimulation in a contact or non-contact manner as described above. The physical stimulation may be applied parallel to the skin surface, i.e. horizontally, or perpendicular to the skin surface, i.e. vertically, or in any other direction such as diagonally or in a twisted direction.

The number of cycles for the physical stimulation is not restricted. Any number of cycles such as 10 to 500 cycles, 20 to 400 cycles, 30 to 300 cycles, 40 to 200 cycles or 50 to 100 cycles may be carried out, for example. It will often be sufficient to carry out 27 cycles as described in the Examples.

With an arbitrary number of cycles defined as 1 set, an arbitrary number of sets, such as 1 to 100 sets, 2 to 50 sets or 3 to 10 sets may be carried out.

The time period for the physical stimulation is also not restricted. For example, the cycles may be repeated, with or without a pause period, for a fixed period of 5 minutes to 3 hours, 10 minutes to 2 hours or 30 minutes to 1 hour.

The time interval between cycles or between sets is not restricted. For example, stretching or compression stimulation may be carried out in a single set or multiple sets, with one or more sets being carried out once every day, or every 2 days, 3 days, 4 days, 5 days, 6 days or 7 days, or once every week or every 2, 3 or 4 weeks, either continuously or intermittently, and regularly or irregularly.

However, the vibrational frequency, stretching rate, number of cycles and frequency of use are not restricted so long as sufficient stimulation is provided to exhibit an inhibiting effect against photoaging and/or pigmentation of skin. The waveform for physical stimulation may also be set as desired, as a square wave, sine wave, triangular wave or sawtooth wave, for example.

The cosmetic treatment is not particularly restricted and may include any treatment thought to be effective for inhibiting photoaging and/or pigmentation, such as application of a cosmetic containing the agent of the invention and other components, for example. The term "cosmetic" refers to a cosmetic to be applied to skin, such as cosmetic water or a latex, essence, cream, foundation or the like, but there is no limitation to these, and it includes all substances that are not directly for the purpose of improving skin condition but are to be applied onto skin, which also include sunscreens, for example. Alternatively, the cosmetic treatment may be application of physical stimulation such as stretching stimulation, pressing stimulation and massaging, onto skin. The cosmetic treatment may be a single treatment, or it may be continuous treatment conducted over several days or several weeks. The cosmetic treatment may be carried out personally, or at a beauty parlor, cosmetic sales outlet or esthetic salon.

## EXAMPLES

The present invention will now be explained in greater detail by examples. However, the invention is in no way limited by the Examples.

### Experiment 1: Tissue staining

Frozen sections of skin were taken from the eye corners of young (20-30 year old) and photoaged elderly (60-70 year old) Caucasians, having the ages shown in Fig. 1a, and after preparing thin slices, the slices were stained with the following macrophage markers.

(1) M1 macrophage staining: Double staining with goat anti-human CD86 antibody (R&D) and rabbit anti-human CD11b antibody (Abcam) (Fig. 1b).

(2) M2 macrophage staining: Double staining with mouse anti-human CD206 antibody (BD) or mouse anti-human CD163 antibody (Leica), and rabbit anti-human CD11b antibody (Abcam) (Fig. 1c).

(3) Total macrophage staining: Double staining with mouse anti-human CD68 antibody (Abcam) and rabbit anti-human CD11b antibody (Abcam).

The antibody double-positive cells in (1) to (3) up to 200 μm directly under the epidermis were counted as the number for each macrophage type (Fig. 1d). In order to examine the relationship between M1 and M2 macrophages and collagen production, double staining was carried out with goat anti-human CD86 antibody (R&D) and rat anti-procollagen antibody (Millipore), or mouse anti-human CD206 antibody (BD) and rat anti-procollagen antibody (Millipore) (Fig. 1e, left).

The tissue staining of (1) to (3) are shown in Fig. 1b and 1c, a graph of the counted results is shown in Fig. 1d, and the results of double staining with M1 and M2 macrophage antibody and anti-procollagen antibody are shown in Fig. 1e, left. As seen in Fig. 1b, 1c and 1d, more M1 macrophages were observable and M2 macrophages were reduced in the elderly which had photoaging. More specifically, as shown in Fig. 1b, M1 macrophages were present only around the blood vessels in the young, but were dispersed throughout the tissue in the elderly. As shown in Fig. 1c, M2 macrophages were present throughout the tissue in the young, but the numbers were reduced in the elderly. As shown in Fig. 1d, the total number of macrophages (M1 + M2) was the same in the young and elderly, the only difference being the M1/M2 balance. In the young subjects, the ratio of M2 to M1 (M2/M1) was in the range of approximately 5/5 to 7/3, whereas in the elderly subjects the ratio of M2 to M1 was drastically reduced, resulting in a large change in the M1/M2 balance which differed significantly from that of the young subjects.

In the two photographs at top left of Fig. 1e, both the young and elderly subjects show a shift in the location of M1 macrophages and procollagen, but the two photographs at bottom left show the same locations for M2 macrophages and procollagen. This suggests the possibility that M1 promotes breakdown of collagen and M2 acts on fibroblasts to promote collagen production, as shown schematically at right in Fig. 1e.

Experiment 2: Stimulation of M1 and M2 differentiation by THP-1

Following the method described in NPL 1, the human established cell line THP-1 was used to induce differentiation to M1 and M2 macrophages. Specifically, THP-1 was cultured with addition of 1 mM Na Pyruvate (Nakalai), 2 mM L Glutamine (Nakalai) and 10% FBS to RPMI 1640 (Nakalai), by the method shown in Fig. 2a. Next, 100 nM PMA (Abcam) was further added prior to 24 hours of stimulation to cause differentiation to macrophages. For differentiation to M1, 100 ng/mL LPS (Sigma) and 20 ng/mL IFNγ (R&D) were further added prior to 24 hours of stimulation, and for differentiation to M2, 20 ng/ml IL-4 (R&D) and 20 ng/mL IL13 (R&D) were further added prior to 24 hours of stimulation. Observation with a microscope confirmed that the forms had changed as shown in Fig. 2b.

After extracting mRNA from the differentiated or undifferentiated cells, IL-1 beta, TNF-alpha and IL-10 probes (Applied Biosystems) were used for realtime PCR using a TaqMan Gene expression assay, to quantify the expression levels (Fig. 2c, top). PCR was likewise conducted using the same CD86 antibody (R&D) and CD206 antibody (BD) used in Experiment 1 for quantification of the expression levels (Fig. 2c, bottom). The values were each calibrated as GAPDH mRNA expression levels.

As shown in Fig. 2c, the macrophages differentiated by the method described in Experiment 2 produced M1-associated inflammatory cytokines (IL-1 beta and TNF-alpha) and an M2-associated anti-inflammatory cytokine (IL-10). The differentiation-induced macrophages all had increased expression of the M1 and M2 macrophage surface markers CD86 and CD206. The results confirmed that differentiation had been successfully induced. M1 and M2 macrophages differentiated by the method of Experiment 2 and undifferentiated M0 macrophages were therefore used for the following Experiments 3 and 4.

Experiment 3: Experiment adding M1 and M2 macrophage supernatants to fibroblasts (1)

As illustrated in Fig. 3a, the supernatant was removed from THP-1 differentiated to M1 or M2 by the same method as Experiment 2, or undifferentiated as M0, and washed once with PBS, after which medium was added and culturing was carried out for 48 hours. The supernatants containing the M1 or M2 secretions (inflammatory or anti-inflammatory cytokines) were added to neonatal fibroblasts. Cells with added RPMI 1640 (Nakalai) were used as the control. After addition of the supernatant, the fibroblasts were cultured for 72 hours and the amount of procollagen in each supernatant was quantified with a PIP ELISA kit (TAKARA) (Fig. 3b). The cell fraction was stained with rabbit anti-human collagen antibody (Cederlane) and biotinylated hyaluronic acid bonded protein (Hokudo) (Fig. 3c). Using β-gal as an index of aging, the cell nuclei were stained with DAPI and then the intracellular β-gal was stained using a Senescence Detection Kit (Abcam) (Fig. 3d), and the number of β-gal-positive cells and DAPI-positive cells were counted (Fig. 3e).

In order to examine the contribution of M1 macrophages and M2 macrophages to melanin production, the macrophage supernatants were each added to fibroblasts and culturing was carried out, in the same manner as above. The fibroblasts were collected, the mRNA was recovered, and HGF, ET1, bFGF, IL-1 alpha, SCF and clusterin probes were used in real time PCR for quantification of the mRNA expression levels (Fig. 3f).

The results are shown in Figs. 3b to 3f. Fig. 3b shows procollagen amounts after addition of each macrophage (M1 or M2) supernatant to fibroblasts, and culturing for 72 hours. Fig. 3c shows localization of collagen and hyaluronic acid in fibroblasts after addition of each macrophage (M1 or M2) supernatant and culturing for 72 hours. From Figs. 3b and 3c it is seen that M1 markedly inhibits collagen production. Fig. 3d shows intracellular β-gal in fibroblasts after addition of each macrophage (M1 or M2) supernatant and culturing for 72 hours. Fig. 3d suggests that β-gal-positive cells increase with M1, thereby promoting aging, whereas M2 inhibits aging. Fig. 3e shows a graph with the count of β-gal-positive cells and DAPI-positive cells. At right are shown the total counts of DAPI-positive cells per well. The graph at right in Fig. 3e suggests that M2 not only inhibits aging of cells but also promotes proliferation of cells. At left in Fig. 3e is a graph showing the proportion (%) of β-gal-positive cells to total DAPI-positive cells, which suggests that M1 has an effect of promoting aging and cell death, while M2 has an effect of inhibiting aging and promoting cell proliferation. As shown in Fig. 3f, in terms of mRNA expression of melanogenesis-associated factors, M1 tended to increase melanin production while M2 tended to inhibit melanin production. As reported in NPL 6, fibroblasts are known to secrete factors such as SCF and HGF under light stimulation by UV and the like, thus leading to cell death. As reported in NPLs 7 to 9, fibroblasts also secrete factors such as HGF, ET1, bFGF, SCF and clusterin under light stimulation, acting either directly or indirectly on melanocytes to produce melanin. It is therefore suggested that cell death and melanin production induced by light stimulation is a result of disturbance in the M1/M2 balance.

Experiment 4: Experiment adding M1 and M2 macrophage supernatants to young and elderly fibroblasts

In order to confirm whether the anti-aging effect of M2 macrophages is useful for fibroblasts, i.e. whether it results in rejuvenation, M1 and M2 macrophage supernatants were added to young and elderly cells, and the difference in effects of the macrophage supernatants was examined based on the difference in fibroblast age.

Specifically, each macrophage supernatant collected by the same method as Experiment 3 was added to neonatal human prepuce fibroblasts (young fibroblasts) and 68-year-old human fibroblasts (elderly fibroblasts), and culturing was carried out for 72 hours. After subsequent β-gal and DAPI staining by the same method as Experiment 3, the numbers of β-gal-positive cells and DAPI-positive cells were counted (Figs. 4a and 4b). In addition, M1 and M2 supernatants were added to the young and elderly fibroblasts in the same manner as Experiment 3, the fibroblasts were cultured for 72 hours, and the procollagen levels in the supernatants were quantified with a PIP ELISA kit (Takara) (Fig. 4c), and stained with rabbit anti-human collagen antibody (Cederlane) and DAPI (Fig. 4d).

Fig. 4a shows β-gal staining images. Fig. 4b shows graphs with the results of the numbers of β-gal-positive cells and the total numbers of cells per well, for the young fibroblasts and elderly fibroblasts. Fig. 4a (bottom) shows that even with the elderly cells, addition of M2 supernatant markedly reduced β-gal-positive cells and inhibited aging. Fig. 4b shows that, regardless of age, aging was promoted by M1 but inhibited by M2. This is also supported by the results shown in Figs. 4c and d, wherein regardless of age, cells to which M1 supernatant was added had markedly lower collagen production compared to M2 supernatant.

Experiment 5: Experiment with co-culturing of M1 macrophages, M2 macrophages and neonatal prepuce fibroblasts

A comparison was made between the amount of collagen produced when neonatal prepuce fibroblasts were cultured in RPMI 1640 (Nakalai) with the same number of M1 or M2 macrophages (prepared by the method of Experiment 2), and when neonatal prepuce fibroblasts were cultured in RPMI with half the number of M1 or M2 macrophages, based on staining with rabbit anti-human collagen antibody (Cederlane). The macrophages were visualized by simultaneous staining with mouse anti-human CD68 antibody (Abcam).

The results are shown in Fig. 5. In the neonatal prepuce fibroblasts as well, addition of M1 supernatant reduced collagen while addition of M2 resulted in increased collagen. In other words it was found that disturbance in M1/M2 balance also affects collagen in young cells.

Experiment 6: Effect of M1/M2 balance disturbance using 3D skin model

In order to examine the effects of M1/M2 macrophages on epidermal cells and fibroblasts, three types of 3D skin models with prepared, as shown in the following table.

[Table 1]

| | M1 model | M2 model | Control model |
|---|---|---|---|
| Upper layer | Epidermal cell layer | | |
| Intermediate layer | Fibroblast + gel + M 1 layer | Fibroblast + gel + M2 layer | Fibroblast + gel layer |
| Lower layer | Fibroblast layer | | |

The 3D skin models were prepared in the following manner. Human dermal fibroblasts ($0.2 \times 10^6$) were seeded in a cell culture insert (12 mmφ, porous membrane mean pore size: 0.4 μm), and 200 μM of magnesium ascorbate-2-phosphate (APM) and 10% FBS-DMEM were used for one week of culturing with medium exchange once every two days. After dispensing 0.5% type I collagen-10% FBS-DMEM solution containing human dermal fibroblasts as a control model, and additionally dispensing 30,000 M1 macrophages or M2 macrophages differentiated by the method of Experiment 2, to the solution, as M1 or M2 model, each of the collagen gels was formed on the human dermal fibroblasts and culturing was carried out for 1 to 5 days.

Epidermal keratinocytes dispersed in Humedia-KG2 medium (Kurabo Industries, Ltd.) were seeded on collagen gel at $5 \times 10^5$/well, medium comprising a mixture of Humedia-KG2 and 10% FBS-DMEM at 1:1 with addition of 200 μM APM was added outside the insert to the same liquid level height as the inner side, and culturing was carried out for 3 days.

After then removing the insert or the medium inside the glass ring, skin model medium (with mixture of 10% FBS-DMEM and Humedia-KG2 EGF (-) at 1:1 and prepared to 1.8 mM Ca), with 200 μM APM, 10 μM N-hydroxy-2-[[(4-methoxyphenyl)sulfonyl]3-picolyl)amino]-3-methylbutaneamide hydrochloride (CGS27023A (MMP inhibitor)) and 10 μM BIPBIPU (heparanase inhibitor), was added on the outer side to the height of the bottom of the insert, and gas-liquid boundary culturing was carried out with the insert interior exposed to air. Culturing was carried out for 2 weeks with medium exchange once every 2 to 3 days.

After fixing the cultured skin model with 4% PFA/PBS (Nakarai), it was stained with anti-CD206 antibody (Abcam), anti-CD68 antibody (Abcam) and anti-CD86 antibody (Abcam) by the same method as Experiment 1, and the presence of M1 and M2 macrophages was confirmed (Fig. 6). After then staining with anti-p21 antibody (Abcam) and DAPI (Vector), the number of cells stained with DAPI in the epidermal cell layer and the fibroblast layer were counted as the total number of cells, among which the number of cells also stained with anti-p21 antibody were counted as the number of p21-positive cells. The percentage of the number of p21-positive cells with respect to the total number of cells was calculated by the following formula.

[Mathematical Formula 2]

$$\text{Percentage of p21-positive cells among total cells} = (\text{p21-positive cell count}/\text{total cell count}) \times 100\ (\%)$$

The results are shown in Figs. 7 and 8. As shown in the photographs, in the M1 model (M1), the p21-positive cells were increased with respect to the control model (cont) in both the upper epidermal cell layer and the lower fibroblast layer in contact with the intermediate layer, whereas they were decreased in the M2 model (M2). This tendency matched the results of Experiment 3 which used monolayer culture. This suggests that even in a 3D skin model used to more closely approximate human skin, M1 macrophages have an effect of promoting aging and cell death while M2 macrophages have an effect of inhibiting aging and cell death.

Experiment 7: Experiment irradiating sunlight onto *ex vivo* model using human skin

NativeSkin (skin taken from 38-year-old female) by Genoskin was cultured for 1 day after arrival with the included culture solution. On the following day, an optical filter was placed in a 1000 W solar simulator by Oriel and UVA and UVB alone were irradiated at 11.5 J/cm$^2$, after which culturing was continued with the included culture solution (irradiation (+)). A sample without irradiation was used as the control (irradiation (-)). The sample was collected five days after irradiation, M1 macrophages, M2 macrophages and total macrophages were stained in the same manner as Experiment 1, and the numbers were plotted on a graph.

The results are shown in Fig. 9. The M1 count increased most with irradiation using a solar simulator. The increase in M2 count, however, was much lower than M1. In other words, light stimulation disrupted the M1/M2 balance causing an increased proportion of M1.

Experiment 8: Experiment of stretching stimulation on *ex vivo* model using human skin

A method of regulating or improving M1/M2 balance was investigated next.

Sample: NativeSkin (skin taken from 38-year-old female) by Genoskin was used (6-well size, diameter: about 2 to 2.5 cm).

Stretching conditions: A stretching instrument was constructed, having a gripping part for gripping both ends of the skin in the wells, as shown in Fig. 11, and causing stretching of the skin by pulling of the gripping part. The wells containing the tissue strips were placed horizontal, the gripping part was operated to stretch the skin from both ends, and stretching was carried out at a speed of 10%/s to a 10% stretching rate as shown in Fig. 10, after which the samples were restored to their original non-stretched state at a recovery speed of 10%/s. With this procedure as one cycle, a total of 90 cycles were carried out over a period of 30 minutes. With 90 cycles as one set, a total of 3 sets (total of 270 cycles) were carried out over a period of 3 hours with a pause period of 30 minutes to 1 hour between sets. A sample without stretching stimulation was used as the control.

Observation method: Skin samples with and without stretching stimulation were stained for M1 macrophages, M2 macrophages and total macrophages by the same method as Experiment 1, except that staining for total macrophages was carried out using only rabbit anti-human CD11b antibody (Abcam), and the counts were measured. The percentage (%) of each macrophage type (M1, M2) with respect to the total number of macrophages was determined and plotted on a graph.

The results are shown in Fig. 12. In comparison to the control, the sample with stretching stimulation had very little change in total macrophage count and M1 count, but a highly increased M2 count. In other words, stretching stimulation improved the M1/M2 balance.

Experiment 9: Effects of M1 and M2 macrophage supernatants on collagen

In order to examine the contribution of M1 macrophages and M2 macrophages to collagen breakdown in addition to Experiment 3, each macrophage supernatant was added to fibroblasts and cultured by the same method as Experiment 3, and the mRNA was collected after 72 hours. MMP-1, MMP-2 and IL-1$\beta$ probes (Taqman probes by Applied Biosystems) were used as indexes of collagen breakdown, and real time PCR was carried out to quantify the mRNA expression level for each (Fig. 14a).

In order to examine the contribution of M1 macrophages and M2 macrophages to collagen production and maturation, each macrophage supernatant was added to fibroblasts and cultured by the same method as Experiment 3, and the mRNA was collected after 72 hours. COL1A1, COL1A2, HSP47 and ADAMTS-2 probes (Taqman probes by Applied Biosystems) were used as indexes of collagen production and maturation, and real time PCR was carried out to quantify the mRNA expression level for each (Fig. 14b).

Figs. 14a and 14b confirmed that M1 contributes to collagen breakdown while M2 contributes to collagen production and maturation.

Experiment 10: Collagen breakdown and production in *ex vivo* model using young and elderly human skin

Skin from the temple of young (age 20 to 30) and elderly (aged 60 to 80) Caucasians, having the ages listed in Fig. 15a, was cut into frozen sections and sliced in the same manner as Experiment 1, and then anti-procollagen antibody (Millipore), which stains the 3/4 collagen fragment, anti-fragmented collagen antibody (AdipoGen), and the same CD68, CD11b, CD206 and CD86 antibodies used in Experiment 1 were used to count positive cells by the same method as Experiment 1.

The results are shown in Figs. 15b, 15c and 15d. The proportion of M1 macrophages was higher and the proportion of M2 macrophages was lower in the elderly group compared to the young group. Regardless of age, however, the number of macrophages exhibiting 3/4 collagen positivity tended to be greater compared to fibroblasts exhibiting 3/4 collagen positivity, and the number of M2 macrophages exhibiting 3/4 collagen positivity tended to be greater than M1 macrophages exhibiting 3/4 collagen positivity.

Experiment 11: Experiment *in vitro* showing uptake of melanin by dermis

THP-1 cells were differentiated to M1 macrophages and M2 macrophages by the same method as Experiment 3. Melanin solution (Sigma Corp., Melanin-BioReagent, Synthetic, suitable for cell culture) was dissolved in PBS and adjusted to 0.02% W/V, and added to the differentiated M1 macrophages, M2 macrophages and fibroblasts (Kurabo Industries, Ltd.). After 24 hours, the cells were washed with PBS and photographed with a microscope, and the cells were collected. The number of collected cells was counted with Alamar Blue (Life Technologies) and the melanin content was calculated and quantified in the following manner.

Melanin quantitation:

Alamar Blue was added to each cell medium (macrophages: RPMI 1640, fibroblasts: 1DMEM) adjusted to 1:10, and then culturing was carried out at 37°C for 30 minutes. The supernatant was then collected at 100 μl/well, and the fluorescence was measured with an Ascent (Thermo) with excitement/emission: 544 nm/590 nm. After measurement, washing was performed with PBS and 1 M NaOH was added, after which the mixture was incubated at room temperature for 3 hours for complete lysis. The cell solution was measured using a POWERSCAN HT (DS Pharma Biomedical) at OD475.

The results are shown in Figs. 16a to 16c. The "melanin/almar blue" units in the graph represent a relative value ((2)/(1)), where (1) is the intensity for cultured cells stained with Alamar Blue and with measurement of the supernatant using fluorescence at 544 nm/590 nm, and (2) is the intensity after lysing the cells, decomposing the melanin and measuring at an absorbance of 475 nm, and it represents the melanin content per cell. These show that M2 cells phagocytose very large amounts of melanin. This tendency was already seen after 24 hours, but the difference became even more apparently observable after continuing cell culturing to more than 5 days (Fig. 16c).

Experiment 12: Experiment *ex vivo* showing uptake of melanin by dermis

Since Experiment 11 showed that M2 macrophages phagocytose larger amounts of melanin than fibroblasts or M1 macrophages, an LSM880 (Carl Zeiss) was used for *ex vivo* observation of the dermis layer of human skin counted in Experiment 11. Both the elderly and young M2 macrophages were stained black with melanin as observed in a bright field, but melanin was not present near the M1 macrophages. This serves as *ex vivo* indication that M2 macrophages in the dermis take up more melanin than fibroblasts or M1 macrophages, and their numbers were therefore counted and plotted on a graph (Fig. 17).

As seen in Fig. 17, the numbers of M2 macrophages taking up melanin were greater than the numbers of M1 macrophages, both in elderly and young, and therefore little difference in age was observed. These results suggest that uptake of melanin in the dermis is by M2 macrophages, and that consequently increasing the proportion of M2 macrophages can rectify M1/M2 balance to prevent and/or ameliorate photoaging and/or dermal pigmentation.

Experiment 13: Search for M1 reducer/M2 inducer

Upon using the different markers shown in Fig. 18 to regulate or improve M1/M2 balance and searching for agents that can prevent and/or ameliorate photoaging and/or dermal pigmentation, hakushinin was found to have a powerful M2-inducing effect. The hakushinin extract used here (non-hydrolyzed or hydrolyzed) was prepared according to Japanese Patent Publication No. 4781842(PTL 9) or International Patent Publication No. WO2012/0571243 (PTL 10), by the following procedure. It is to be understood, however, that the procedure for preparing hakushinin extract to be used for the invention is not limited to the following.

<Preparation of hakushinin extract>

Hakushinin seeds (non-pulverized, outer shells removed as necessary) were immersed in a 5-fold volume (v/w) of acetone, ethyl acetate or hexane and extracted for 10 days at room temperature. The residue was removed beforehand with a nylon mesh (100 mesh), and filtration was carried out with filter paper. After removing the solvent from the filtrate using a rotary evaporator, 0.5 to 15 N NaOH was added as NaOH in a range of 100 to 300 g/l of extract, while stirring with a stirring blade (temperature: 50°C). Treatment was carried out for 5 hours while stirring at 200 rpm. After then gradually adding 5N $H_2SO_4$ to lower the pH to near 1 while stirring, the separated oily substance was collected. An equivolume of water was added to the collected oily substance for washing to remove the impurities, salts and excess acid. The oily substance was dried under reduced pressure and subjected to alkali treatment (for hydrolyzed hakushinin extract). As a comparative control, the extract prior to alkali treatment was used (for non-hydrolyzed hakushinin extract).

THP-1 cells were used in the M0 undifferentiated state and cultured overnight at 37°C. Next, the non-hydrolyzed (non-alkali treated) hakushinin (control) dissolved in a solvent (DMSO) was added at 3 ppm, or the hakushinin hydrolysate was added at 0.3 ppm, 1 ppm or 3 ppm, and culturing was carried out for two nights at 37°C. The cells were recovered and the RNA was extracted, and the expression levels of CD86, CCR7, TNF-alpha, CD206, CD163, IL-10 and GAPDH were quantified by real time PCR, dividing the expression level of each gene by the expression level of GAPDH. As a result, the hakushinin hydrolysate lowered CD86 gene and TNF-α gene expression levels while increasing CD206 gene and IL-10 gene levels, in a concentration-dependent manner. This indicated that hakushinin hydrolysate has an effect of inhibiting M1 induction and promoting M2 induction (Fig. 18).

Experiment 14: Melanin phagocytosis-increasing effect of hakushinin

Non-hydrolyzed (non-alkali treated) hakushinin (control) dissolved in a solvent (DMSO) was added at 3 ppm, or hakushinin hydrolysate was added at 1.0 ppm or 3.0 ppm, to immature (M0) THP-1 cells. Culturing was carried out at 37°C, melanin solution (Sigma Corp.) was added after 48 hours, and the mixture was observed with a microscope (Bio Station CT (Nikon)).

The results are shown in Figs. 19a and 19b. The hakushinin-added macrophages began to take up melanin about 30 minutes after addition of melanin (black arrows in Fig. 19b). After another 3 hours, the control still had many cells that had not taken up melanin, whereas all of the cells of the hakushinin hydrolysate-added sample had taken up melanin.

Since photoaging was shown to increase the proportion of M1 (Experiment 7), and a higher proportion of M2 tended to result in a greater amount of skin collagen (Experiments 9 and 10) and a higher pigment phagocytosis effect (Experiments 11 and 12), this suggests that hakushinin which was screened as a M2-inducing agent, together with weak physical stimulation according to the invention which has an effect of improving M1/M2 balance, has a high effect of inhibiting pigmentation in the dermis.

Experiment 15: Macrophage intracellular activity

M0 macrophages, M1 macrophages and M2 macrophages induced to differentiate from THP-1 by the same method as Experiment 2 were measured for intracellular activity using an XFe24 (24-well) extracellular flux analyzer by Agilent Technologies (previously Seahorse Bioscience) (hereunder referred to as "flux analyzer"), with reference to Nat Immunol, 2014. 15(9): pp846-855 (NPL 14). A flux analyzer is a device that allows non-invasive, highly sensitive periodic measurement of cells to determine the state of glycolysis as the main cellular energy metabolism pathway, and aerobic respiration by mitochondria.

Using a flux analyzer, oligomycin (ATP synthase inhibitor) ("Oligo"), FCCP (uncoupling agent) and rotenone (mitochondrial complex I inhibitor) + antimycin A (mitochondria complex III inhibitor) ("Rot + Ant") were added at predetermined times and the oxygen consumption rate (OCR) and extracellular acidification rate (ECAR) were measured. The ECAR and OCR values used were the basal OCR and basal ECAR values at 18.3 minutes after measurement. Each measured value was calibrated by the number of cells determined by counting the cell nuclei stained by addition of Hoechst.

The results showed that respiration activity by mitochondria was higher in the order: M2 macrophages, M0 macrophages, M1 macrophages (Fig. 20).

Experiment 16: Effect of hakushinin extract on intracellular activity of macrophages

M0, M1 and M2 differentiated from THP-1 were prepared by the same method as Experiment 2, and hakushinin extract (hakushinin hydrolysate) prepared by the same method as Experiment 13 was added to the M0 (at 0.3 ppm, 1 ppm or 3 ppm). At 48 hours after incubation, the oxygen consumption rate (OCR) was measured using a flux analyzer. Each measured value was calibrated by the number of cells determined by counting the cell nuclei stained by addition of Hoechst (Fig. 21).

As a result it was found that addition of hakushinin extract significantly increased the Basal OCR value 18.3 minutes after the start of measurement (measured value for 3 points) (Fig. 21), and the mitochondrial respiration value (the value of the Basal OCR at 18.3 minutes after the start of measurement minus non-mitochondrial respiration 95.1 minutes after the start of measurement (non-mitochondrial oxygen consumption)) (Fig. 22).

Experiment 17: Effect of different components in hakushinin extract, on intracellular activity of macrophages (1)

Hakushinin extract is known to contain unsaturated fatty acids such as the ω3 fatty acids linolenic acid and juniperonic acid, and the ω6 fatty acid linoleic acid. It has been reported that unsaturated fatty acids (EPA, DHA) act on M1 inflammation related genes, thus inhibiting inflammation (NPL 15 (Cell Metab. 2017 Feb 7; 25(2):412-427)). The effects of the unsaturated fatty acids in hakushinin extract on the intracellular activity of macrophages was investigated.

M0 and M2 differentiated from THP-1 were prepared by the same method as Experiment 2, linoleic acid, linolenic acid or juniperonic acid (3 ppm) was added to M0, and after 48 hours the oxygen consumption rate (OCR) was measured with a flux analyzer by the same method as Experiment 15. Each measured value was calibrated by the number of cells determined by counting the cell nuclei stained by addition of Hoechst.

As a result, the values for Basal OCR and mitochondrial respiration for the M0 macrophages by addition of linoleic acid, linolenic acid and juniperonic acid in the hakushinin extract were not significantly different from the control which had addition of the solvent (DMSO) alone (Fig. 23).

Experiment 18: Effect of different components in hakushinin extract, on intracellular activity of macrophages (2)

The Basal OCR and mitochondrial respiration values for M0 were examined with the incubation time changed to 24 hours after addition of the hakushinin extract or unsaturated fatty acid. The experiment method was carried out by the same method as described in Experiment 17, except that the incubation time was changed to 24 hours after addition of the hakushinin extract or unsaturated fatty acid.

As a result, no increase was seen in the Basal OCR and mitochondrial respiration values 24 hours after addition of the hakushinin extract and each unsaturated fatty acid (Fig. 24).

Experiment 19: Effect of different components in hakushinin extract, on intracellular activity of M1-differentiated macrophages

A flux analyzer was used to analyze the change in intracellular activity with addition of hakushinin extract or an unsaturated fatty acid (linoleic acid, linolenic acid or juniperonic acid) in the step of further inducing differentiation to M1 from M0 cells differentiated from THP-1 by the same method as Experiment 2 (Fig. 25).

The oxygen consumption rate (OCR value) and extracellular acidification rate (ECAR value) were measured by the same method shown in Fig. 15 using a flux analyzer, 24 hours after addition of hakushinin extract or an unsaturated fatty acid during further differentiation to M1 from M0 cells differentiated from THP-1.

As a result, Basal OCR and mitochondrial respiration were only significantly increased when hakushinin extract was added (Figs. 26(A) and (B)). A strong inhibiting effect on the ECAR value, and thus increased OCR/ECAR value, was observed only with linolenic acid, which is reported to have a powerful anti-inflammatory effect.

Mitochondrial respiration in macrophages during M1 differentiation was significantly increased by hakushinin extract, but no significant increase was found using only the unsaturated fatty acids that are present in hakushinin extract, suggesting that the effect of increasing mitochondrial respiration may be a specific effect attributable to mixture of the different components in hakushinin extract.

Experiment 20: Confirmation of SDF-1$\alpha$ in M1 and M2 macrophage supernatants

It has been reported that SDF-1$\alpha$ (CXCL12) expression levels are lower in fibroblasts present below blemished areas of skin, and that the reduction in SDF-1 levels may therefore be related to formation of blemishes in skin (NPL 16 (Theranostics. 2018 Sep 9; 8(17):4620-4632)). The expression levels of SDF-1$\alpha$ (CXCL12) in M1/M2 macrophages themselves were therefore examined.

After differentiation of THP-1 to M1 and M2 by the same method as Experiment 2, the supernatants were removed and washed once with PBS, after which RPMI (0.5% FBS) was added and culturing was carried out for 24 hours, and the SDF-1$\alpha$ (CXCL12) levels in the supernatants were determined using a cytokine array (RayBiotech) (Fig. 27(A)).

As a result, the M2 supernatant was shown to have more SDF-1$\alpha$ (CXCL12) than the M1 supernatant (Fig. 27(B)).

Experiment 21: Experiment adding M1 and M2 macrophage supernatants to fibroblasts (2)

The effects of factors produced by M1 and M2 macrophages on expression of SDF-1$\alpha$ (CXCL12) in fibroblasts were examined (Fig. 28(A)).

After differentiation of THP-1 to M1 and M2 by the same method as Experiment 2, the supernatants were removed and washed once with PBS, and then RPMI (10% FBS, 500 $\mu$M ascorbic acid) was added and culturing was carried out for 48 hours. The supernatants containing the M1 or M2 secretions (inflammatory or anti-inflammatory cytokines) were added to skin fibroblasts. Cells with only RPMI (10% FBS, 500 $\mu$M ascorbic acid) added were used as the control. After addition of supernatant, the fibroblasts were cultured for 72 hours. The fibroblasts were then collected and the mRNA was recovered and analyzed for SDF-1$\alpha$ (CXCL12) expression level using RNA-seq (Fig. 28(A)).

The RNA-seq lead was mapped to the human genome (hg19) using STAR. After filtering out the low expression genes, the obtained count data were corrected for sample size and converted to log2. The log2 (CPM) values calculated for each of the samples by the method described above were compared between the two groups by quasi-likelihood F-test, using a glmQLFit and glmQLFTest as R package edgeR functions, and were then corrected by multiple comparison test using the Benjamini-Hochberg method to calculate the q-value.

The results showed that the M1 supernatant-added fibroblasts had notably reduced SDF-1a expression level (Fig. 28(B)).

The results described above suggest that regulating or ameliorating M1/M2 balance can prevent and/or ameliorate photoaging and/or dermal pigmentation. Regulation or improvement in M1/M2 balance is achieved by applying stretching stimulation or compression stimulation, or by applying hakushinin, whereby it is expected that photoaging and/or dermal pigmentation can be prevented and/or improved.

## Claims

1. An agent for preventing and/or improving photoaging and/or dermal pigmentation, which contains hakushinin extract as an active ingredient.

2. The agent according to claim 1, which prevents and/or ameliorates photoaging and/or dermal pigmentation by regulation of M1/M2 balance.

3. The agent according to claim 2, wherein the regulation of M1/M2 balance is increase in the ratio of M2 with respect to M1.

4. A cosmetic method for preventing and/or ameliorating photoaging and/or dermal pigmentation of a subject, wherein the method includes:

   (a) a process in which an agent according to any one of claims 1 to 3 is applied to the skin of a subject.

5. The cosmetic method according to claim 4, which further includes a process in which weak physical stimulation is applied to the skin, the process of applying physical stimulation comprising application of physical stimulation to the skin of the subject in which a cycle that includes, for example:

   (b-1) stretching the skin of the subject to a stretching rate of 0.1% to 50.0%, where the stretching rate is calculated by:
   [Mathematical Formula 1]

$$\text{Stretching rate (\%)} = \frac{\text{Distance from fixed point A to fixed point B after application of physical stimulation} - \text{distance from fixed point A to fixed point B before application of physical stimulation}}{\text{Distance from fixed point A to fixed point B before application of physical stimulation}} \times 100 \quad \text{(Formula 1)}$$

   (where fixed points A and B are any arbitrary locations on the epidermis or a matrix bonded to the epidermis, a straight line passing through fixed points A and B being parallel to the stretching direction); and
   (c-1) restoring the skin of the subject from the stretched state; and/or
   (b-2) compressing the skin of the subject by 1 μm to 1000 μm; and
   (c-2) restoring the skin of the subject from the compressed state;

   wherein the cycle of (b-1) and (c-1), and/or (b-2) and (c-2), is carried out at a vibrational frequency of 60 Hz or lower.

6. A cosmetic device for application in the cosmetic method according to claim 5, wherein the device comprises:

   a stimulation generator that produces physical stimulation, and
   a stimulation applicator that applies the physical stimulation generated by the stimulation generator to skin,
   the device serving to carry out a process in which weak physical stimulation is applied to skin, and
   the process carries out a cycle that includes, for example:

   (i-1) stretching the skin to a stretching rate of 0.1% to 50.0%; and
   (ii-1) restoring the skin of the subject from the stretched state; and/or
   (i-2) compressing the skin of the subject by 1 μm to 1000 μm; and
   (ii-2) restoring the skin of the subject from the compressed state;

   at a vibrational frequency of 60 Hz or lower,
   where the stretching rate is calculated by Formula 1 above.

FIG. 1a

- M1 macrophage marker :CD86
- M2 macrophage markers :CD206, CD163
- Total macrophage markers :CD11b, CD68
- Sample: Caucasian, eyelids

| | Age | Gender |
|---|---|---|
| Young | 27 | female |
| | 33 | female |
| | 34 | female |
| | 36 | female |
| Elderly | 64 | female |
| | 66 | female |
| | 69 | female |
| | 70 | female |
| | 75 | female |

FIG. 1b

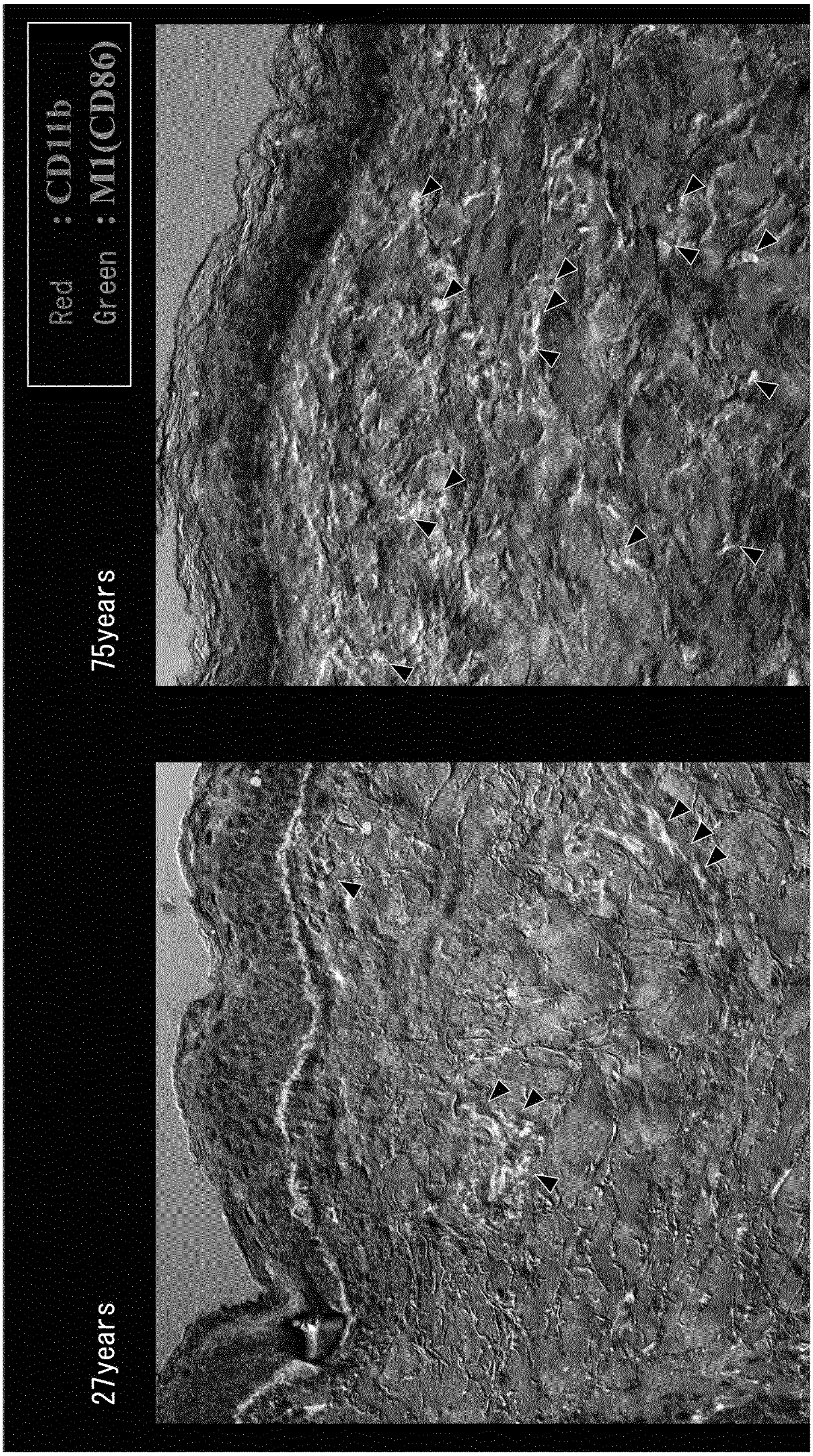

FIG. 1c

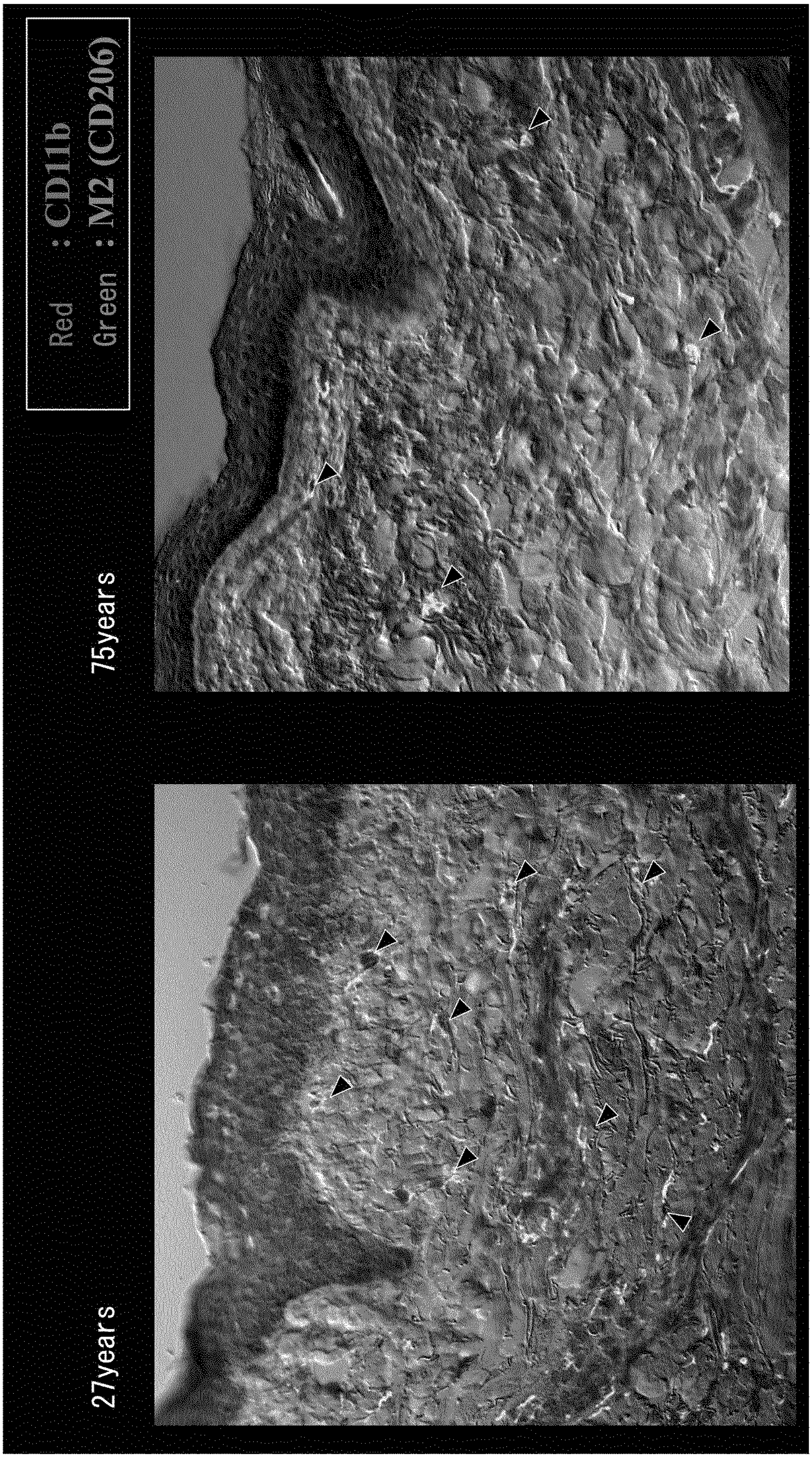

FIG. 1d

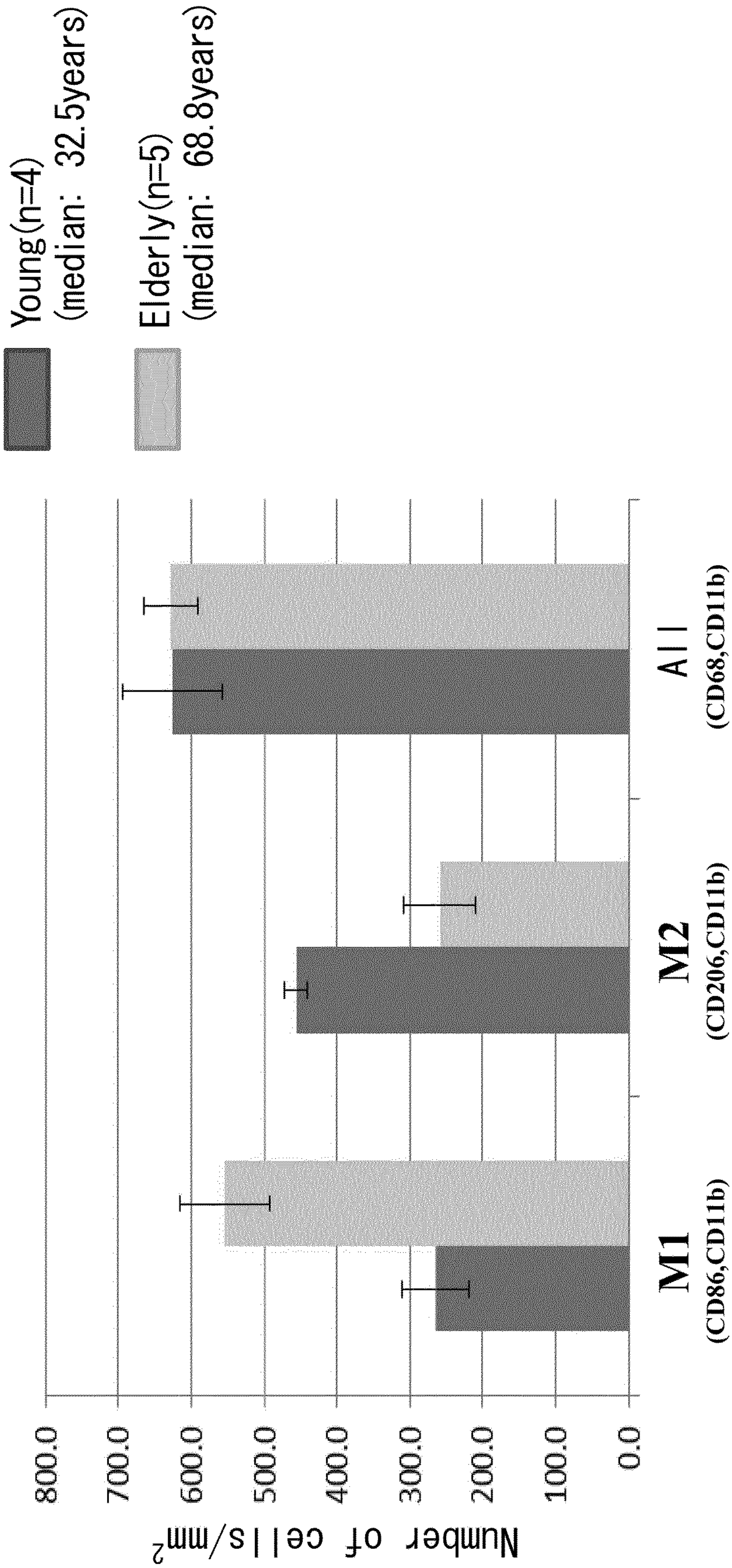
Young(n=4)
(median: 32.5years)
Elderly(n=5)
(median: 68.8years)
Number of cells/mm²
800.0
700.0
600.0
500.0
400.0
300.0
200.0
100.0
0.0
M1
(CD86,CD11b)
M2
(CD206,CD11b)
All
(CD68,CD11b)

FIG. 1e

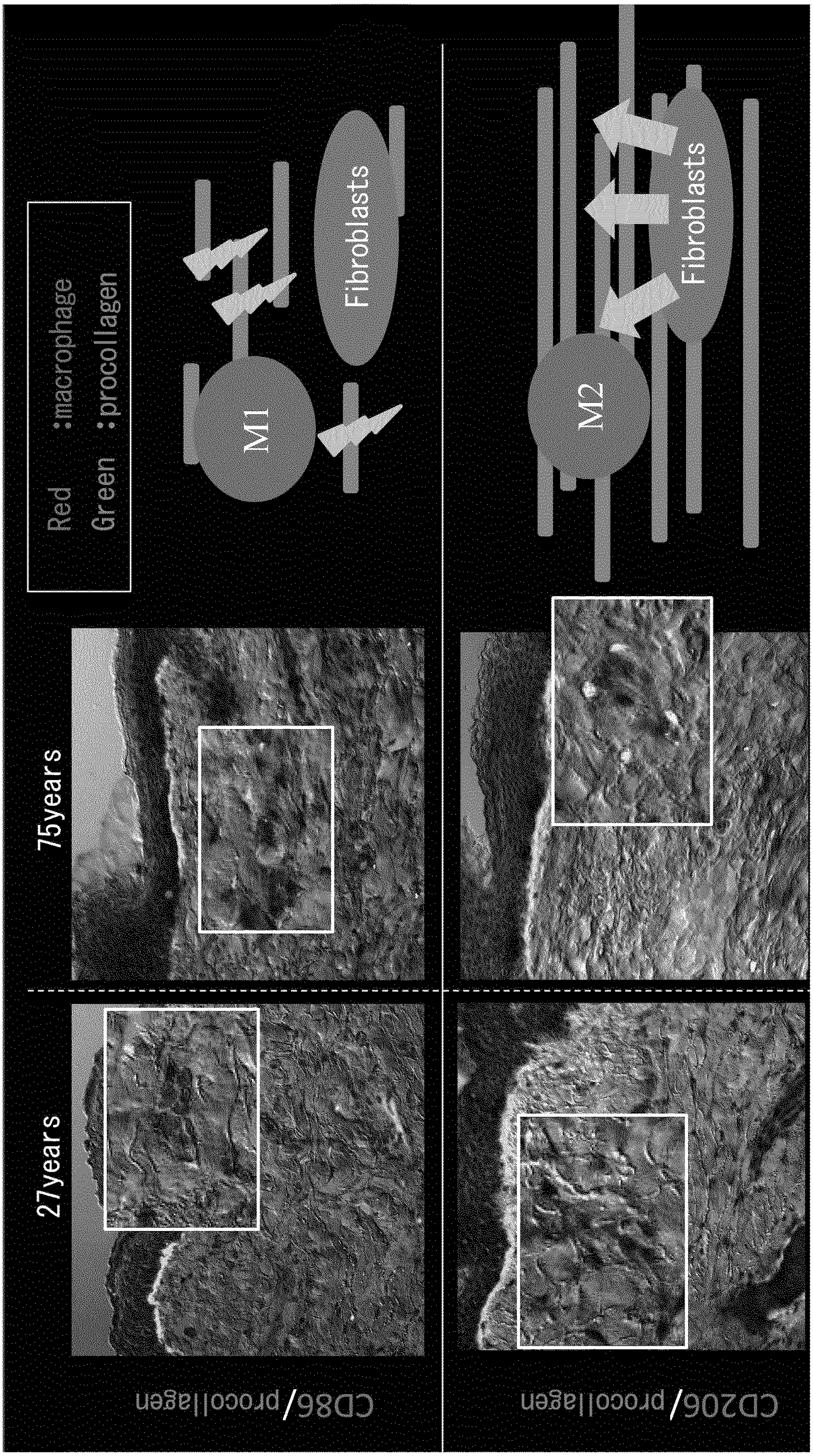
27years
75years
Red :macrophage
Green :procollagen
M1
Fibroblasts
M2
Fibroblasts
CD86/procollagen
CD206/procollagen

FIG. 2a

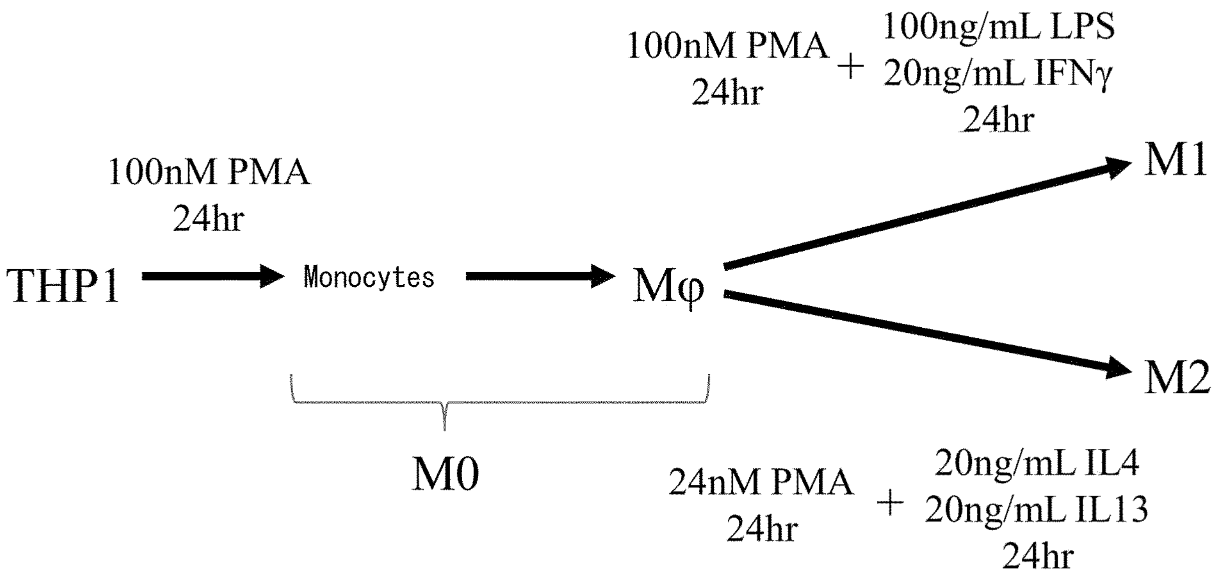
100nM PMA
24hr
+
100ng/mL LPS
20ng/mL IFNγ
24hr
M1
100nM PMA
24hr
THP1
Monocytes
Mφ
M2
M0
24nM PMA
24hr
+
20ng/mL IL4
20ng/mL IL13
24hr

FIG. 2b

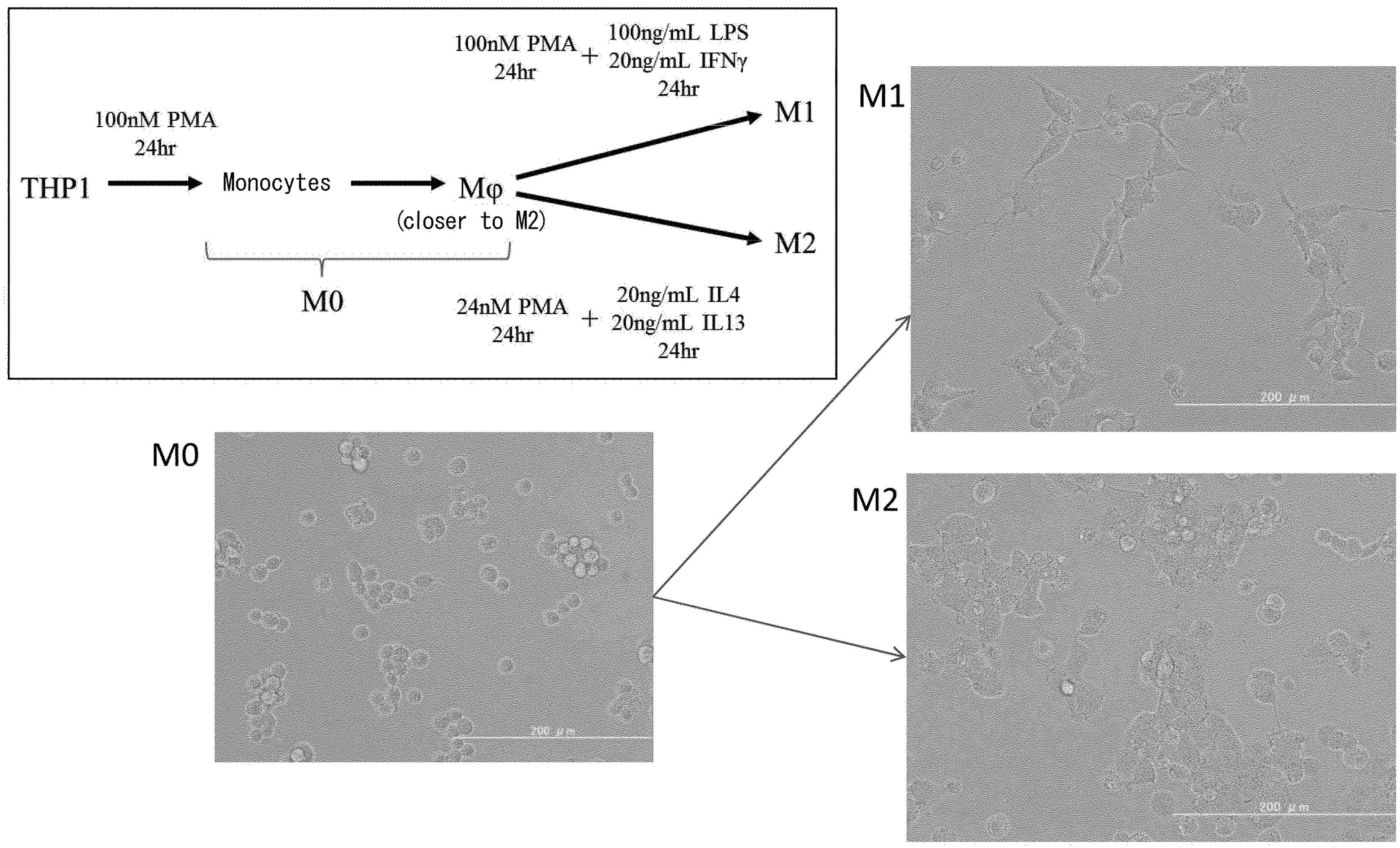
100nM PMA
24hr
THP1
Monocytes
Mφ
(closer to M2)
M0
100nM PMA
24hr
+
100ng/mL LPS
20ng/mL IFNγ
24hr
M1
M2
24nM PMA
24hr
+
20ng/mL IL4
20ng/mL IL13
24hr
M0
M1
M2
200 μm

FIG. 2C

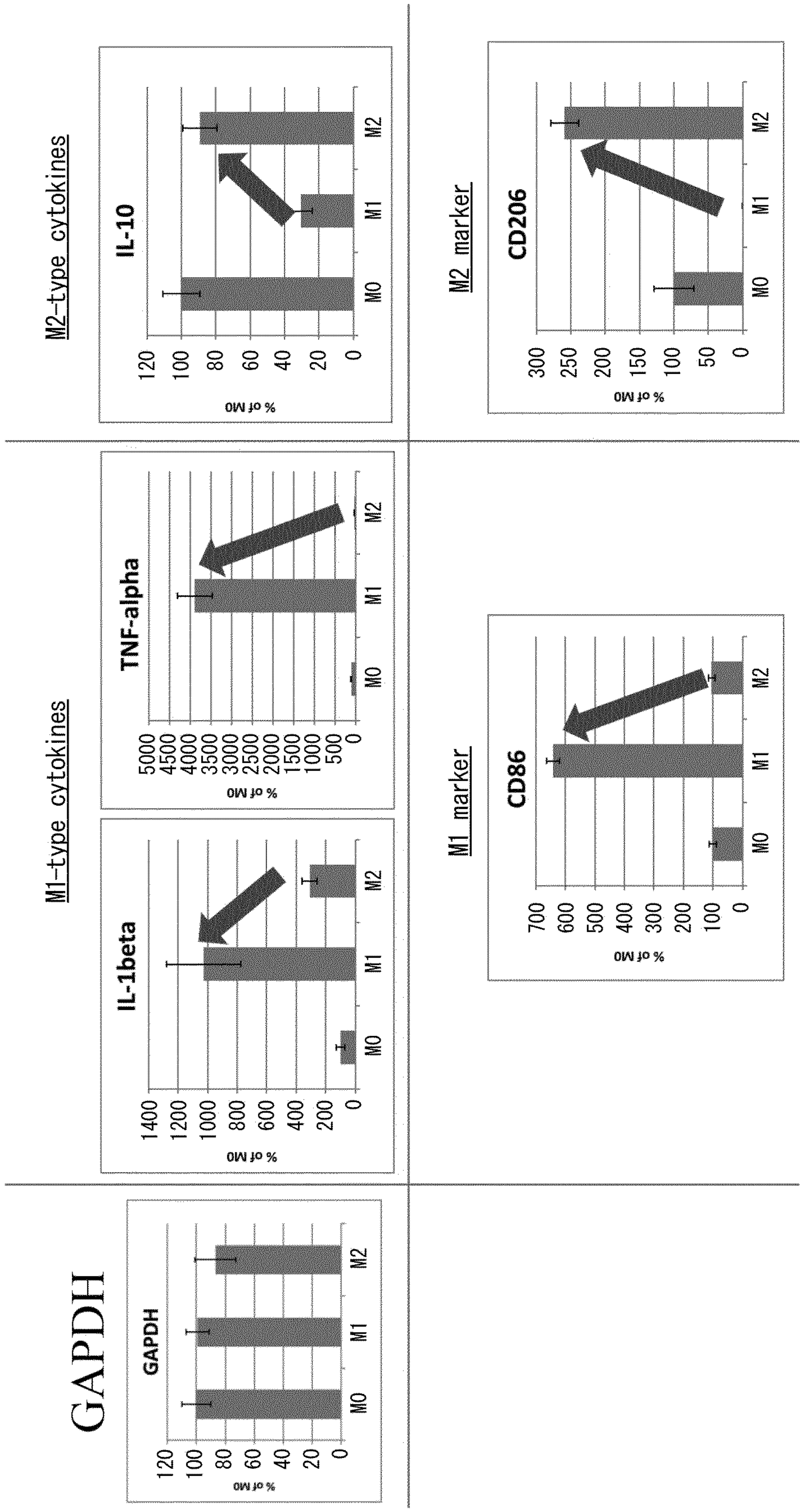
GAPDH
M1-type cytokines
IL-1beta
TNF-alpha
M2-type cytokines
IL-10
M1 marker
CD86
M2 marker
CD206
% of M0
M0
M1
M2

FIG. 3a

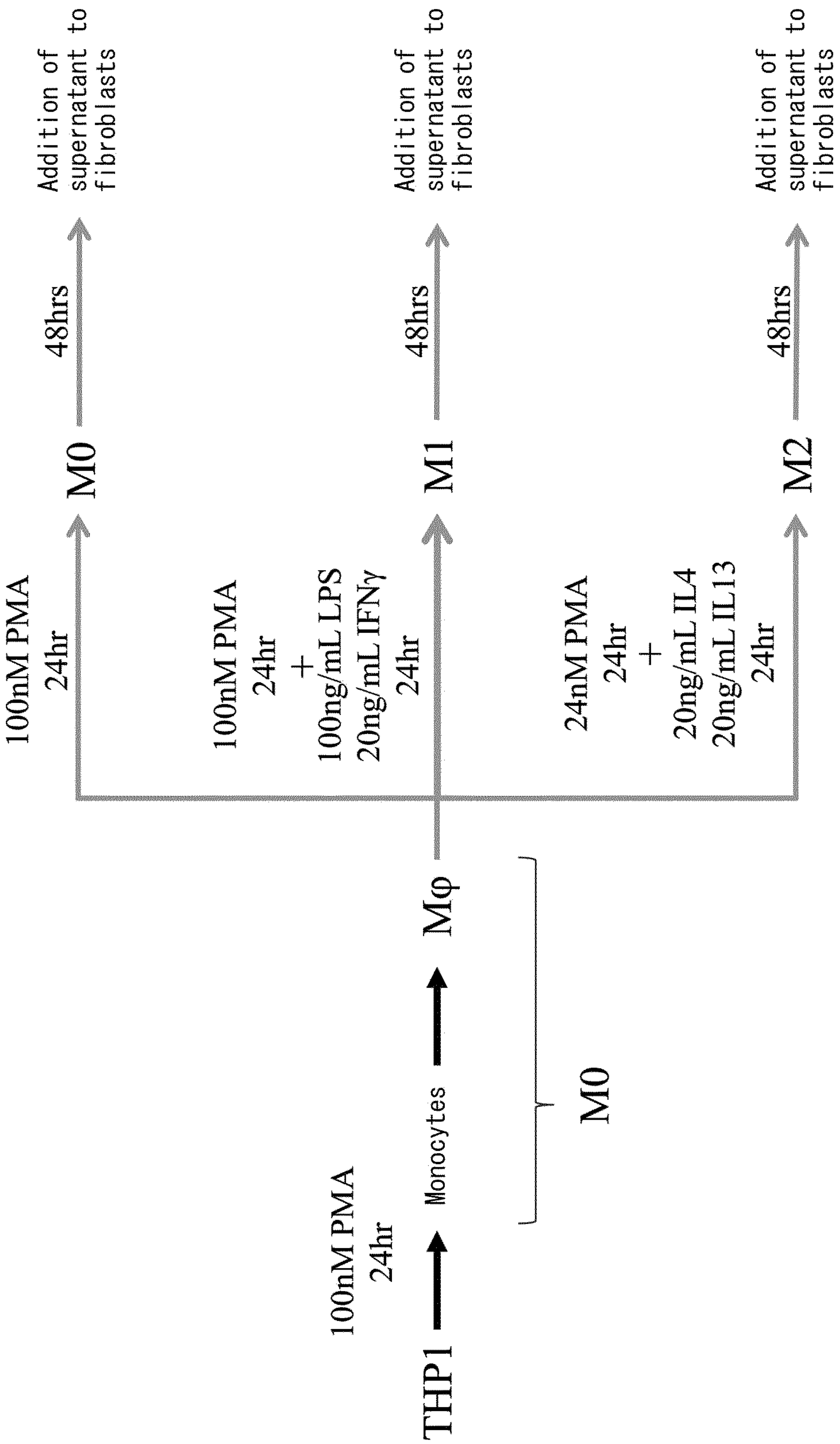
THP1
100nM PMA
24hr
Monocytes
Mφ
M0
100nM PMA
24hr
M0
48hrs
Addition of supernatant to fibroblasts
100nM PMA
24hr
+
100ng/mL LPS
20ng/mL IFNγ
24hr
M1
48hrs
Addition of supernatant to fibroblasts
24nM PMA
24hr
+
20ng/mL IL4
20ng/mL IL13
24hr
M2
48hrs
Addition of supernatant to fibroblasts

FIG. 3b

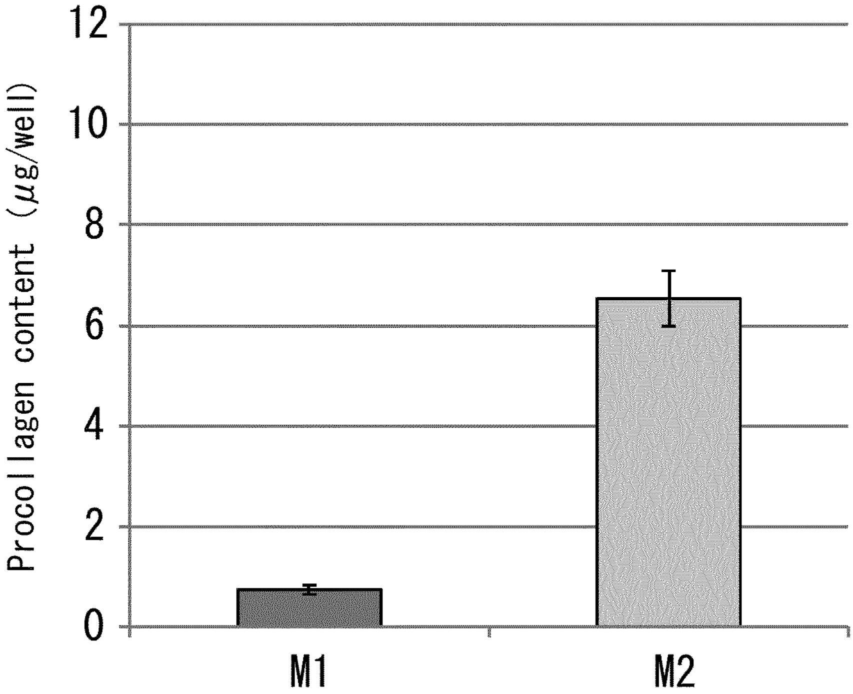

FIG. 3C

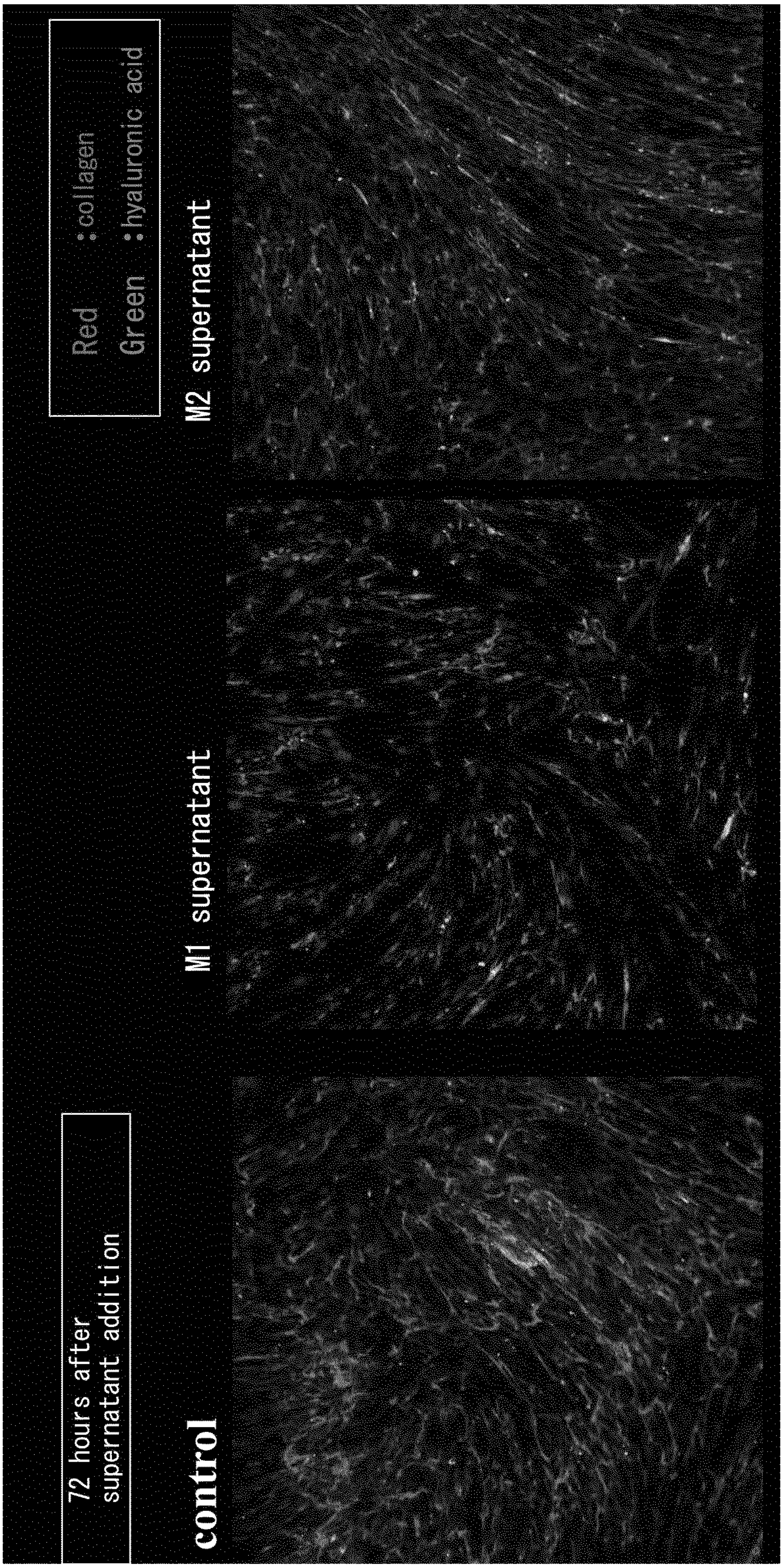
72 hours after
supernatant addition
control
M1 supernatant
M2 supernatant
Red :collagen
Green :hyaluronic acid

FIG. 3d

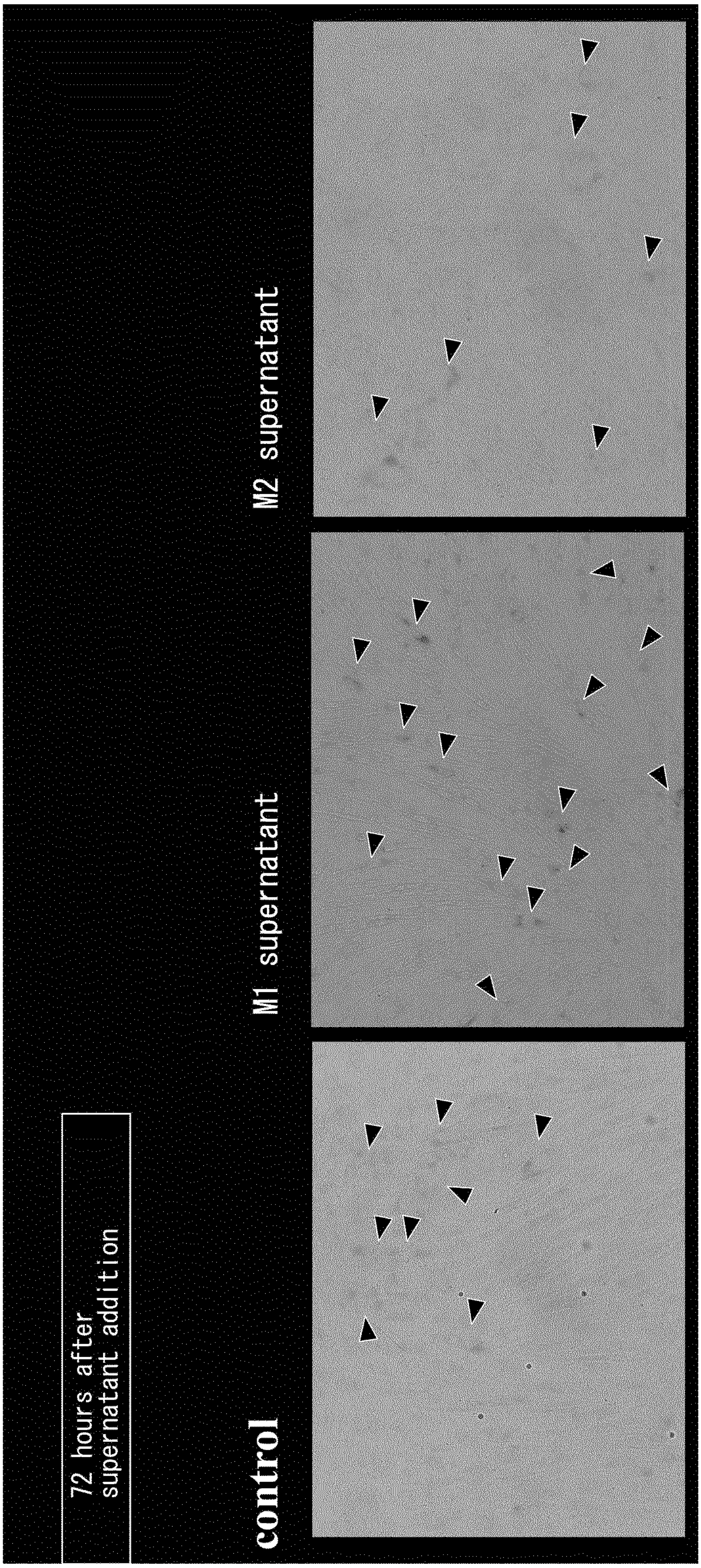
72 hours after
supernatant addition
control
M1 supernatant
M2 supernatant

FIG. 3e

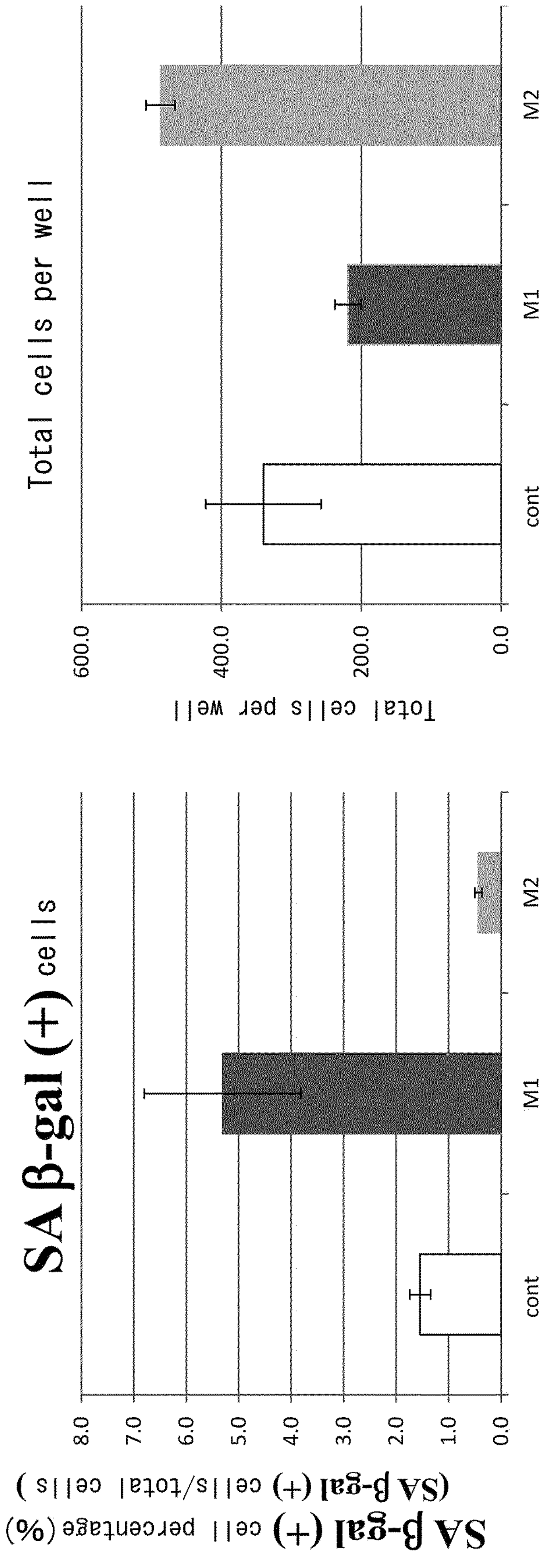
SA β-gal (+) cells
SA β-gal (+) cell percentage (%)
(SA β-gal (+) cells/total cells)
8.0
7.0
6.0
5.0
4.0
3.0
2.0
1.0
0.0
cont
M1
M2
Total cells per well
Total cells per well
600.0
400.0
200.0
0.0
cont
M1
M2

FIG. 3f

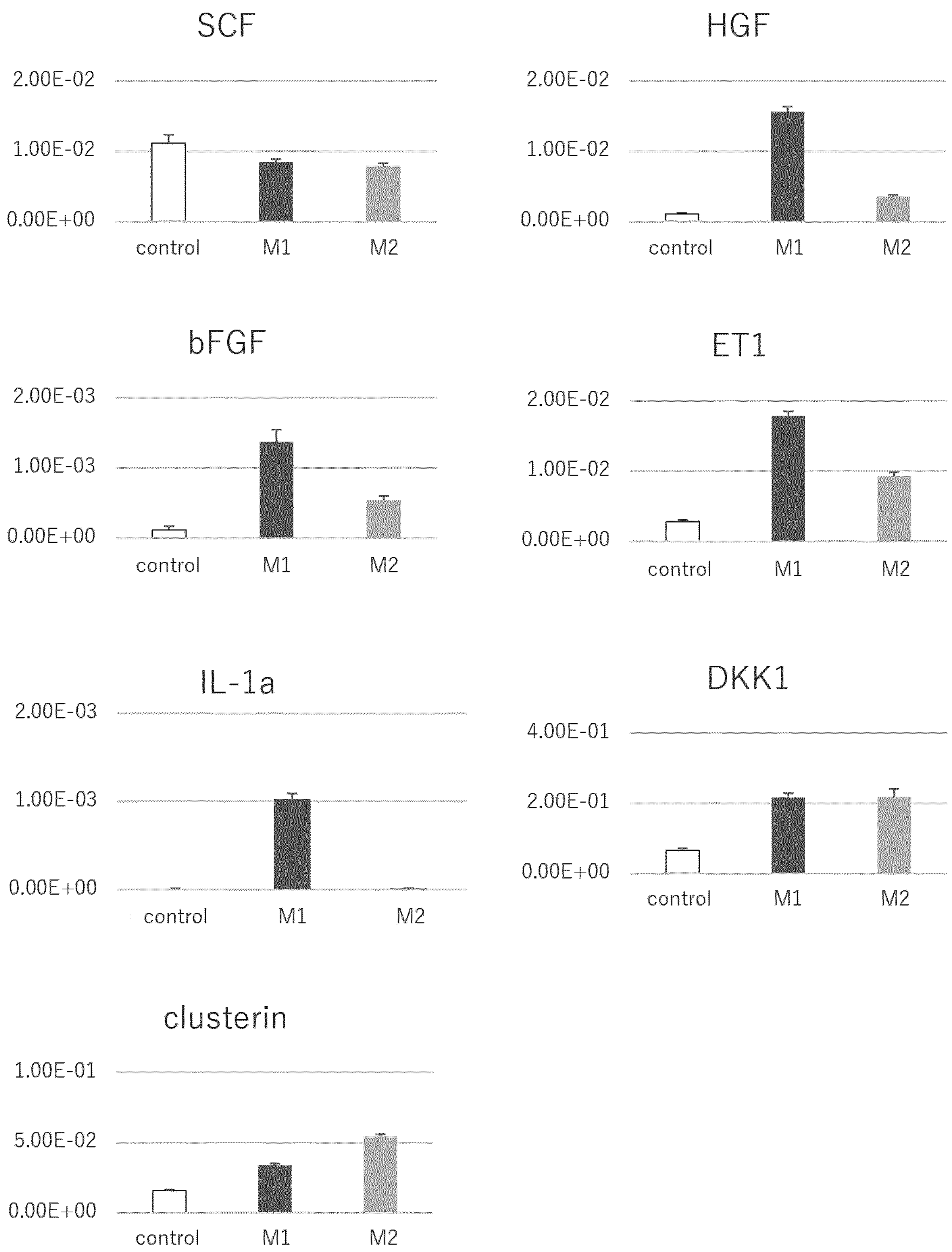
SCF
2.00E-02
1.00E-02
0.00E+00
control
M1
M2
HGF
2.00E-02
1.00E-02
0.00E+00
control
M1
M2
bFGF
2.00E-03
1.00E-03
0.00E+00
control
M1
M2
ET1
2.00E-02
1.00E-02
0.00E+00
control
M1
M2
IL-1a
2.00E-03
1.00E-03
0.00E+00
control
M1
M2
DKK1
4.00E-01
2.00E-01
0.00E+00
control
M1
M2
clusterin
1.00E-01
5.00E-02
0.00E+00
control
M1
M2

FIG. 4a

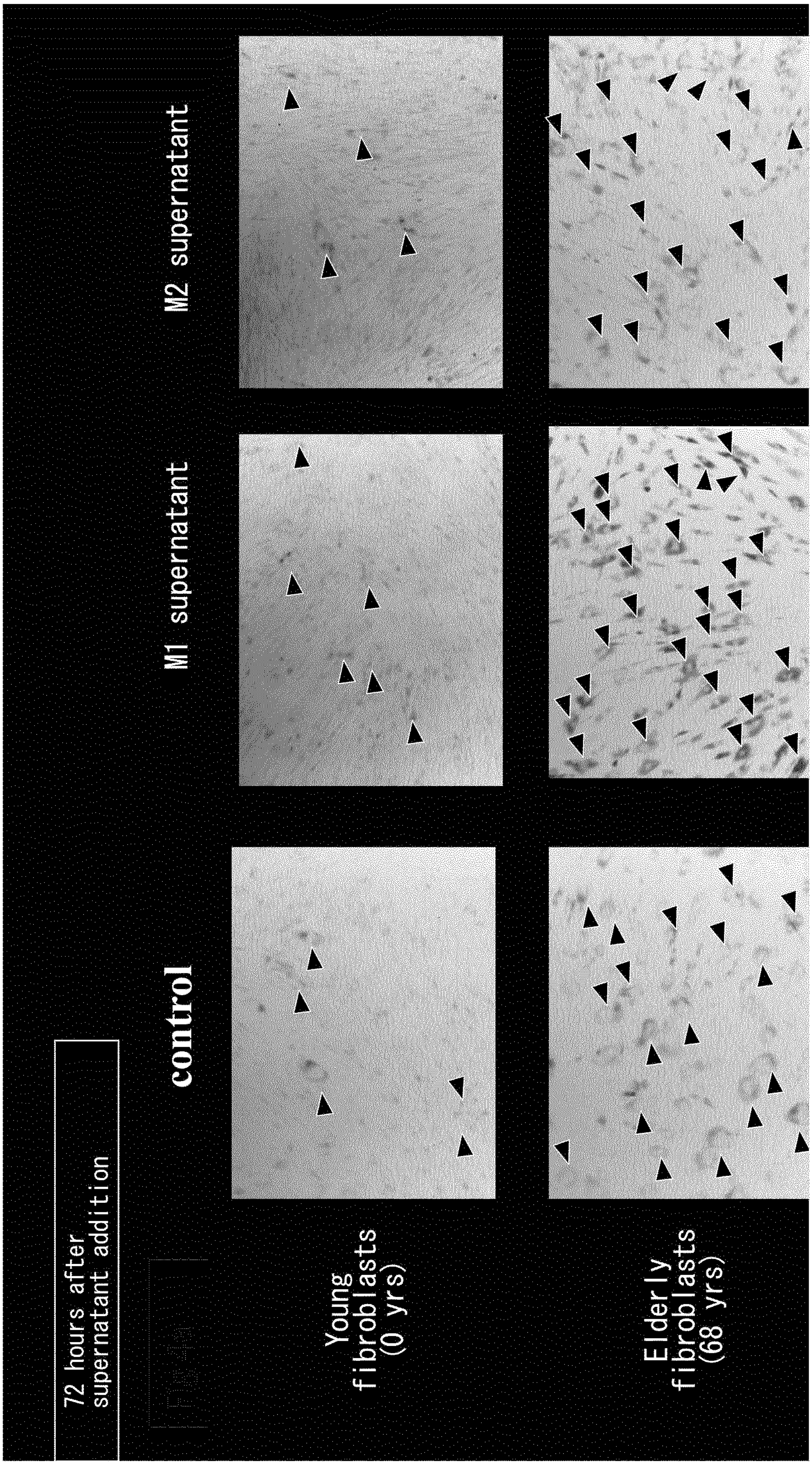

72 hours after
supernatant addition
control
M1 supernatant
M2 supernatant
Young
fibroblasts
(0 yrs)
Elderly
fibroblasts
(68 yrs)

FIG. 4b

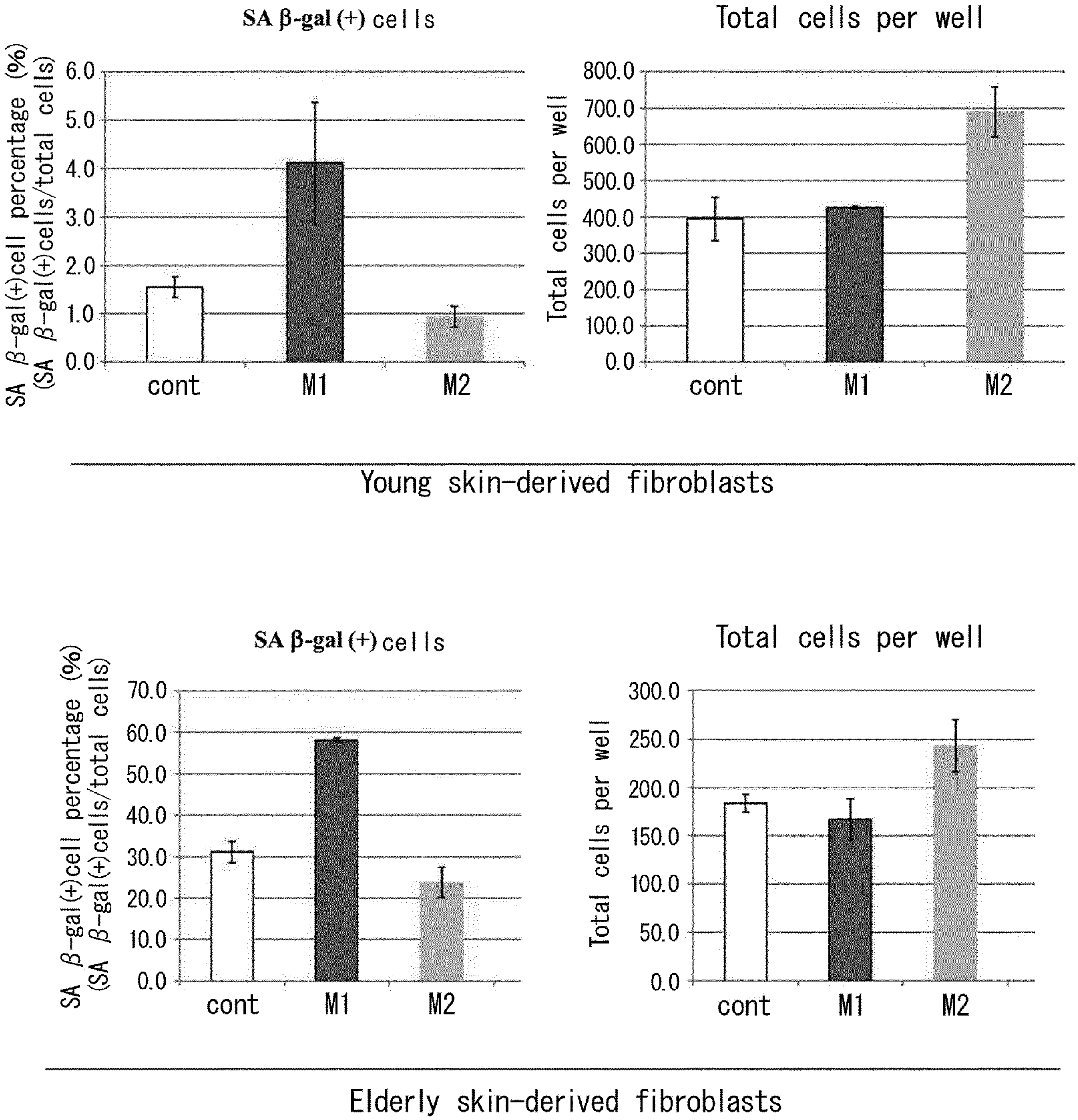
SA β-gal (+) cells
SA β-gal(+)cell percentage (%) (SA β-gal(+)cells/total cells)
6.0
5.0
4.0
3.0
2.0
1.0
0.0
cont
M1
M2
Total cells per well
Total cells per well
800.0
700.0
600.0
500.0
400.0
300.0
200.0
100.0
0.0
cont
M1
M2
Young skin-derived fibroblasts
SA β-gal (+) cells
SA β-gal(+)cell percentage (%) (SA β-gal(+)cells/total cells)
70.0
60.0
50.0
40.0
30.0
20.0
10.0
0.0
cont
M1
M2
Total cells per well
Total cells per well
300.0
250.0
200.0
150.0
100.0
50.0
0.0
cont
M1
M2
Elderly skin-derived fibroblasts

FIG. 4c

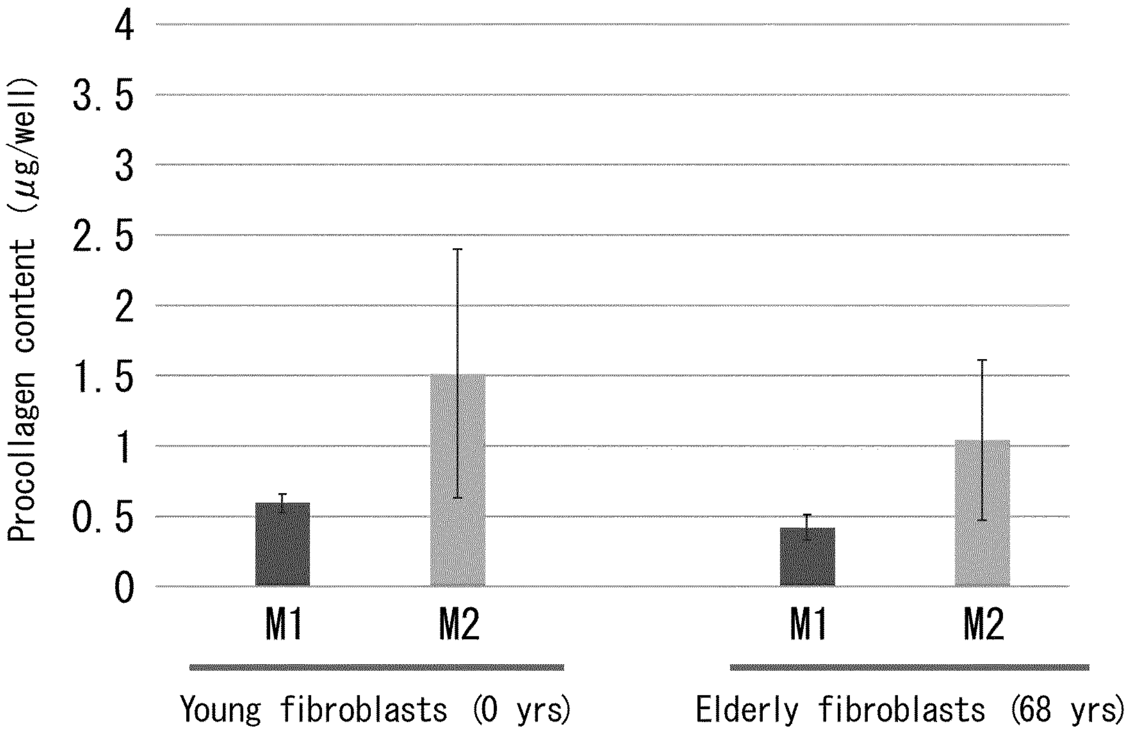
Procollagen content (μg/well)
4
3.5
3
2.5
2
1.5
1
0.5
0
M1
M2
M1
M2
Young fibroblasts (0 yrs)
Elderly fibroblasts (68 yrs)

FIG. 4d

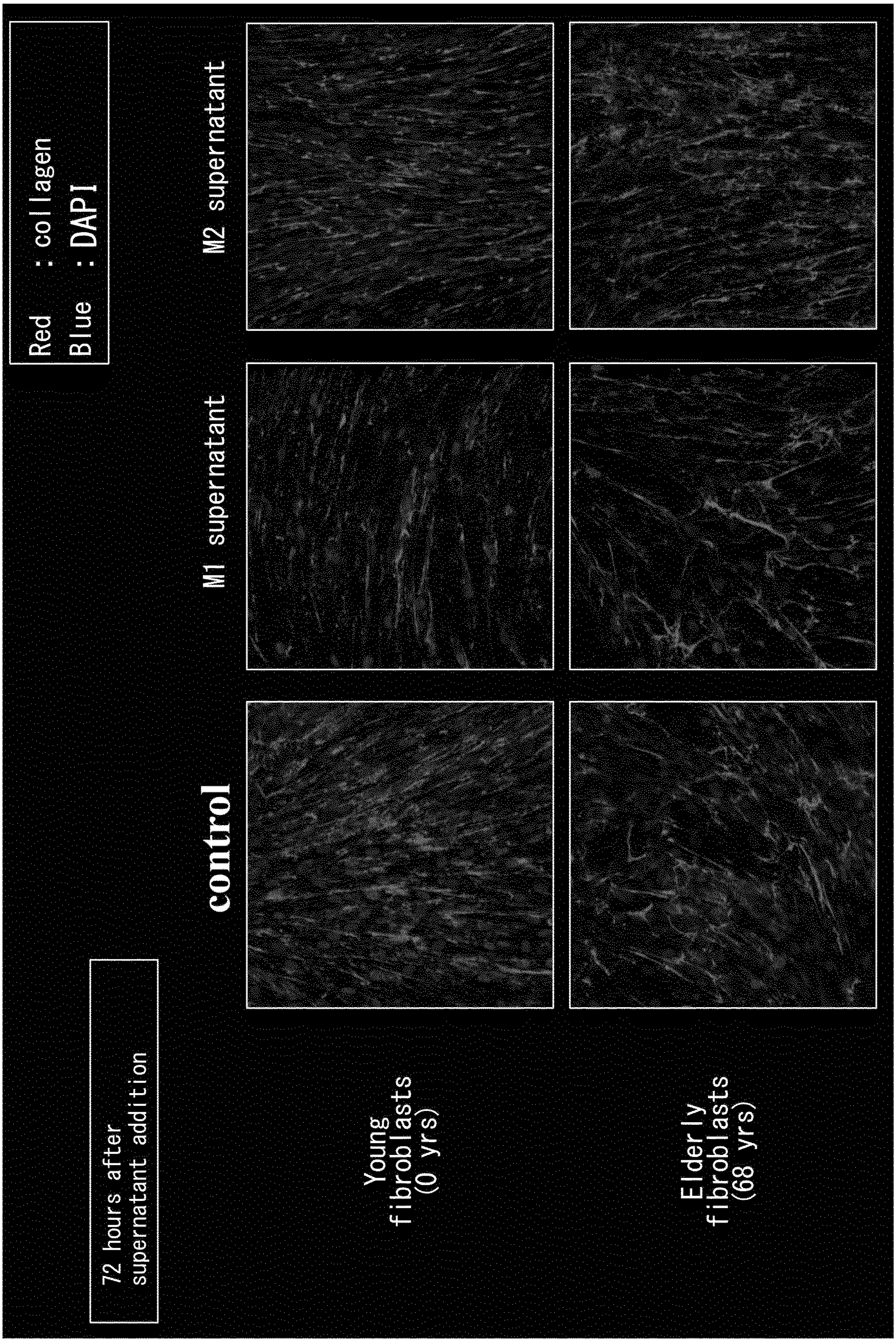
72 hours after
supernatant addition
Red : collagen
Blue : DAPI
control
M1 supernatant
M2 supernatant
Young
fibroblasts
(0 yrs)
Elderly
fibroblasts
(68 yrs)

FIG. 5

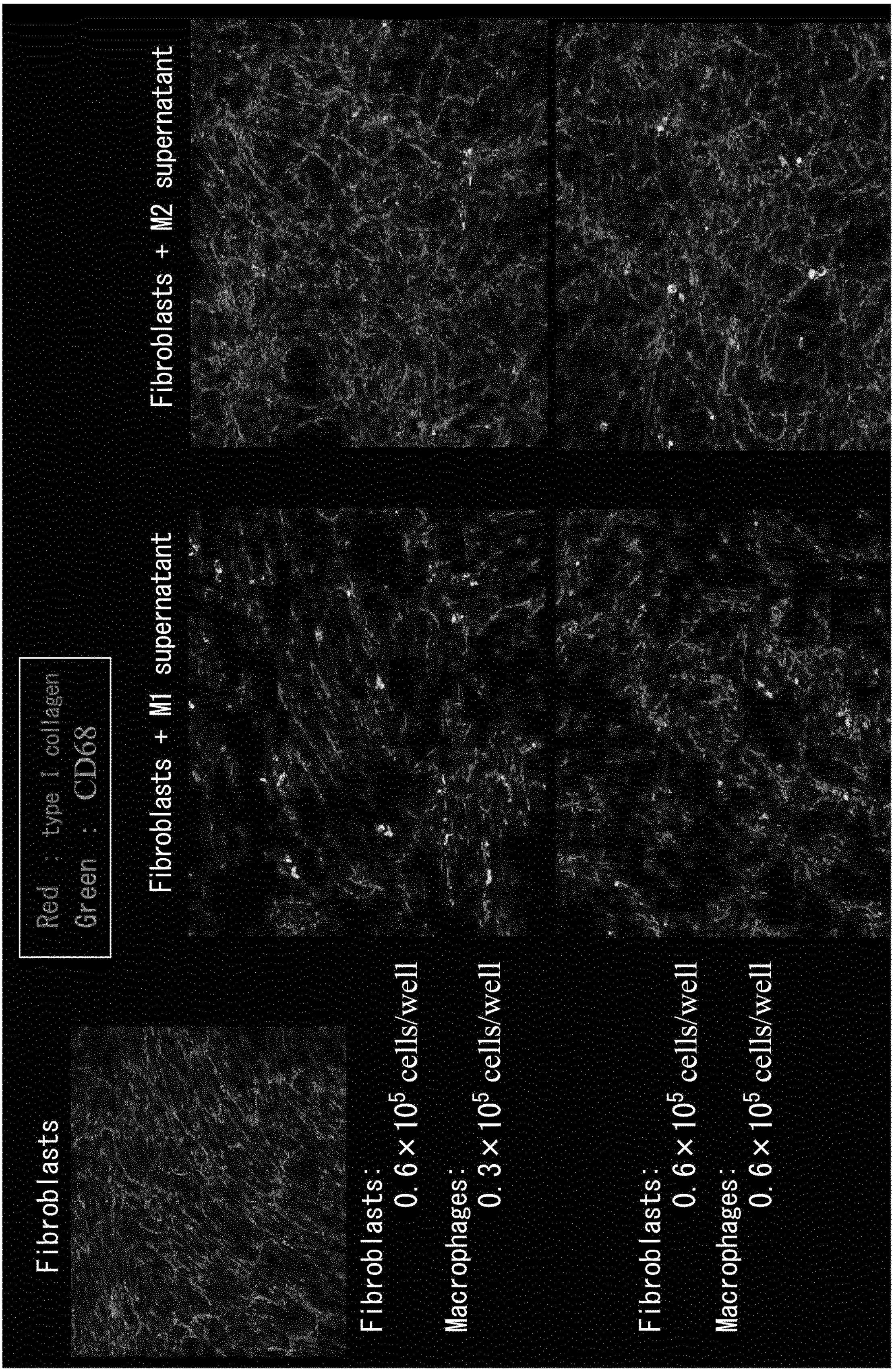
Fibroblasts
Red : type I collagen
Green : CD68
Fibroblasts + M1 supernatant
Fibroblasts + M2 supernatant
Fibroblasts: 0.6×10⁵ cells/well
Macrophages: 0.3×10⁵ cells/well
Fibroblasts: 0.6×10⁵ cells/well
Macrophages: 0.6×10⁵ cells/well

FIG. 6

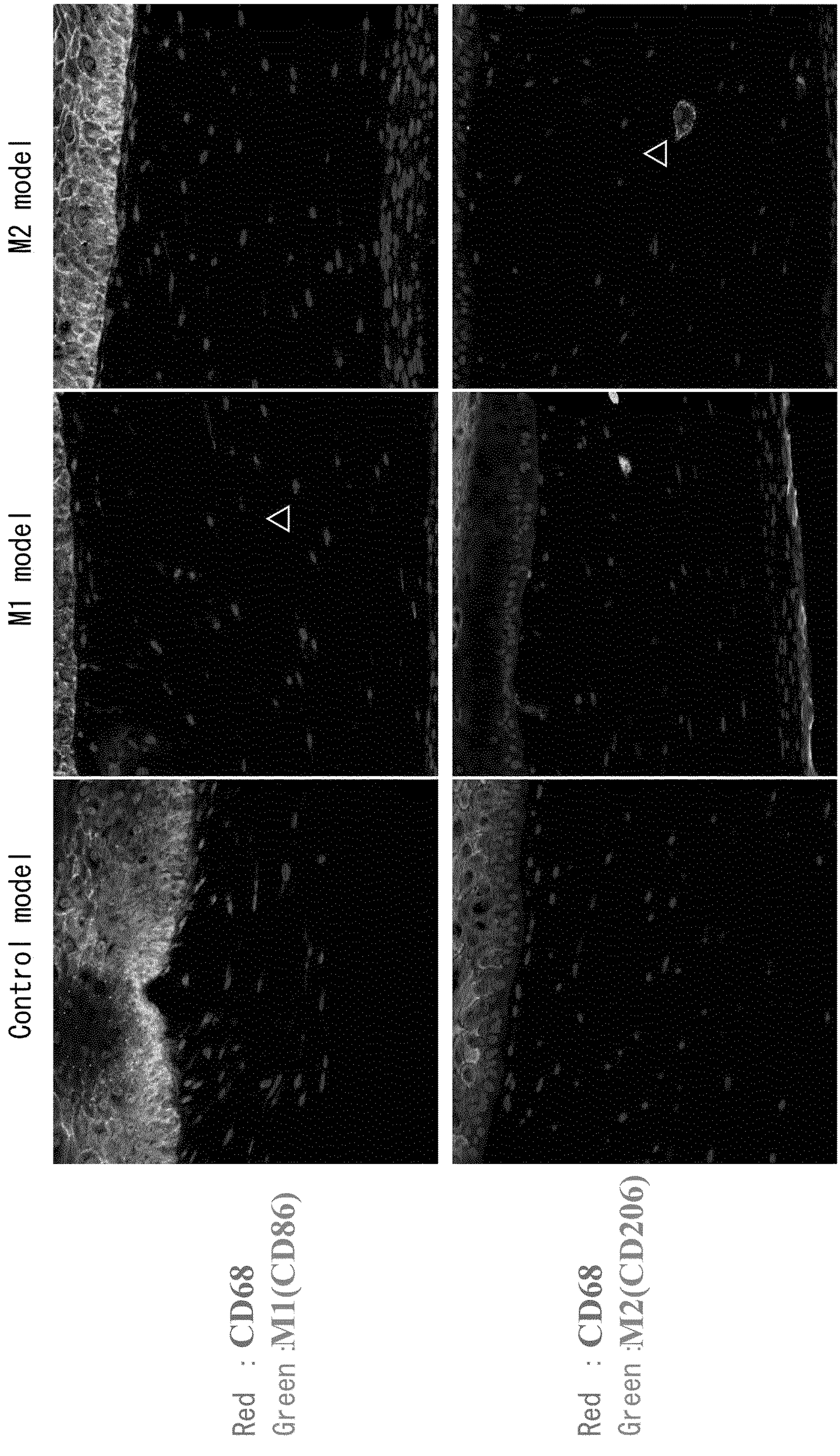
Control model
M1 model
M2 model
Red : CD68
Green :M1(CD86)
Red : CD68
Green :M2(CD206)

FIG. 7

Red :p21
Blue: nuclei

Control model M1 model M2 model

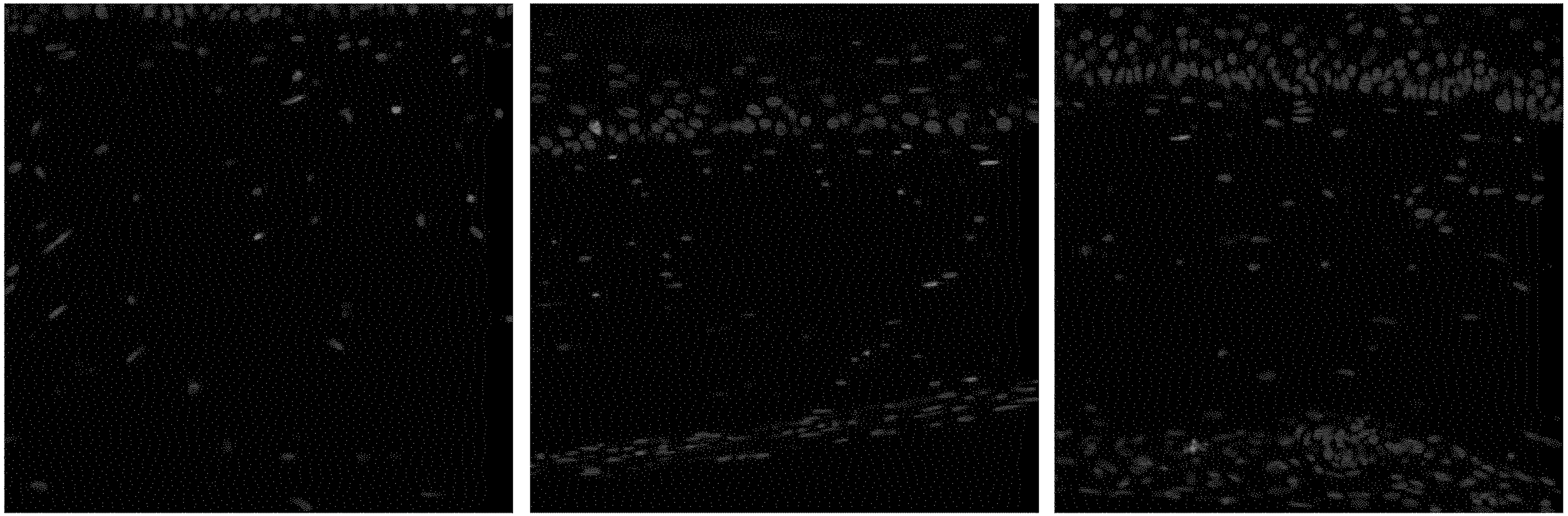

FIG. 8

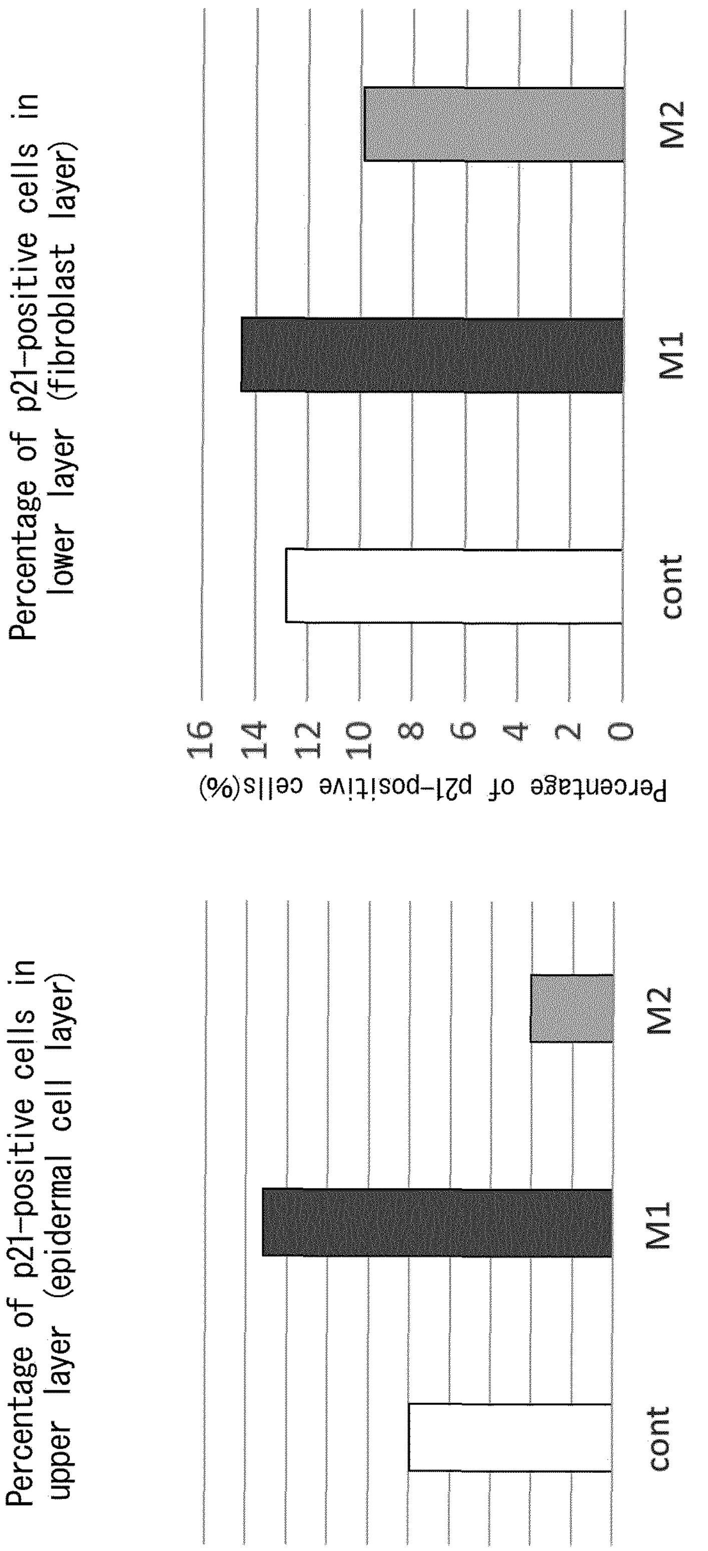
Percentage of p21-positive cells in upper layer (epidermal cell layer)
Percentage of p21-positive cells(%)
2
1.8
1.6
1.4
1.2
1
0.8
0.6
0.4
0.2
0
cont
M1
M2
Percentage of p21-positive cells in lower layer (fibroblast layer)
Percentage of p21-positive cells(%)
16
14
12
10
8
6
4
2
0
cont
M1
M2

FIG. 9

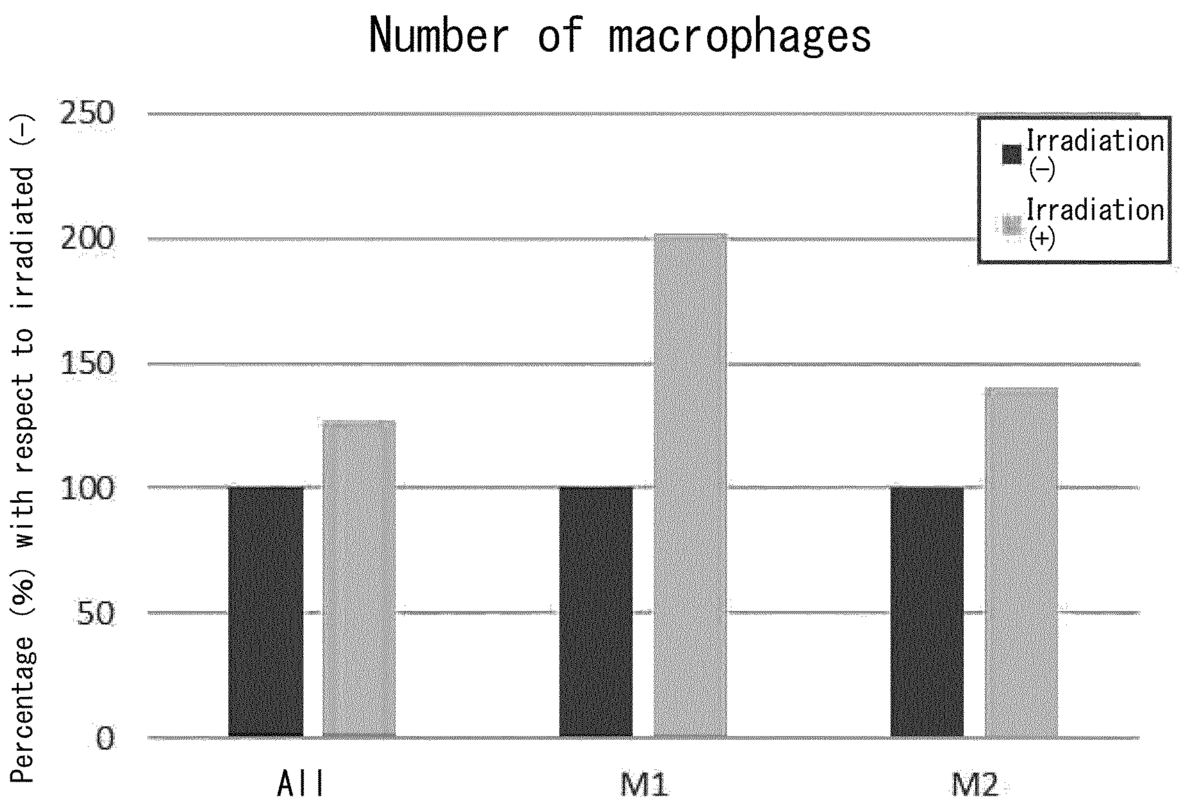
Number of macrophages
Percentage (%) with respect to irradiated (−)
250
200
150
100
50
0
All
M1
M2
Irradiation (−)
Irradiation (+)

FIG. 10

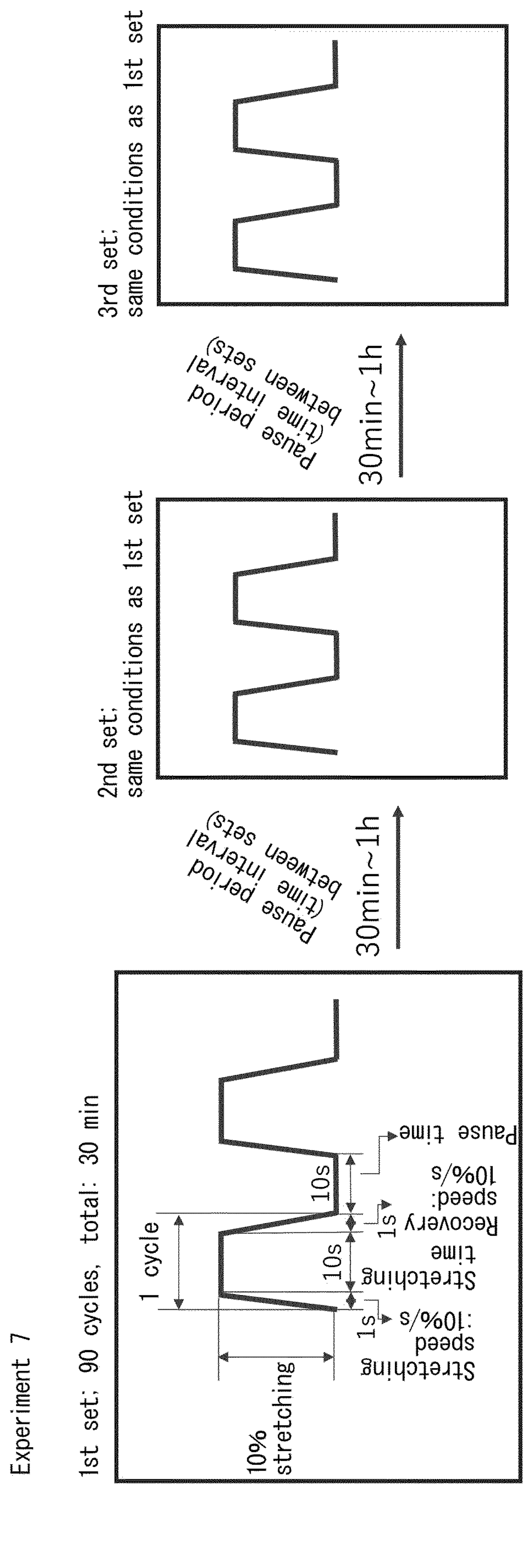
Experiment 7
1st set: 90 cycles, total: 30 min
10% stretching
1 cycle
Stretching speed :10%/s
1s
10s
Stretching time
1s
Recovery speed: 10%/s
10s
Pause time
Pause period (time interval between sets)
30min~1h
2nd set; same conditions as 1st set
Pause period (time interval between sets)
30min~1h
3rd set; same conditions as 1st set

FIG. 11

Device full image

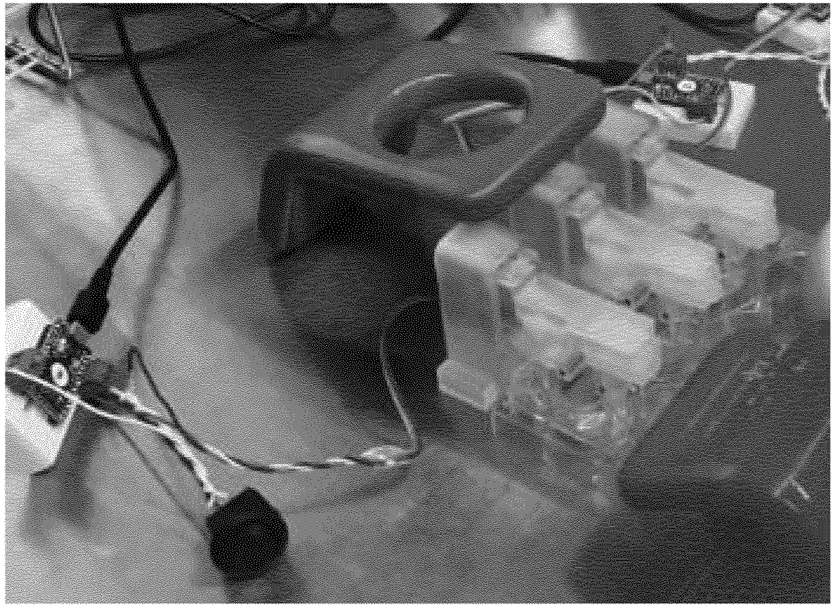

*ex vivo* cultured skin tissue and device periphery diagram

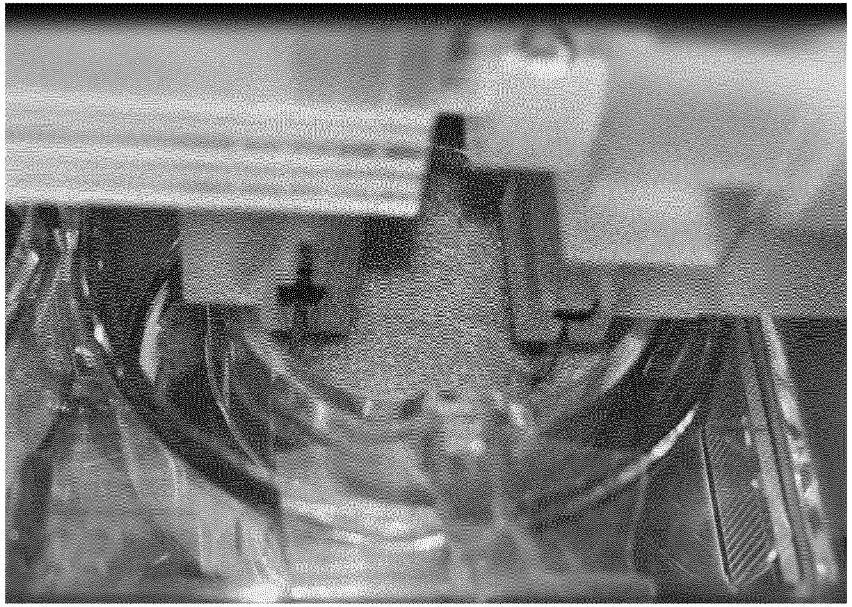

FIG. 12

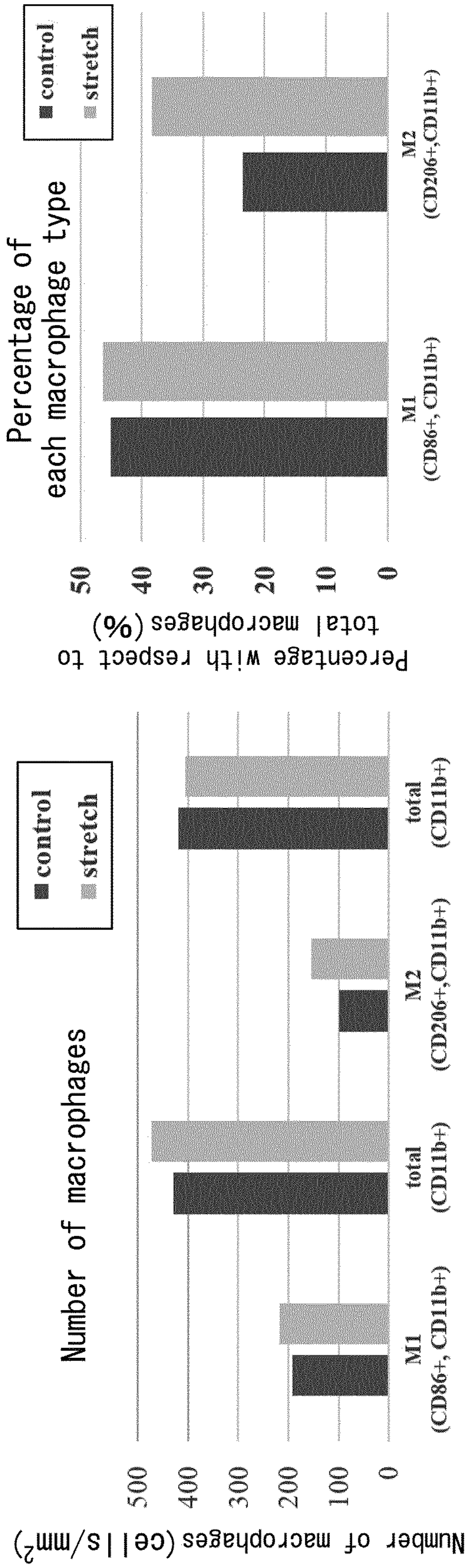
Number of macrophages
Number of macrophages (cells/mm²)
500
400
300
200
100
0
M1 (CD86+, CD11b+)
total (CD11b+)
M2 (CD206+,CD11b+)
total (CD11b+)
control
stretch
Percentage of each macrophage type
Percentage with respect to total macrophages (%)
50
40
30
20
10
0
M1 (CD86+, CD11b+)
M2 (CD206+,CD11b+)
control
stretch

FIG. 13

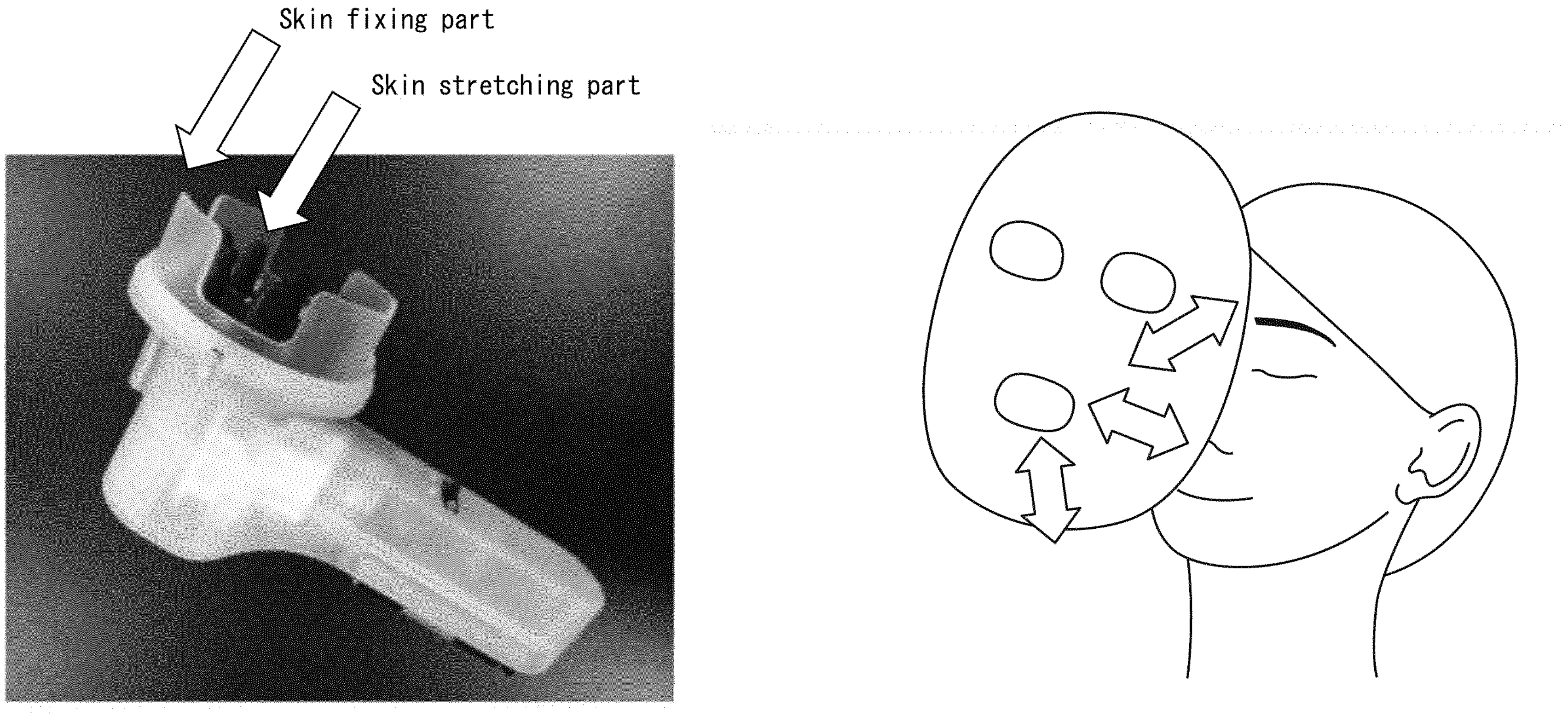

FIG. 14a

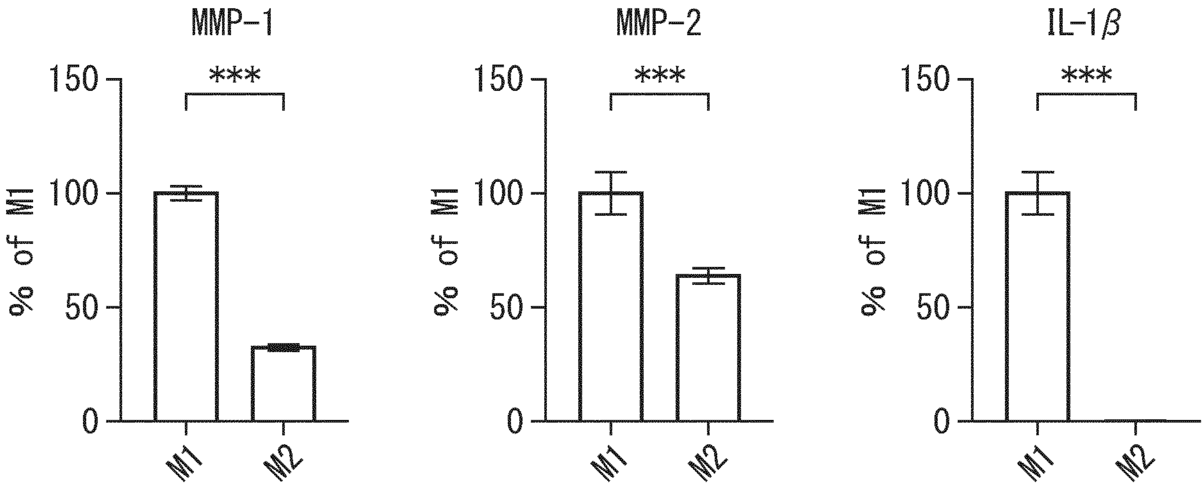


*** P<0.001, student's t-test

FIG. 14b

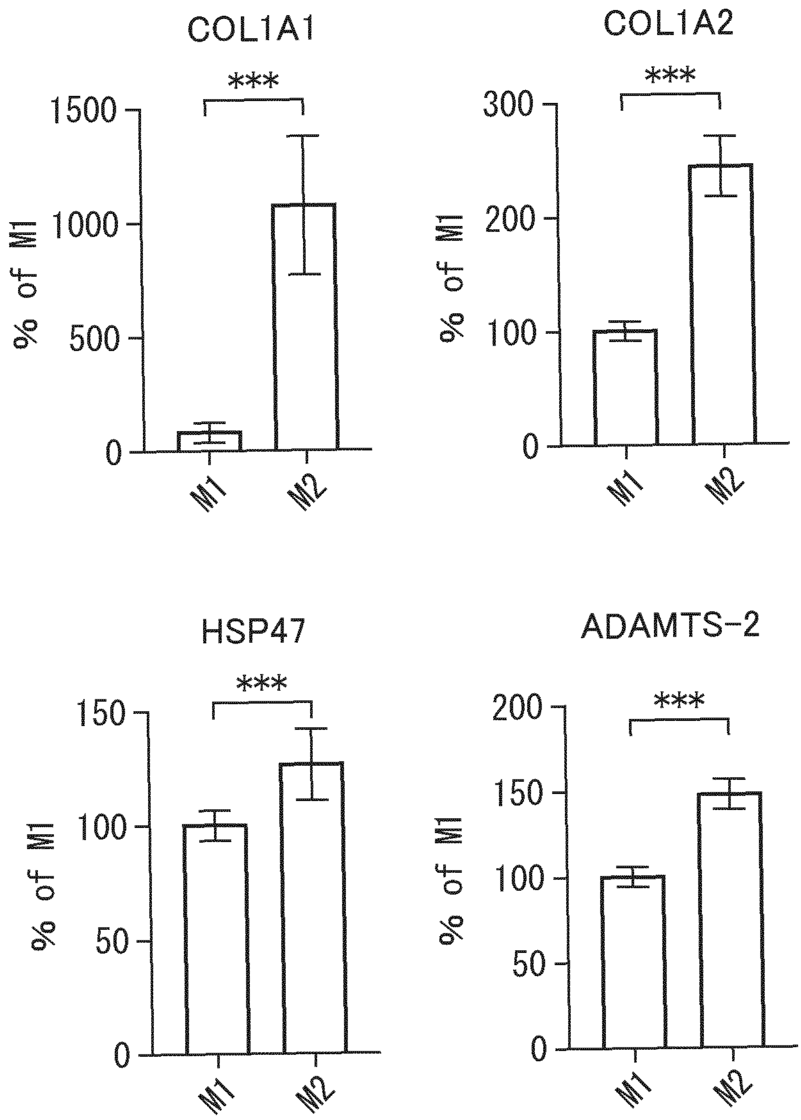


* P<0. 005, *** P<0. 001, student's t-test

FIG. 15a

| | Young group | | | Elderly group |
|---|---|---|---|---|
| | age | | | age |
| 1 | 25 | | 11 | 62 |
| 2 | 27 | | 12 | 63 |
| 3 | 27 | | 13 | 66 |
| 4 | 28 | | 14 | 69 |
| 5 | 33 | | 15 | 70 |
| 6 | 35 | | 16 | 75 |
| 7 | 38 | | 17 | 76 |
| 8 | 39 | | 18 | 83 |
| 9 | 40 | | 19 | 83 |
| 10 | 43 | | 20 | 88 |

FIG. 15b

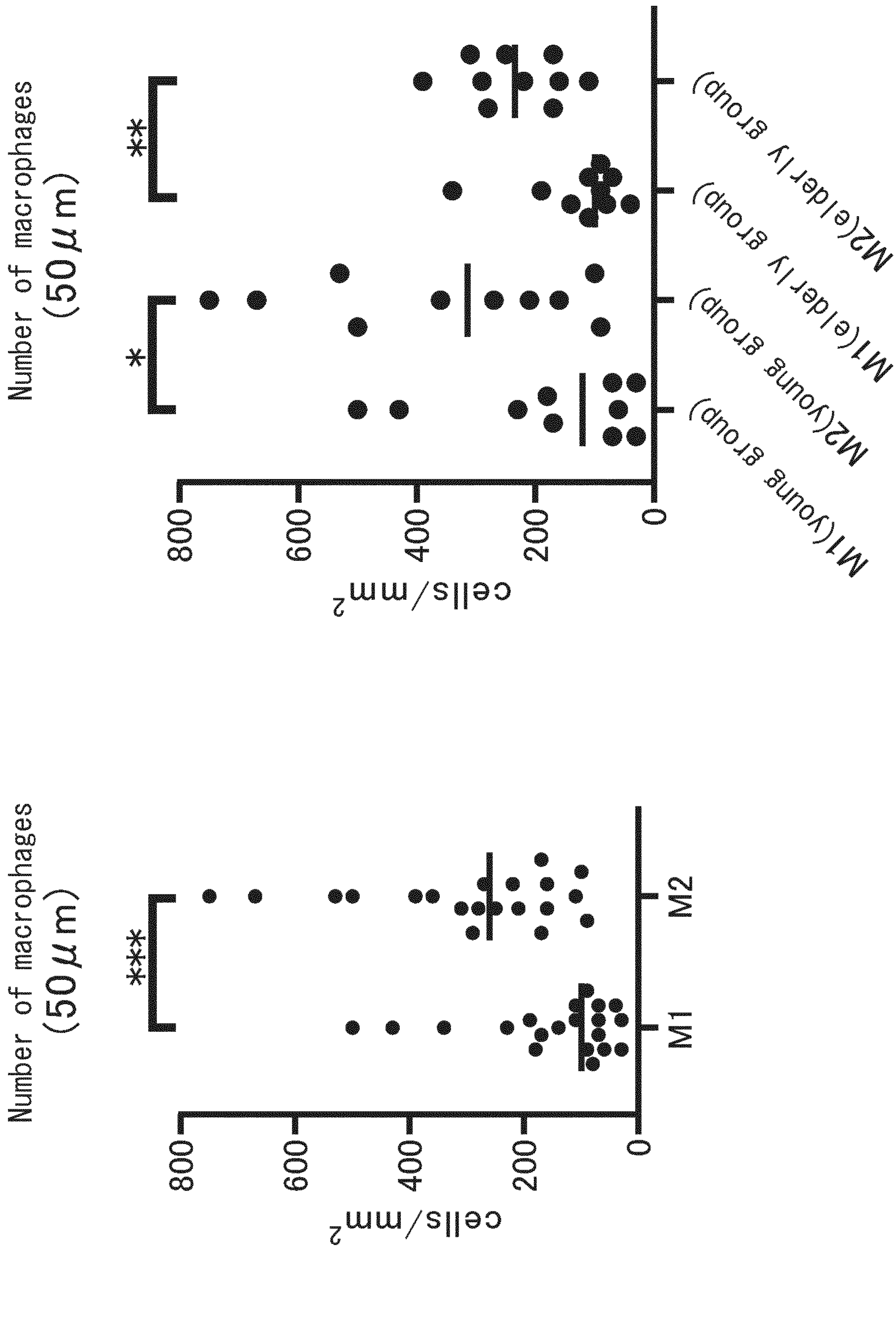
Number of macrophages
(50μm)
***
cells/mm²
800
600
400
200
0
M1
M2
Number of macrophages
(50μm)
*
**
cells/mm²
800
600
400
200
0
M1(young group)
M2(young group)
M1(elderly group)
M2(elderly group)

FIG. 15c

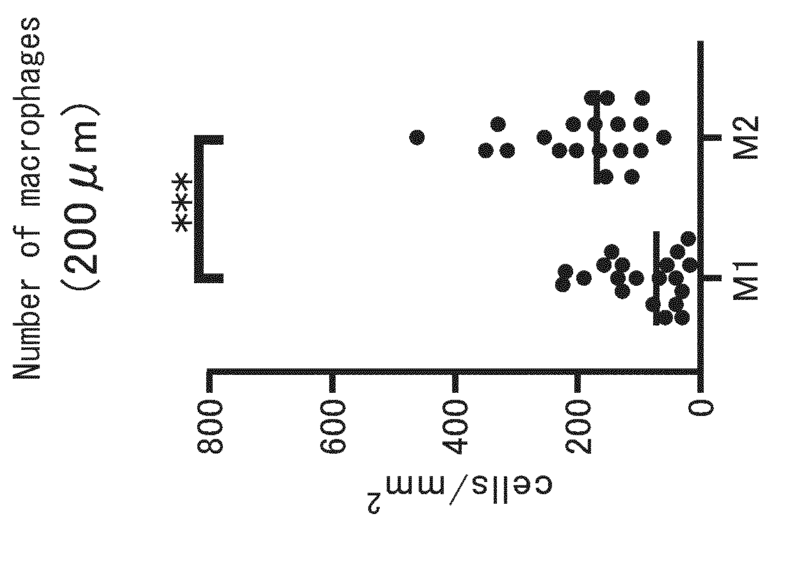
Number of macrophages
(200 μm)
***
cells/mm2
800
600
400
200
0
M1
M2

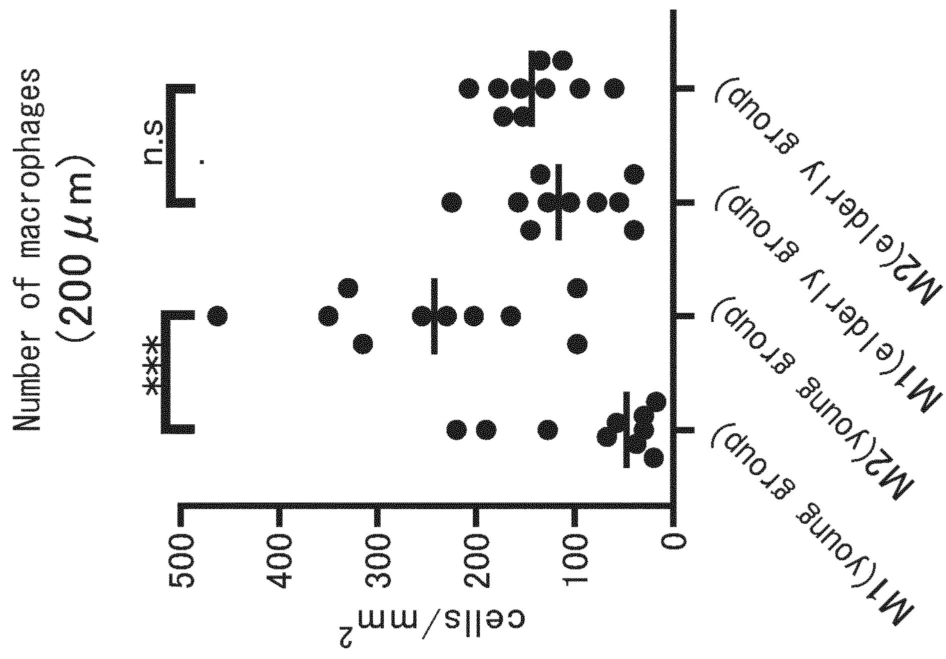
Number of macrophages
(200 μm)
***
n.s
cells/mm2
500
400
300
200
100
0
M1(young group)
M2(young group)
M1(elder-ly group)
M2(elder-ly group)

FIG. 15d

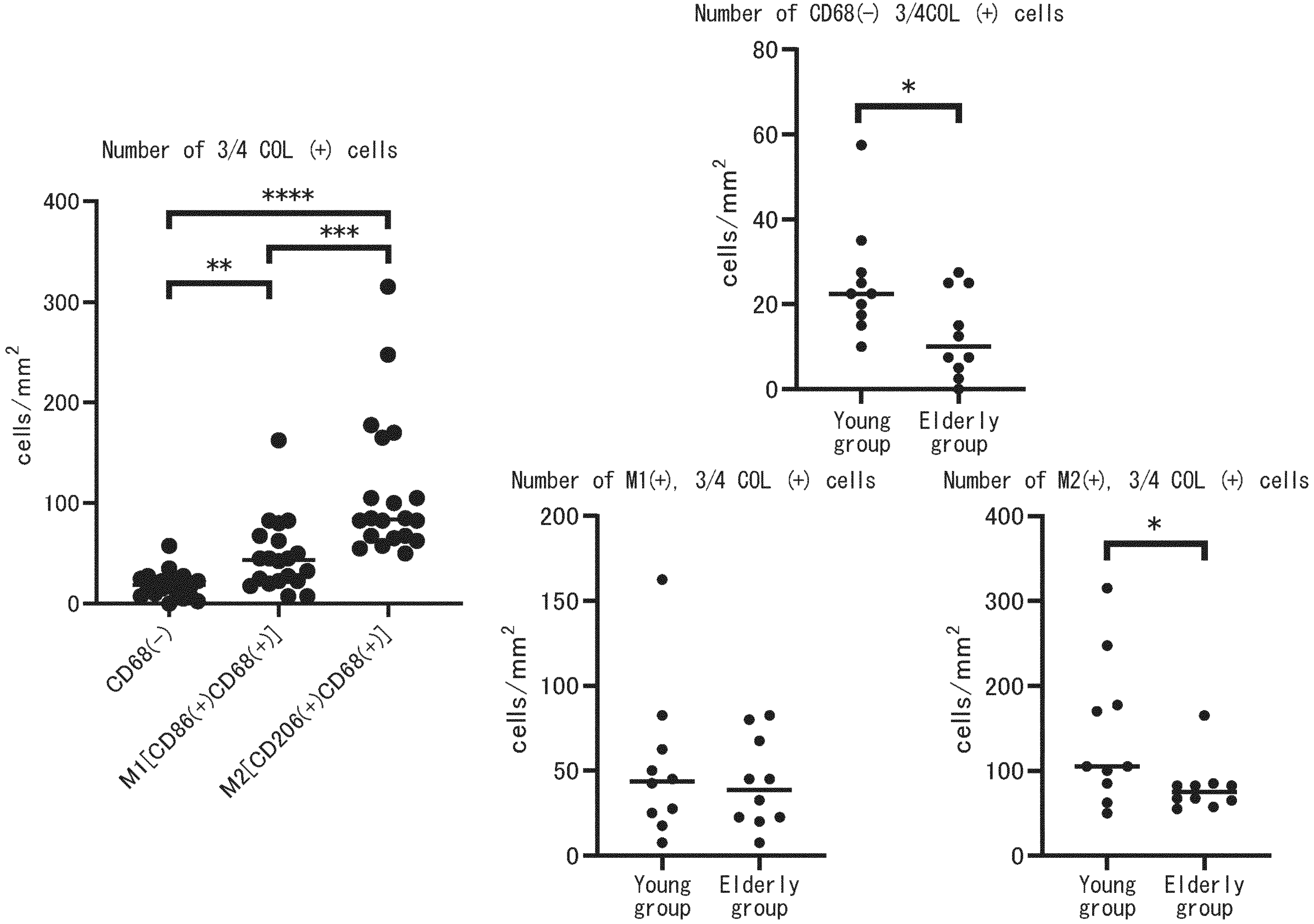
Number of 3/4 COL (+) cells
cells/mm2
400
300
200
100
0
****
***
**
CD68(−)
M1[CD86(+)CD68(+)]
M2[CD206(+)CD68(+)]
Number of CD68(−) 3/4COL (+) cells
80
60
40
20
*
Young group
Elderly group
Number of M1(+), 3/4 COL (+) cells
200
150
100
50
Number of M2(+), 3/4 COL (+) cells

FIG. 16a

24 hours after melanin addition

Fibroblasts

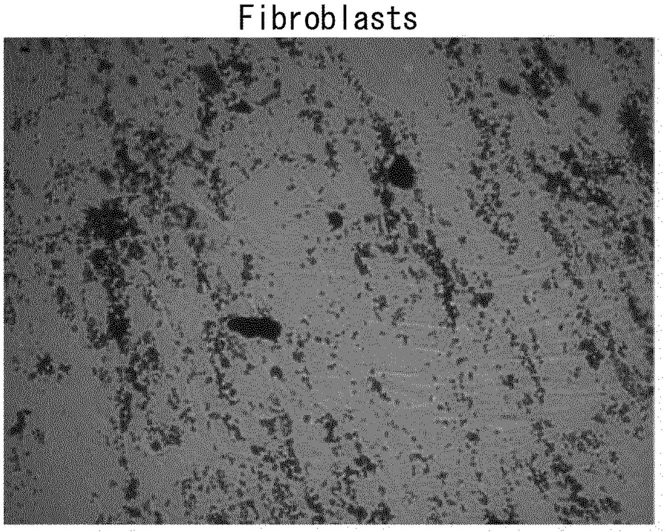

M1 macrophages

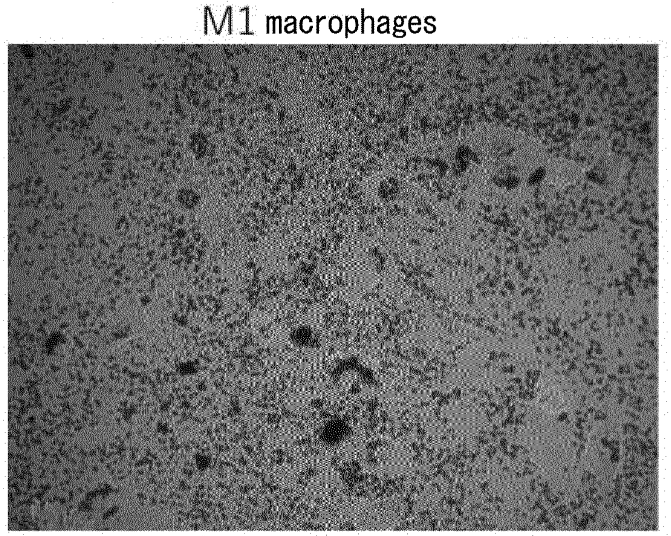

M2 macrophages

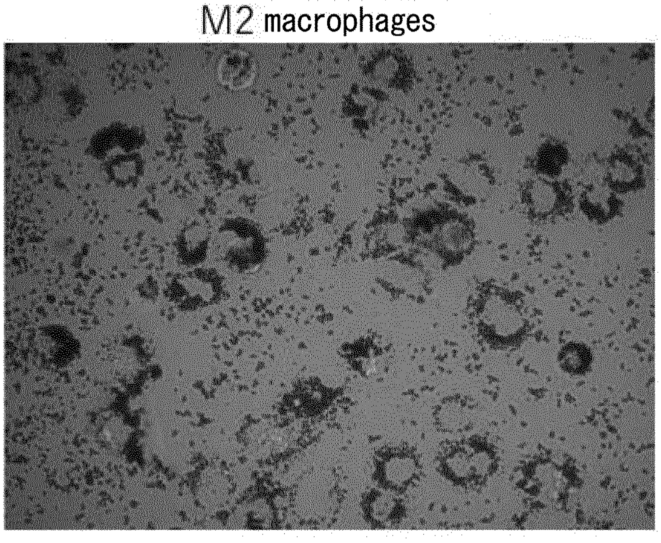

FIG. 16b

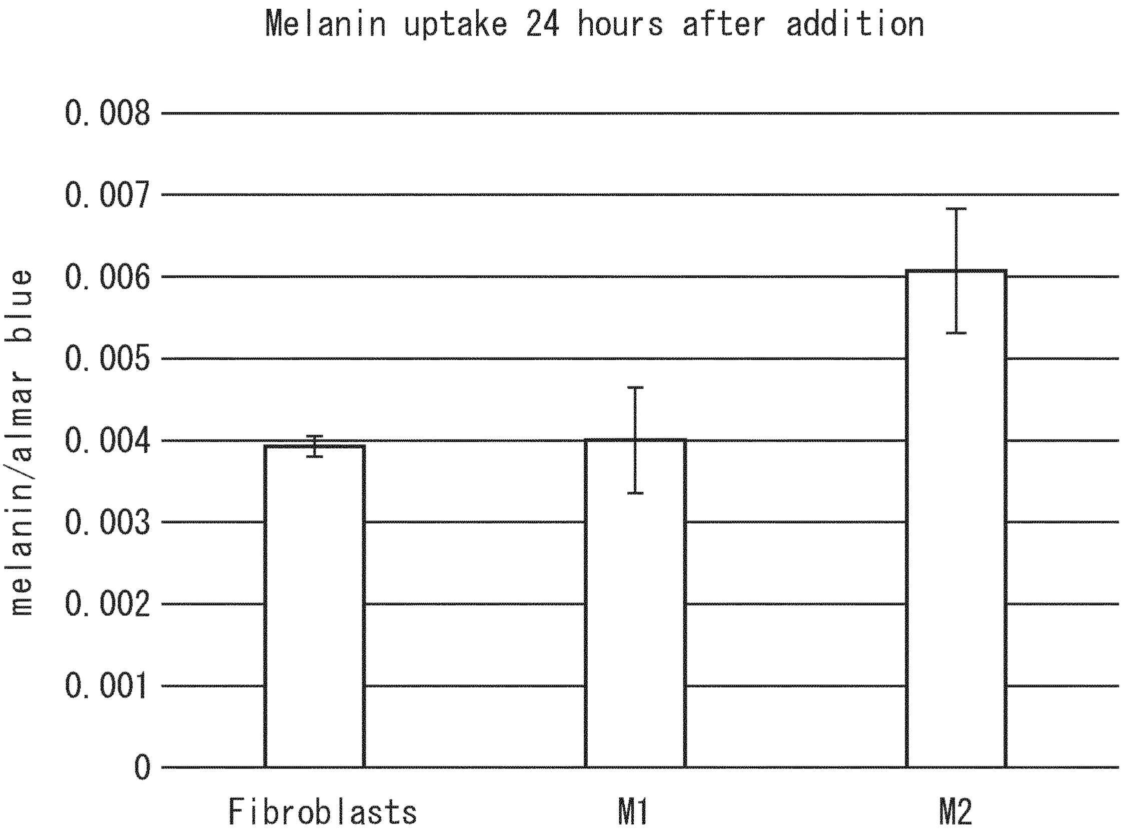
Melanin uptake 24 hours after addition
melanin/almar blue
0.008
0.007
0.006
0.005
0.004
0.003
0.002
0.001
0
Fibroblasts
M1
M2

FIG. 16c

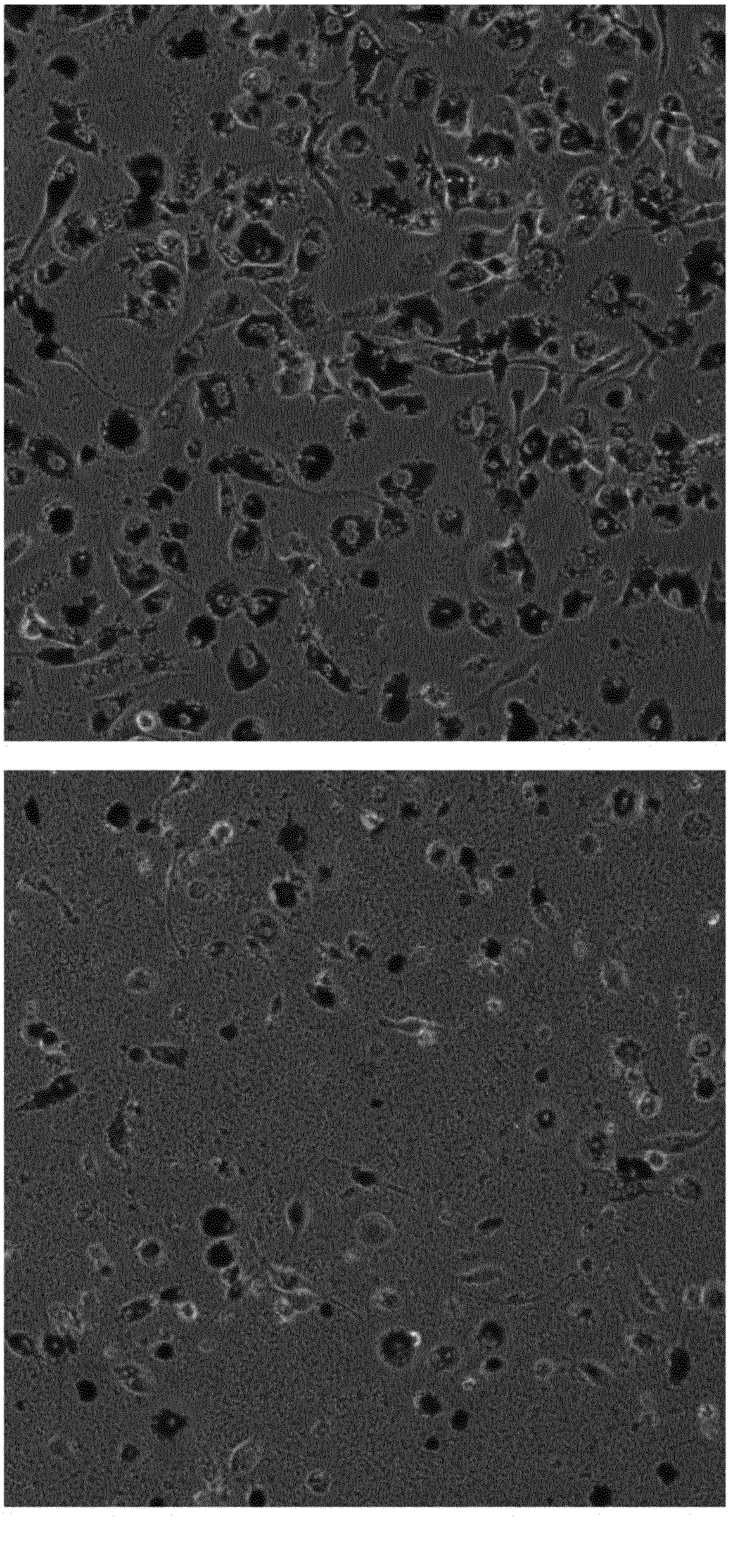

5 days after melanin addition

M2 macrophages

M1 macrophages

FIG. 17

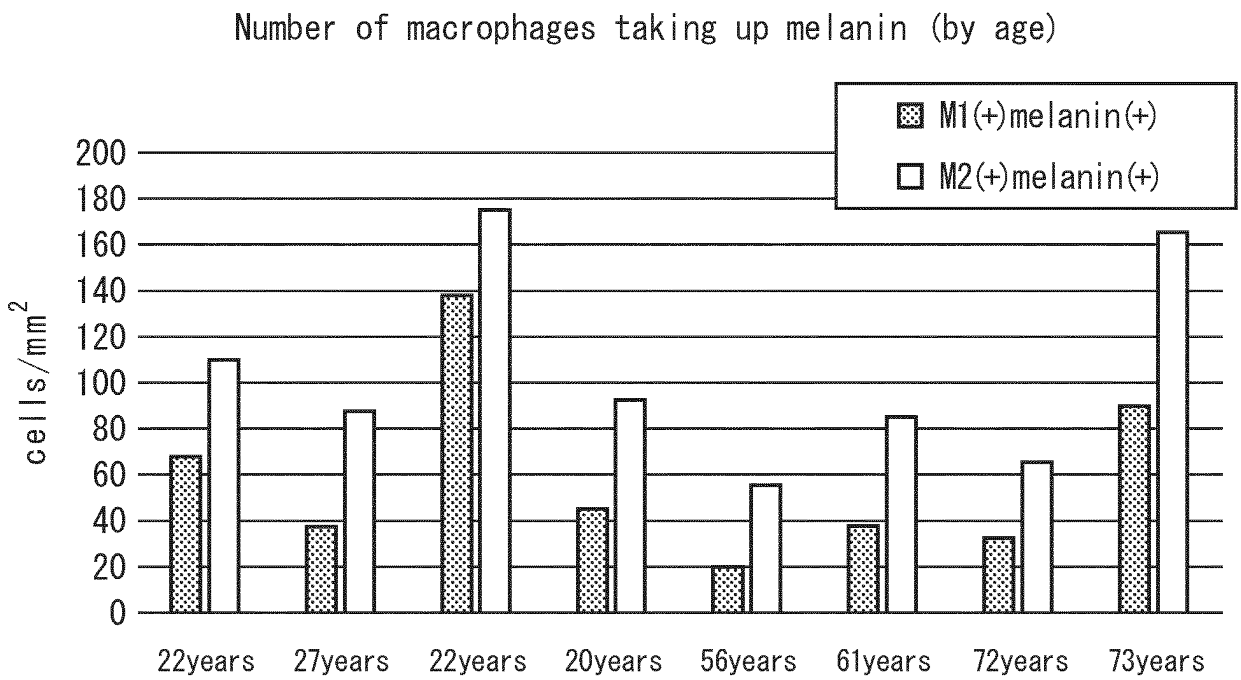
Number of macrophages taking up melanin (by age)
M1(+)melanin(+)
M2(+)melanin(+)
cells/mm2
200
180
160
140
120
100
80
60
40
20
0
22years
27years
22years
20years
56years
61years
72years
73years

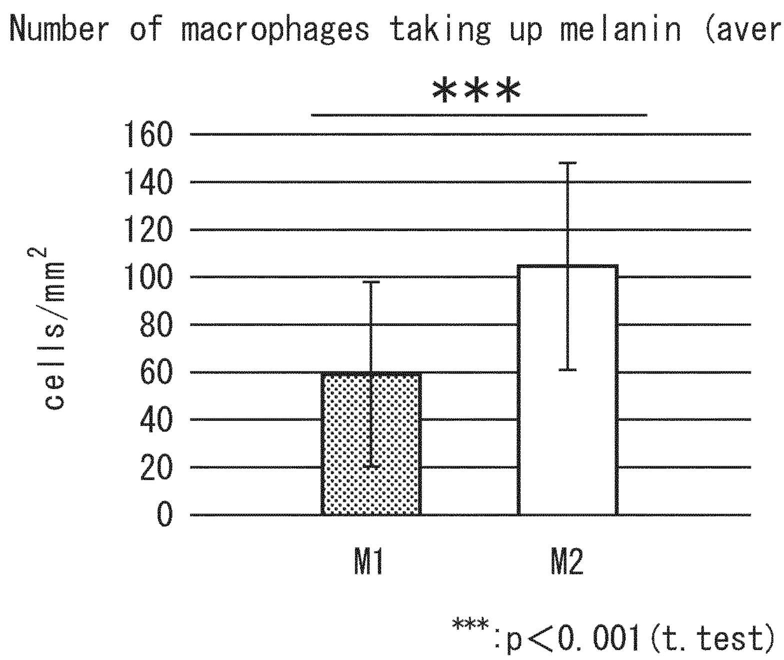
Number of macrophages taking up melanin (average)
***
cells/mm2
160
140
120
100
80
60
40
20
0
M1
M2
***:p<0.001 (t. test)

FIG. 18

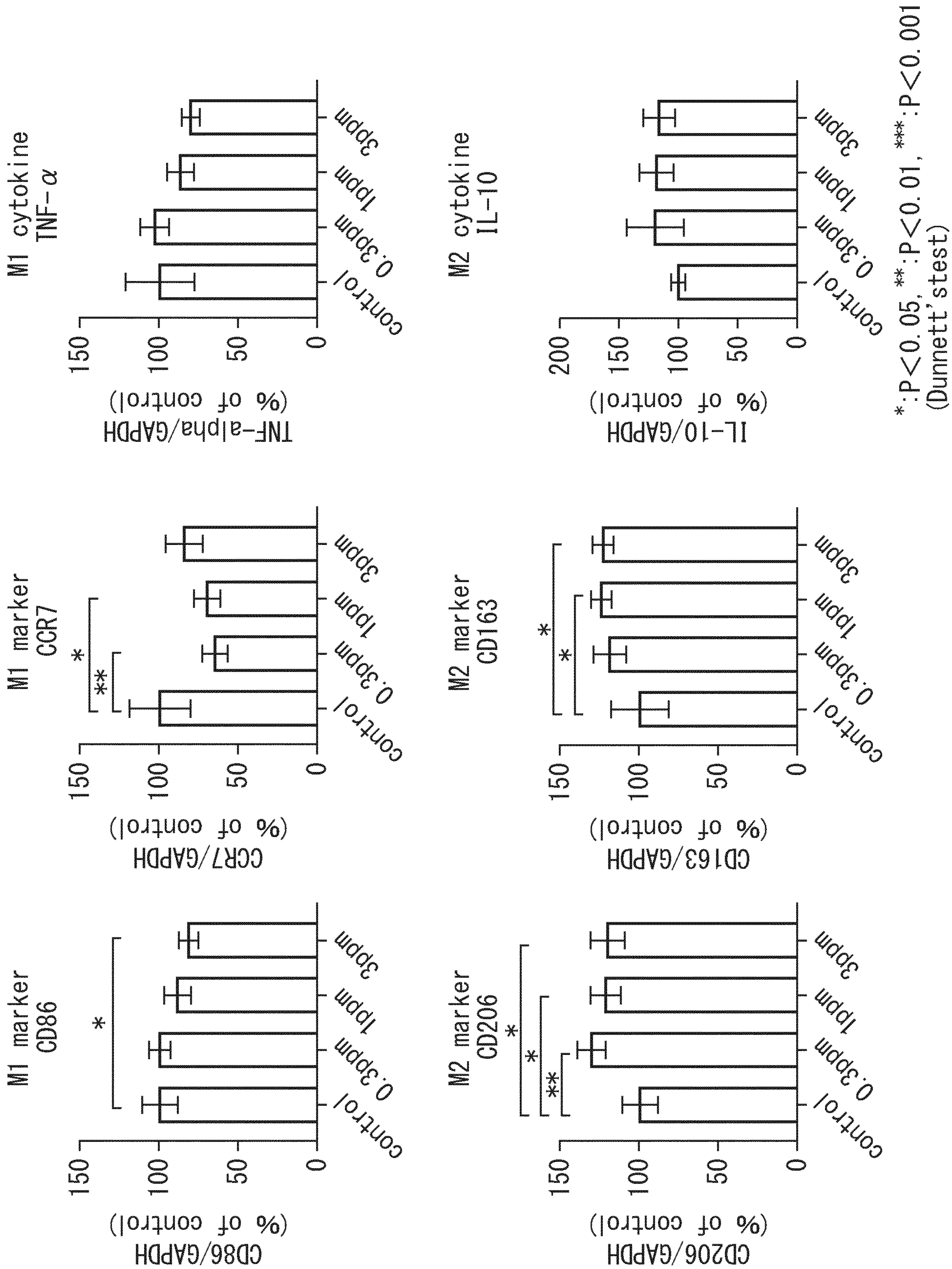
M1 marker CD86
CD86/GAPDH (% of control)
M1 marker CCR7
CCR7/GAPDH (% of control)
M1 cytokine TNF-α
TNF-alpha/GAPDH (% of control)
M2 marker CD206
CD206/GAPDH (% of control)
M2 marker CD163
CD163/GAPDH (% of control)
M2 cytokine IL-10
IL-10/GAPDH (% of control)
control
0.3ppm
1ppm
3ppm
*:P<0.05, **:P<0.01, ***:P<0.001
(Dunnett'stest)

FIG. 19a

30 minutes after melanin addition

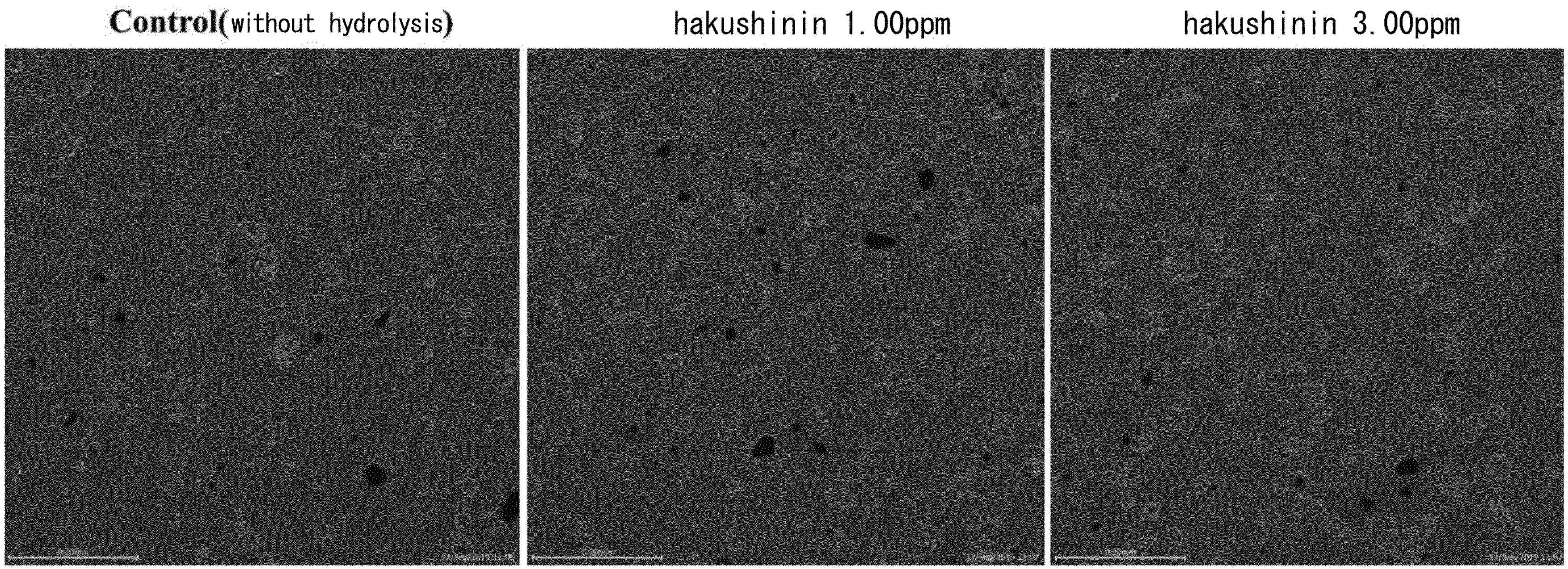

FIG. 19b

Enlarged view

hakushinin 3.00ppm

hakushinin 1.00ppm

Control(without hydrolysis)

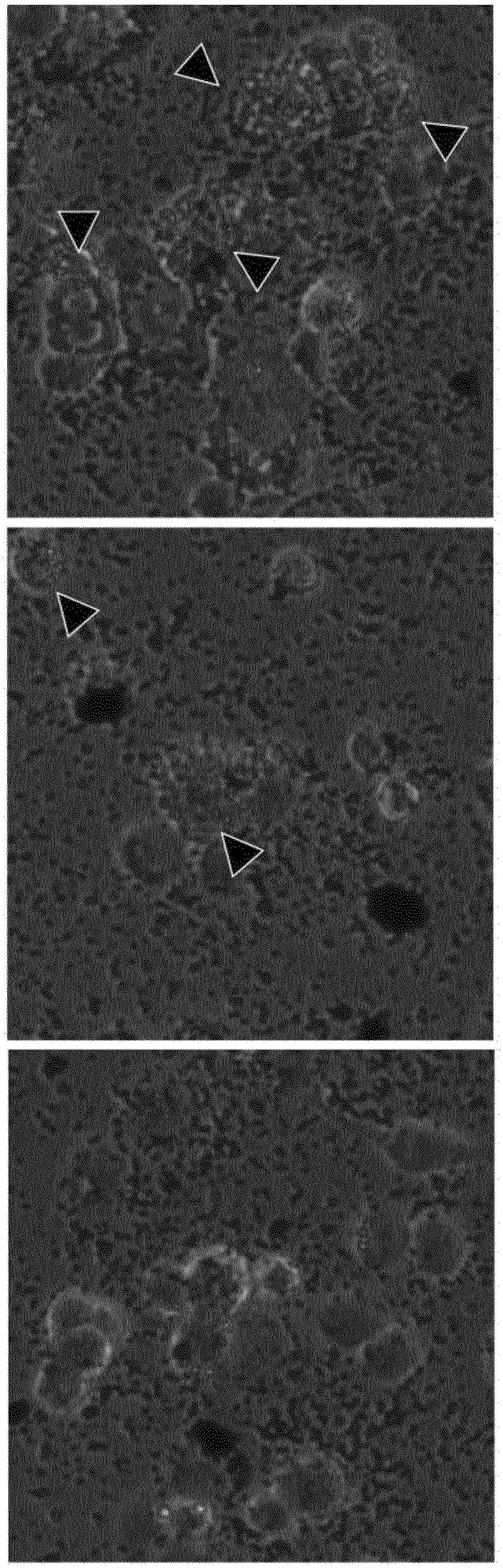

FIG. 20

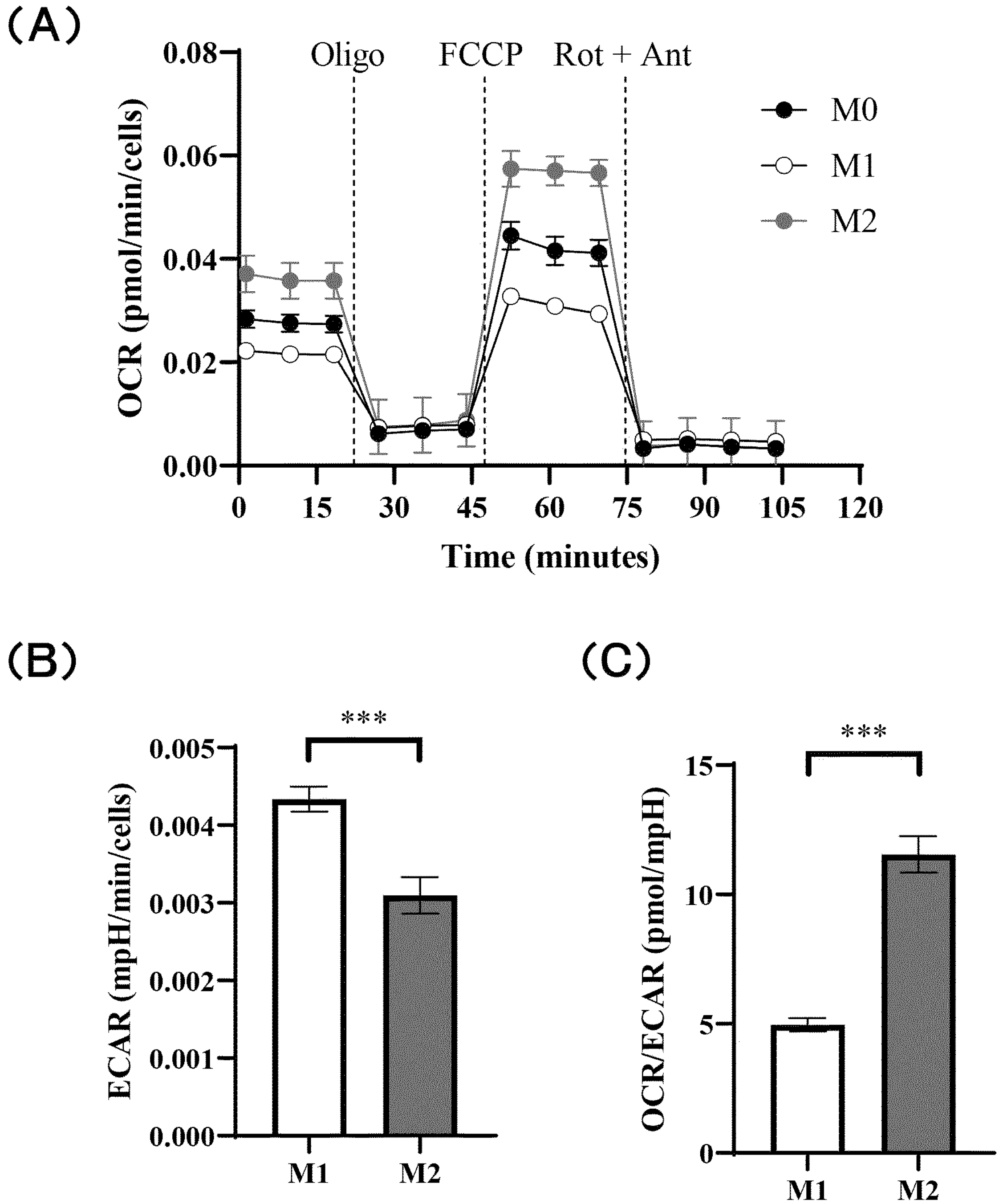
(A)
0.08
0.06
0.04
0.02
0.00
OCR (pmol/min/cells)
Oligo
FCCP
Rot + Ant
M0
M1
M2
0
15
30
45
60
75
90
105
120
Time (minutes)
(B)
***
0.005
0.004
0.003
0.002
0.001
0.000
ECAR (mpH/min/cells)
M1
M2
(C)
***
15
10
5
0
OCR/ECAR (pmol/mpH)
M1
M2

FIG. 21

(A)

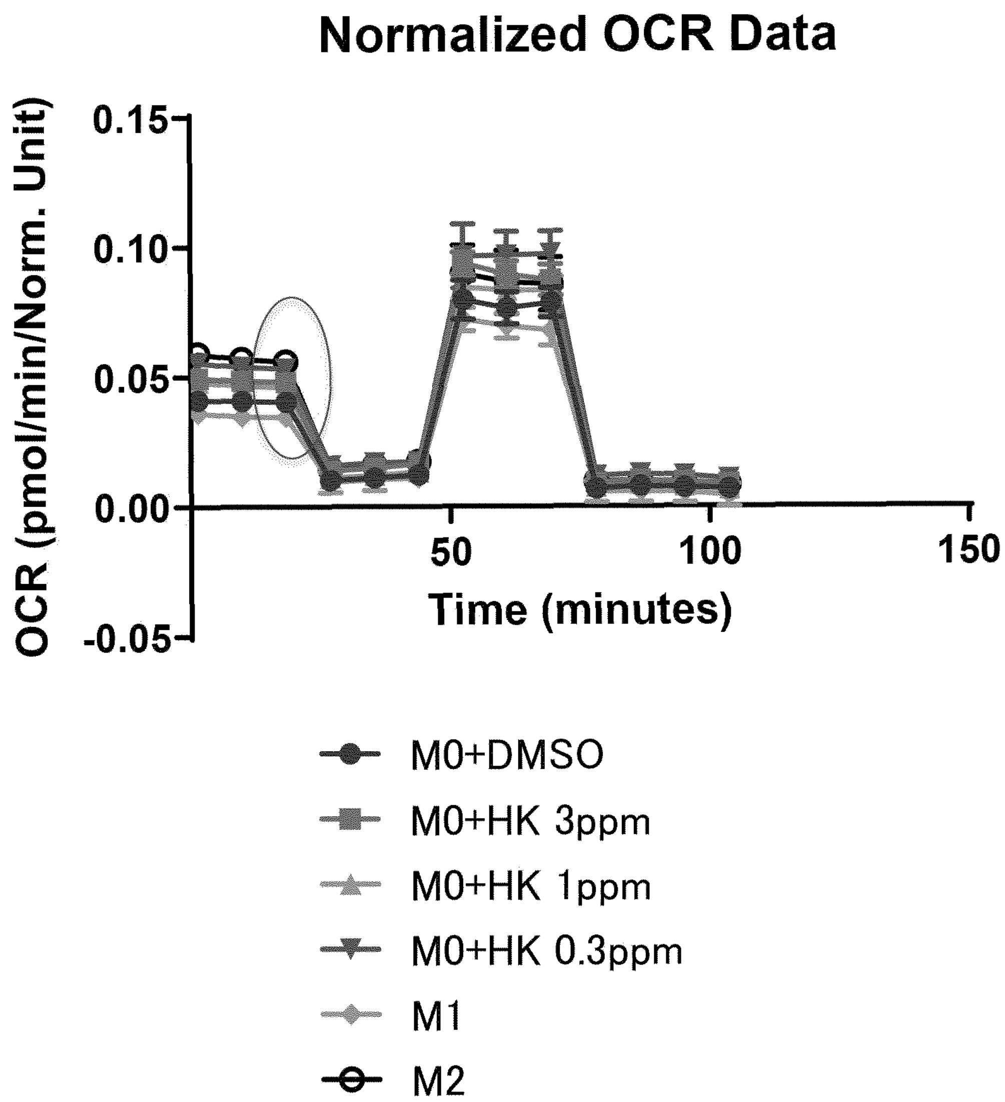
Normalized OCR Data
OCR (pmol/min/Norm. Unit)
0.15
0.10
0.05
0.00
-0.05
50
100
150
Time (minutes)
M0+DMSO
M0+HK 3ppm
M0+HK 1ppm
M0+HK 0.3ppm
M1
M2

(B)

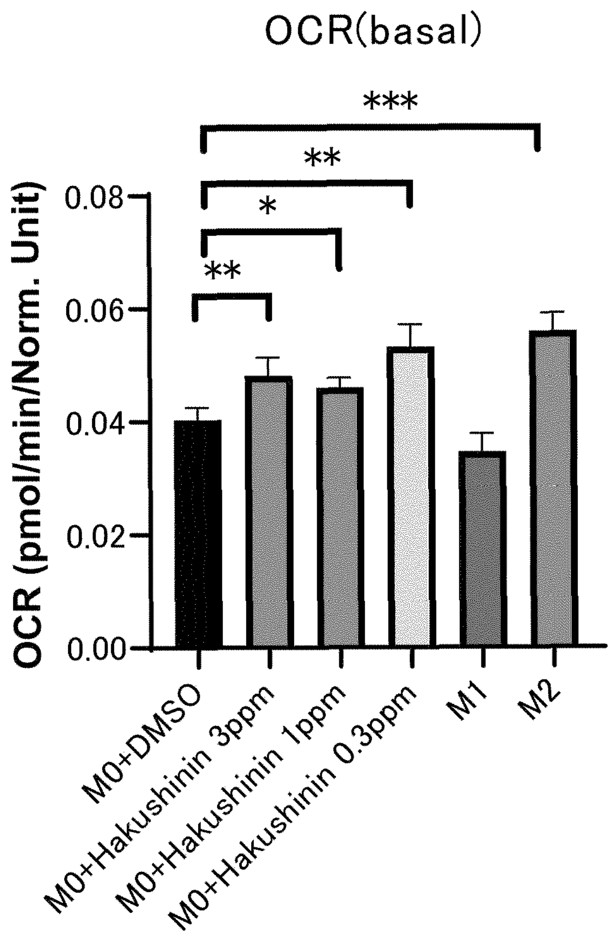
OCR(basal)
OCR (pmol/min/Norm. Unit)
0.08
0.06
0.04
0.02
0.00
***
**
*
**
M0+DMSO
M0+Hakushinin 3ppm
M0+Hakushinin 1ppm
M0+Hakushinin 0.3ppm
M1
M2

FIG. 22

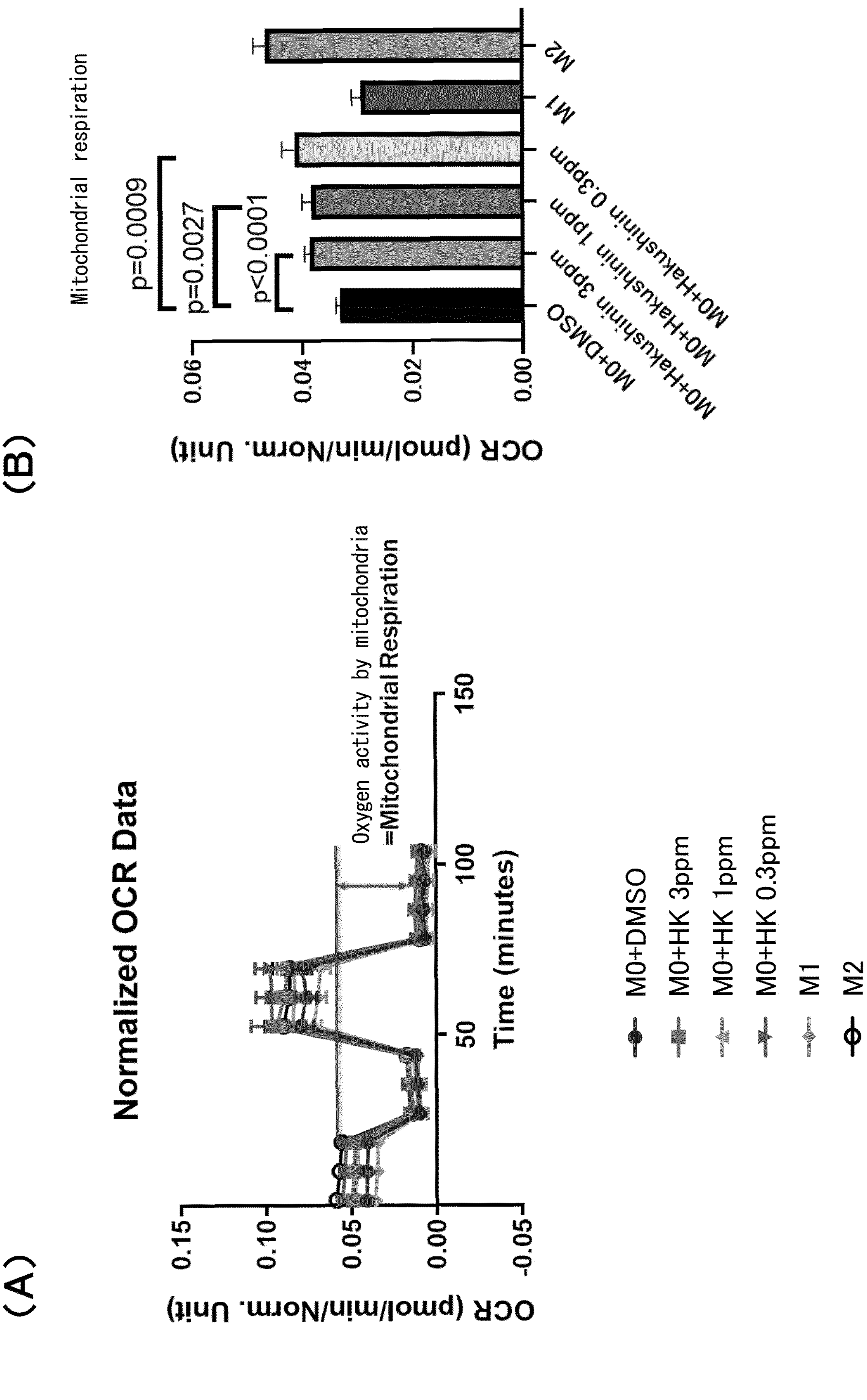
(A)
Normalized OCR Data
OCR (pmol/min/Norm. Unit)
0.15
0.10
0.05
0.00
-0.05
50
100
150
Time (minutes)
Oxygen activity by mitochondria
=Mitochondrial Respiration
M0+DMSO
M0+HK 3ppm
M0+HK 1ppm
M0+HK 0.3ppm
M1
M2
(B)
Mitochondrial respiration
OCR (pmol/min/Norm. Unit)
0.06
0.04
0.02
0.00
p=0.0009
p=0.0027
p<0.0001
M0+DMSO
M0+Hakushinin 3ppm
M0+Hakushinin 1ppm
M0+Hakushinin 0.3ppm
M1
M2

FIG. 23

48 hours after addition

(A)

(B)

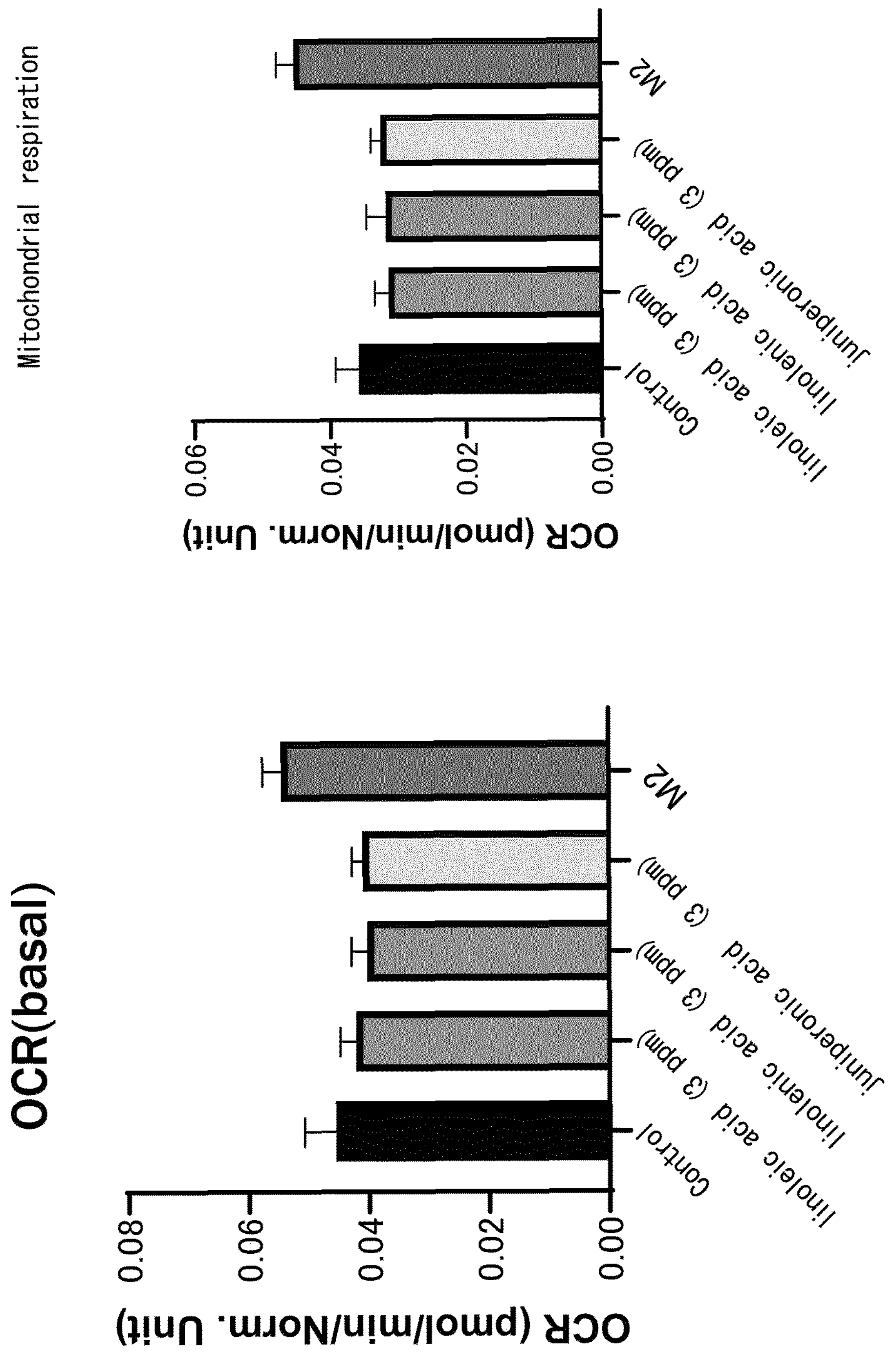

FIG. 24

24 hours after addition

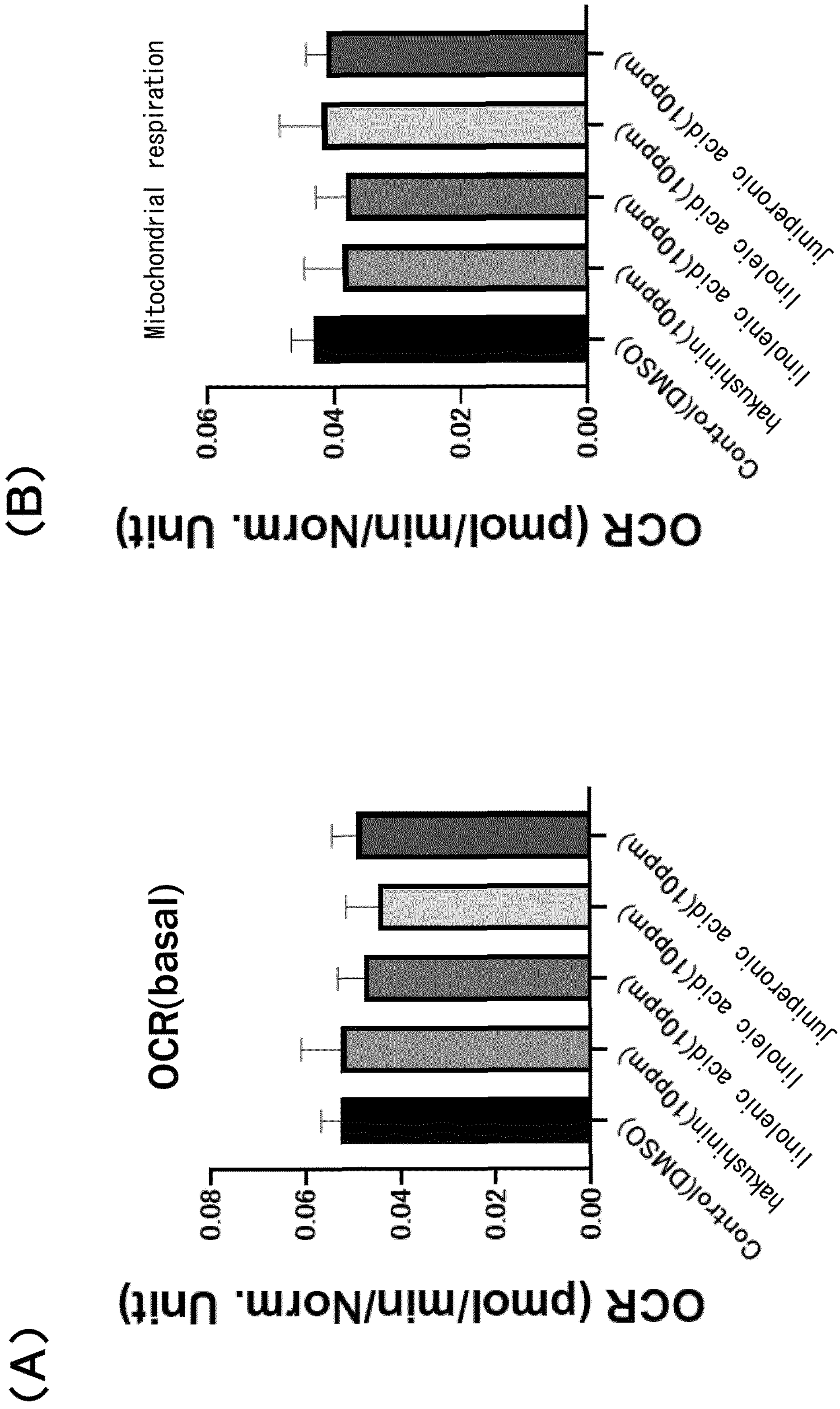

FIG. 25

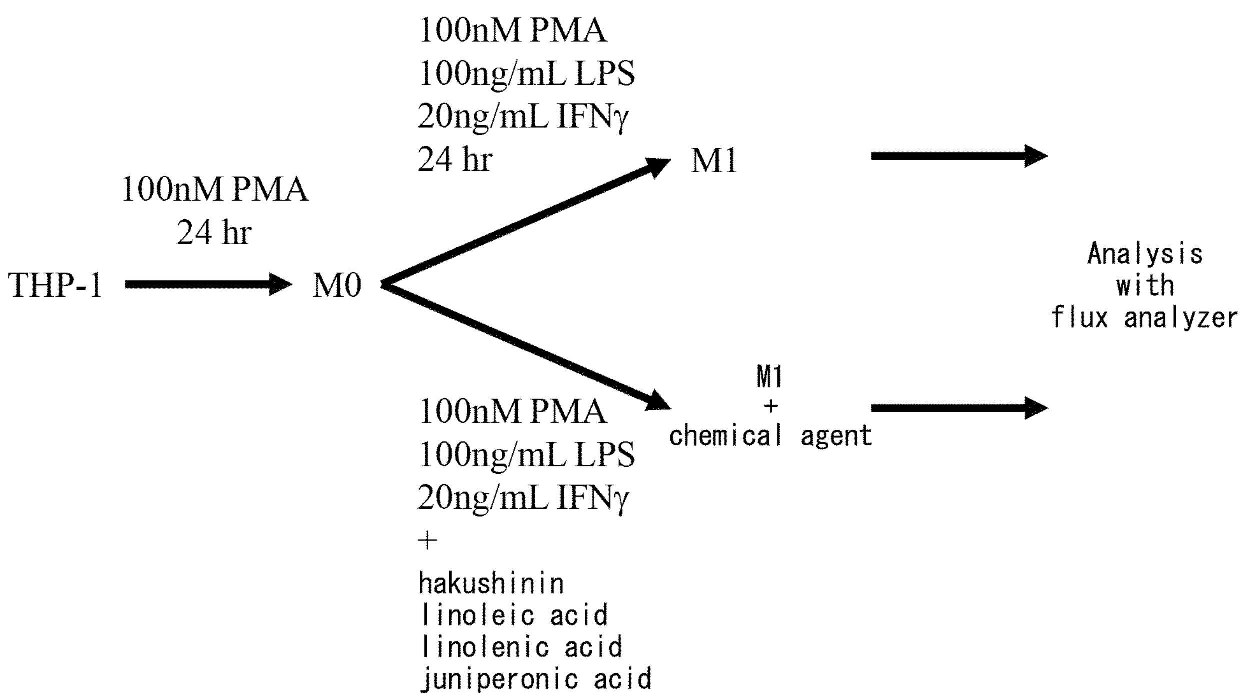
100nM PMA
100ng/mL LPS
20ng/mL IFNγ
24 hr
M1
100nM PMA
24 hr
THP-1
M0
Analysis
with
flux analyzer
M1
+
chemical agent
100nM PMA
100ng/mL LPS
20ng/mL IFNγ
+
hakushinin
linoleic acid
linolenic acid
juniperonic acid

FIG. 26

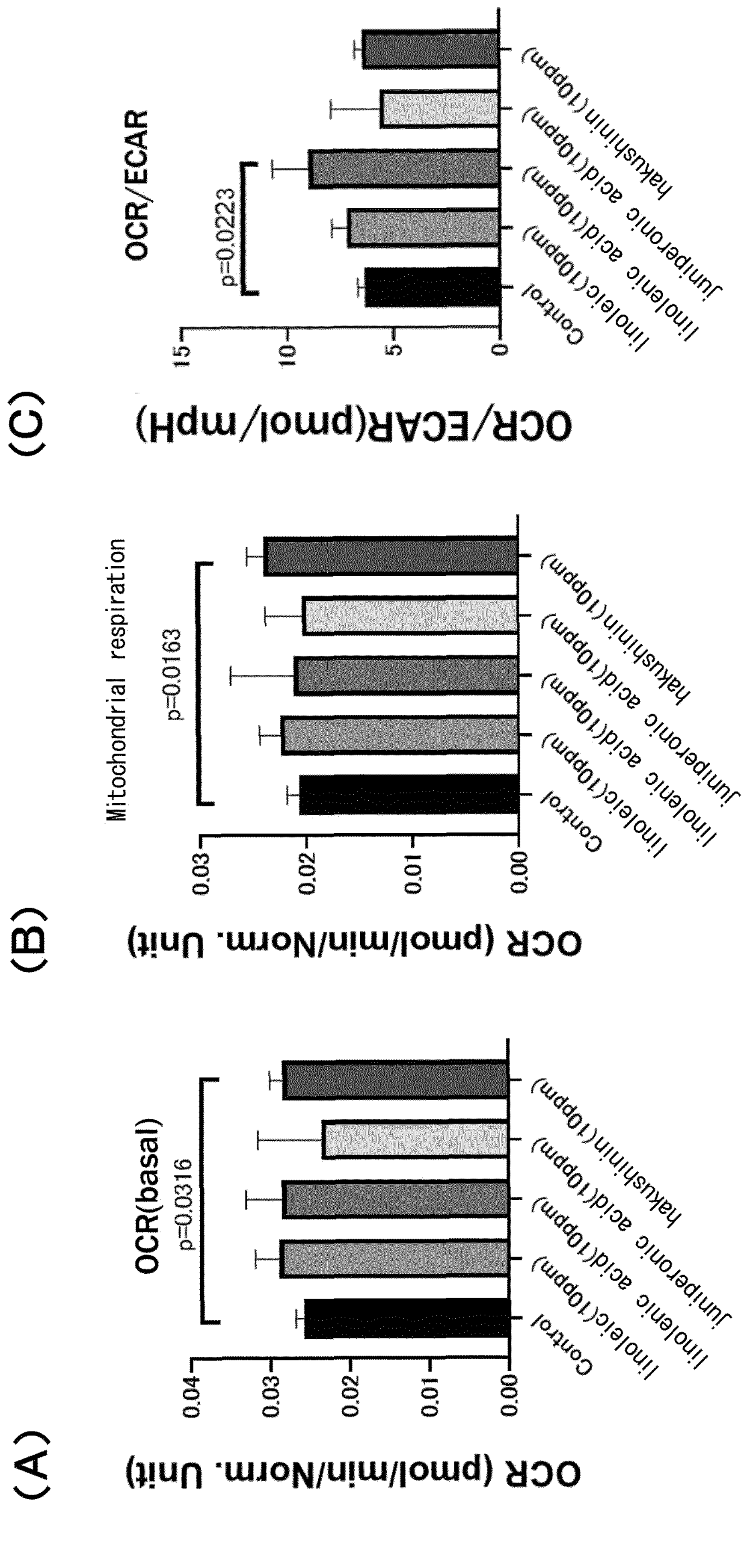
(A)
OCR (pmol/min/Norm. Unit)
0.00
0.01
0.02
0.03
0.04
OCR(basal)
p=0.0316
Control
linoleic(10ppm)
linolenic acid(10ppm)
Juniperonic acid(10ppm)
hakushinin(10ppm)
(B)
OCR (pmol/min/Norm. Unit)
0.00
0.01
0.02
0.03
Mitochondrial respiration
p=0.0163
Control
linoleic(10ppm)
linolenic acid(10ppm)
Juniperonic acid(10ppm)
hakushinin(10ppm)
(C)
OCR/ECAR(pmol/mpH)
0
5
10
15
OCR/ECAR
p=0.0223
Control
linoleic(10ppm)
linolenic acid(10ppm)
Juniperonic acid(10ppm)
hakushinin(10ppm)

FIG. 27

(A)

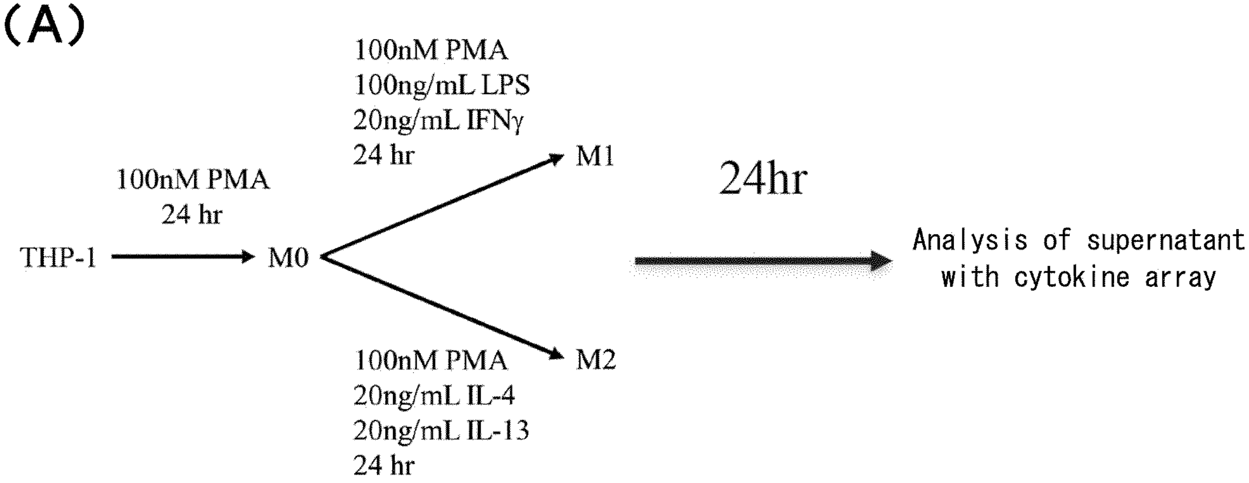


(B)

Cytokine array in supernatant
SDF-1a abundant in M2 supernatant

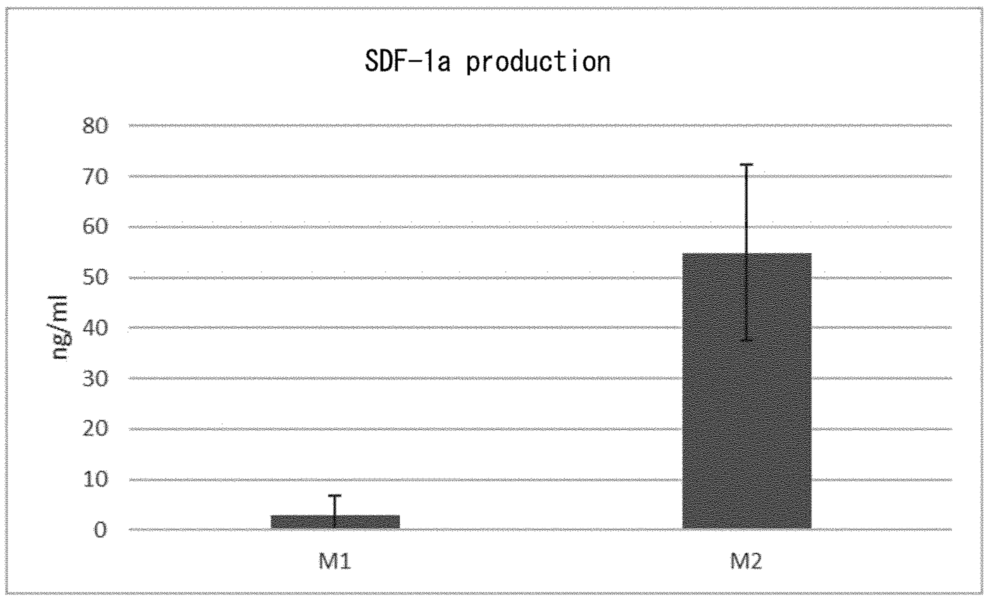

FIG. 28

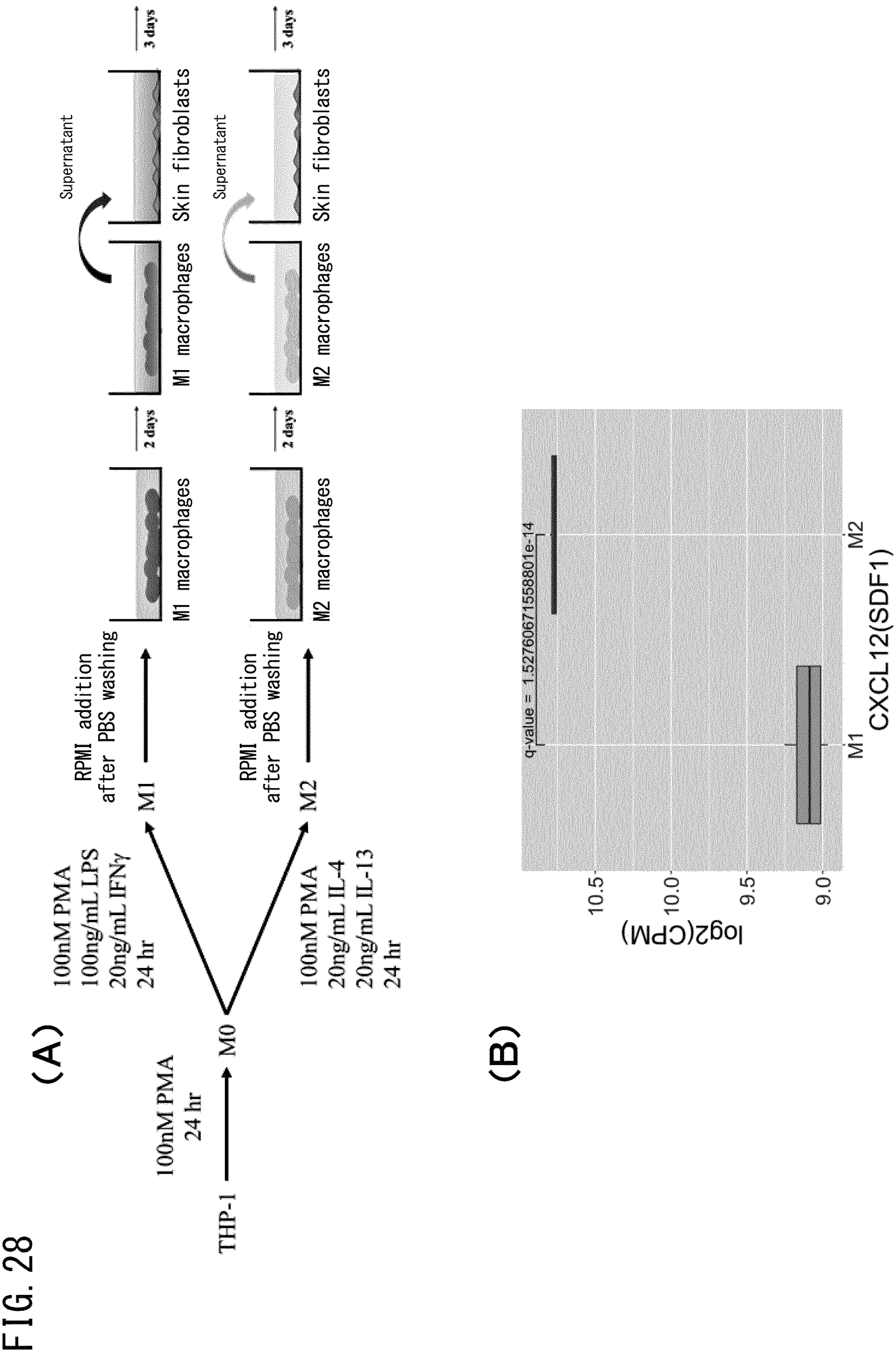
(A)
THP-1
100nM PMA
24 hr
M0
100nM PMA
100ng/mL LPS
20ng/mL IFNγ
24 hr
100nM PMA
20ng/mL IL-4
20ng/mL IL-13
24 hr
M1
M2
RPMI addition
after PBS washing
M1 macrophages
M2 macrophages
2 days
Supernatant
Skin fibroblasts
3 days
(B)
q-value = 1.52760671558801e-14
log2(CPM)
10.5
10.0
9.5
9.0
M1
M2
CXCL12(SDF1)

## INTERNATIONAL SEARCH REPORT

International application No.
PCT/JP2021/015919

A. CLASSIFICATION OF SUBJECT MATTER
A61Q 17/04(2006.01)i; A61Q 19/00(2006.01)i; A61Q 19/08(2006.01)i; A61H 23/02(2006.01)i; A61K 8/9761(2017.01)i
FI: A61K8/9761; A61Q19/08; A61Q19/00; A61Q17/04; A61H23/02 360

According to International Patent Classification (IPC) or to both national classification and IPC

B. FIELDS SEARCHED

Minimum documentation searched (classification system followed by classification symbols)
A61Q17/04; A61Q19/00; A61Q19/08; A61H23/02; A61K8/9761

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2005-179226 A (SHISEIDO CO., LTD.) 07 July 2005 (2005-07-07) claims 1-8, paragraph [0038], examples 1-25, table 2 | 1-4<br>5-6 |
| X<br>Y | JP 07-025746 A (SUNTORY LIMITED) 27 January 1995 (1995-01-27) claim 1, examples 3, 14, 18, table 1 | 1-4<br>5-6 |
| X<br>Y | JP 2007-223944 A (SHISEIDO CO., LTD.) 06 September 2007 (2007-09-06) claims 1-6, paragraphs [0015], [0045]-[0046], [0053], examples, fig. 1-2 | 1-4<br>5-6 |
| X<br>Y | KR 10-2012-0115436 A (SEOUL PERFUMERY) 18 October 2012 (2012-10-18) claims 1-11, paragraphs [0001]-[0003], [0059]-[0155] | 1-4<br>5-6 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 June 2021 (15.06.2021) | 29 June 2021 (29.06.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2021/015919

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2011-522673 A (PACIFIC BIOSCIENCE LABORATORIES, INC.) 04 August 2011 (2011-08-04) claim 1, paragraphs [0026]-[0027], fig. 1 | 6<br>5-6 |
| X<br>Y | JP 2016-120107 A (KONINKLIJKE PHILIPS N.V.) 07 July 2016 (2016-07-07) claims 1, 15, paragraphs [0003], [0022], fig. 1, 6 | 6<br>5-6 |
| A | JP 05-345719 A (NIPPON SHINYAKU CO., LTD.) 27 December 1993 (1993-12-27) | 1-6 |
| A | JP 2005-281205 A (NARIS COSMETICS CO., LTD.) 13 October 2005 (2005-10-13) | 1-6 |
| A | 真鍋 一郎, 慢性炎症と加齢関連疾患, 日本老年医学会雑誌, 2017, 54(2), pp. 105-113, 108-109, "immunosenescence and inflammaging", (MANABE, Ichiro, "Inflammaging and age-associated diseases", Japanese Journal of Geriatrics) | 1-6 |

Form PCT/ISA/210 (continuation of second sheet) (January 2015)

## INTERNATIONAL SEARCH REPORT

Information on patent family members

International application No.
PCT/JP2021/015919

| Patent Documents referred in the Report | Publication Date | Patent Family | Publication Date |
|---|---|---|---|
| JP 2005-179226 A | 07 Jul. 2005 | (Family: none) | |
| JP 07-025746 A | 27 Jan. 1995 | (Family: none) | |
| JP 2007-223944 A | 06 Sep. 2007 | (Family: none) | |
| KR 10-2012-0115436 A | 18 Oct. 2012 | (Family: none) | |
| JP 2011-522673 A | 04 Aug. 2011 | US 2009/0306577 A1<br>claim 1, paragraphs [0039]-[0040], fig. 1<br>WO 2009/152056 A1<br>EP 2291839 A1<br>CN 102119411 A | |
| JP 2016-120107 A | 07 Jul. 2016 | (Family: none) | |
| JP 05-345719 A | 27 Dec. 1993 | (Family: none) | |
| JP 2005-281205 A | 13 Oct. 2005 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 5657723 B
- JP 2017203004 A
- JP 2018177805 A
- JP 2014504629 A
- JP 2015140334 A
- JP 6178088 B
- JP 6273304 B
- JP 2018072098 A
- JP 4781842 B
- WO 2012057123 A
- WO 20120571243 A

### Non-patent literature cited in the description

- *Journal of the American College of Cardiology,* 12 November 2013, vol. 62 (20), 1890-901
- *Experimental & Molecular Medicine,* 2014, vol. 46, e70
- *Nature,* 28 March 2013, vol. 495, 524-530
- *Journal of Investigative Dermatology,* 09 October 2008, vol. 129 (4), 1016-25
- *Stem Cell Research & Therapy,* 2018, vol. 9, 88, https://doi.org/10.1186/s13287-018-0821-5
- *Journal of the European Academy of Dermatology and Venereology 2011 European Academy of Dermatology and Venereology,* 2012, vol. 26, 1577-1580
- *British Journal of Dermatology,* 2005, vol. 153, 733-739
- *Pigment Cell Melanoma Research,* vol. 27 (3), 502-504
- *Ann Dermatol,* 2016, vol. 28 (3), 279-289
- *Endocrinology,* 2011, vol. 152 (10), 3779-90
- *British Journal of Dermatology,* 2005, vol. 2 (153), 37-46
- *Experimental Dermatology,* 2011, vol. 20 (11), 953-5
- *Fujifilm Research & Development,* (55-2010), 33-37
- *Nat Immunol,* 2014, vol. 15 (9), 846-855
- *Cell Metab.,* 07 February 2017, vol. 25 (2), 412-427
- *Theranostics,* 09 September 2018, vol. 8 (17), 4620-4632
- *Cell Metab,* 07 February 2017, vol. 25 (2), 412-427
- *Theranostics.,* 09 September 2018, vol. 8 (17), 4620-4632